# EUROPEAN PATENT SPECIFICATION

(11) **EP 3 307 781 B1**
(45) Date of publication and mention of the grant of the patent: **28.10.2020**
(21) Application number: 16733251.9
(22) Date of filing: 10.06.2016
(51) Int. Cl.: A61K 31/166, A61K 31/426, A61P 43/00, C07K 16/28, G01N 33/68

(54) **ANTIBODIES, COMPOUNDS AND SCREENS FOR IDENTIFYING AND TREATING CACHEXIA OR PRE-CACHEXIA**
ANTIKÖRPER, VERBINDUNGEN UND TESTS ZUR IDENTIFIZIERUNG UND BEHANDLUNG VON KACHEXIE ODER PRÄKACHEXIE
ANTICORPS, COMPOSÉS ET ÉCRANS PERMETTANT L'IDENTIFICATION ET LE TRAITEMENT LA CACHEXIE OU PRÉ-CACHEXIE

(30) Priority: 10.06.2015 US 201562173901 P; 10.06.2015 US 201562173908 P; 19.06.2015 US 201562182140 P; 16.07.2015 US 201562193516 P
(43) Date of publication of application: 18.04.2018
(60) Divisional application: 20196326.1
(73) Proprietor: The Broad Institute, Inc., Cambridge, MA 02142 (US); Dana-Farber Cancer Institute Inc. et Al., Boston, MA 02215 (US)
(72) Inventor: THOMAS, David, K., Brookline, MA 02446 (US); GOLUB, Todd, R., Newtown, MA 02464 (US)
(74) Representative: Plasseraud IP
(86) International application number: PCT/US2016/037082
(87) International publication number: WO 2016/201368

(56) References cited:
- WO-A1-2010/019656
- WO-A1-2011/042548
- WO-A1-2015/085097
- WO-A2-2007/109747
- MORLEY JOHN E ET AL: "Cachexia: pathophysiology and clinical relevance", THE AMERICAN JOURNAL OF CLINICAL NUTRITION, AMERICAN SOCIETY FOR NUTRITION, US, vol. 83, no. 4, 1 April 2006 (2006-04-01), pages 735-743, XP007913220, ISSN: 0002-9165
- SPARVERO LOUIS J ET AL: "RAGE (Receptor for Advanced Glycation Endproducts), RAGE Ligands, and their role in Cancer and Inflammation", JOURNAL OF TRANSLATIONAL MEDICINE, BIOMED CENTRAL, LONDON, GB, vol. 7, no. 1, 17 March 2009 (2009-03-17), page 17, XP021050751, ISSN: 1479-5876, DOI: 10.1186/1479-5876-7-17
- LECLERC E ET AL: "Binding of S100 proteins to RAGE: An update", BIOCHIMICA ET BIOPHYSICA ACTA. MOLECULAR CELL RESEARCH, ELSEVIER SCIENCE PUBLISHERS, AMSTERDAM, NL, vol. 1793, no. 6, 1 June 2009 (2009-06-01) , pages 993-1007, XP026151996, ISSN: 0167-4889, DOI: 10.1016/J.BBAMCR.2008.11.016 [retrieved on 2008-12-11]

## Description

This application claims benefit of and priority to U.S. Provisional Application Nos. 62/173,901 filed June 10, 2015, 62/173,908 filed June 10, 2015, 62/182,140 filed June 19, 2015 and 62/193,516 filed July 16, 2015.

All documents cited or referenced herein ("herein cited documents"), and all documents cited or referenced in herein cited documents, together with any manufacturer's instructions, descriptions, product specifications, and product sheets for any products mentioned herein or in any document cited by reference herein, may be employed in the practice of the invention. More specifically, all referenced documents are cited to the same extent as if each individual document was specifically and individually indicated to be cited.

### STATEMENT AS TO FEDERALLY SPONSORED RESEARCH

This invention was made with government support under Grant No. CA190101 awarded by the National Institutes of Health. The government has certain rights in the invention.

### FIELD OF THE INVENTION

The invention relates to cachexia, including compositions and methods for detecting individuals who are or who are likely to suffer from cachexia, and compositions and methods and uses of compositions for treating or alleviating cachexia.

### BACKGROUND OF THE INVENTION

Cachexia is a wasting syndrome associated with chronic diseases. Cachexia is defined as weight loss exceeding 5% within the previous 3-12 months, combined with fatigue, loss of skeletal muscle, and biochemical abnormalities (e.g., anemia or insulin resistance). Cancer-induced cachexia (CIC) is experienced by up to 80% of patients with advanced stage cancer, particularly those with gastrointestinal, pancreatic, thoracic and head and neck malignancies. CIC has been implicated in up to 25% of cancer-related deaths. Despite interventions such as total parenteral nutrition (complete daily intravenous nutrition), anti-inflammatory medications, and anabolic stimulation, a patient with cancer-induced cachexia will continue to lose weight, often becoming so frail that they are unable to receive anti-cancer therapies. This distinguishes CIC from other forms of cachexia, which may respond to nutrition supplementation coupled with anti-inflammatory therapy.

Despite being common in many solid tumor cancers, cachexia remains poorly studied, under-diagnosed and a largely untreated complication that predisposes patients to an increased mortality. Treatment approaches for CIC, including anabolic steroids, anti-catabolic therapies, appetite stimulants, and nutritional interventions, have failed to show significant efficacy. In fact, once established, no therapeutic approach has been able to reverse cancer-induced cachexia. Moreover, no diagnostic for CIC is available; rather clinicians are forced to rely on a description of clinical changes observed in patients with advanced disease.

Accordingly, methods for identifying patients before they meet the clinical criteria for cachexia, i.e., when they are pre-cachectic, are urgently required, as well as therapeutic methods for disrupting the patient's progression from pre-cachexia to cachexia (WO2015085097).

The receptor for advanced glycation end products (RAGE) is a multiligand cell surface protein belonging to the immunoglobulin superfamily. Most importantly, RAGE is considered a receptor for HMGB1 and several S100 proteins, which are Damage-Associated Molecular Pattern molecules (DAMPs).

Anti-RAGE monoclonal antibodies are known in the art and are developed to treat conditions known to involve the RAGE receptor, and compete for binding with a range of RAGE ligands including HMGB1. These anti-RAGE monoclonal antibodies are not the subject of the invention and teach away from the instant invention.

### SUMMARY OF THE INVENTION

The scope of the invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human (or animal) body by therapy (or for diagnosis).

The role of the receptor for advanced glycation end products (RAGE) in cachexia and pre-cachexia and its ligand(s) in inducing pre-cachexia and/or cachexia is poorly understood. Applicants have now demonstrated the role of particular RAGE ligands in pre-cachexia and/or cachexia, and disclose herein therapeutic engineered biologic compositions for advantageously and more specifically targeting RAGE in a subject at risk of developing pre-cachexia and/or cachexia or a subject suffering from pre-cachexia and/or cachexia.

The invention provides therapeutic, engineered protein/peptide compositions comprising e.g., anti-RAGE antibodies, to target a RAGE receptor (including soluble forms thereof and endogenous secretory RAGE), directly and/or *via* differential competition with one or more pre-cachexia and/or cachexia-associated RAGE ligands or markers. In aspects of the invention pre-cachexia is characterized by an alteration in biomarker or specific component levels in cells or body tissue like RAGE ligands (e.g. S100A7, S100A8 and S100A9), Myosin Heavy Chain (MYH) (e.g. any of skeletal or cardiac muscle MYHs, like myosin, heavy chain 3, skeletal muscle, embryonic (MYH3); myosin, heavy chain 6, cardiac muscle, alpha (MYH6); myosin, heavy chain 2, skeletal muscle, adult (MYH1, MYH2); myosin, heavy chain 7, cardiac muscle, beta (MYH7); myosin, heavy chain 7B, cardiac muscle, beta (MYH7B) etc), Soluble Insulin Receptor (INSR) and Branched Chain Amino Acids (BCAA). An alteration in the levels of the biomarkers may be an increase in level or a decrease in level of the biomarkers as compared to a baseline reference value level (e.g. a reference value of the biomarker level in a healthy or control subject or individual). Loss of lipid content in cells and cell atrophy are also changes that are observed in a pre-cachexia or cachexia state. Loss of lipid content may be associated with increased levels of lipid metabolites like sphingomyelins, lysophospholipids, di-acyl-glycerides, triacyl glycerides, cholesterol esters, and/ or phospholipids. In a preferred embodiment of the invention, increase in the levels of one or more of S100A7, S100A8, S100A9, MYH1, MYH2, MYH3, MYH6, MYH7, MYH7B and INSR are correlated to pre-cachexia and cachexia. The levels of BCAA are increased in pre-cachexia but are decreased in advanced or late stage cachexia. Applicants have also determined that increase in the plasma levels of one of more of S100A4, S100A6, S100A11, S100A12, S100P and HMGB1 are anti-correlated to pre-cachexia and cachexia. In a preferred embodiment the increase in levels of said biomarkers are determined in fluid obtained from a subject or patient, wherein the fluid may be plasma, serum, urine, saliva, ascites or pleural fluid. In a more preferred embodiment, the levels of said biomarkers are determined in plasma or serum.

In one aspect, the invention provides an anti-RAGE antibody or antibodies specific for a RAGE receptor, which antibody competes for binding to the RAGE receptor with a RAGE ligand of the S100 family, *e.g*., S100A7, S100A8, S100A9 and/or Calprotectin, and preferably may not compete for binding to the RAGE receptor with the RAGE ligands HMGB1 or S100B.

In another aspect, the invention provides an anti-RAGE antibody mixture/cocktail comprising one or more antibodies which compete for ligand binding to the RAGE receptor, wherein each antibody in said mixture/cocktail competes for binding of one or more specific S100 family member/ligand (*e.g*., S100A7, S100A8, S100A9 and/or Calprotectin) to the RAGE receptor, and preferably may not compete for binding to the RAGE receptor with the RAGE ligands HMGB1 or S100B.

In another aspect, the invention provides an antibody specific for a RAGE receptor, which antibody competes for binding to the RAGE receptor with a RAGE ligand of the S100 family, wherein said competition is *via* a moiety/epitope (linear and/or conformational) which is bound by one or more specific S100 family members/ligands that induce pre-cachexia and/or cachexia, but is not shared by S100 RAGE ligands which do not induce cachexia or pre-cachexia.

In one embodiment, the antibody binds to an epitope on the RAGE receptor selected from an epitope, which is bound by S100A7, S100A8, S100A9 and/or Calprotectin, and preferably may not bind to an epitope, which is bound by the RAGE ligand HMGB1.

For example, the antibody binds to a RAGE epitope bound by S100A7, S100A8, S100A9 and/or Calprotectin by mimicking the linear epitope defined by any one of residues 41-93 in S100A7; residues 36-89 in S100A8; or residues 45-98 in S100A9. In another example, the antibody binds to a RAGE epitope bound by S100A7, S100A8, S100A9 and/or Calprotectin by mimicking the conformational epitope represented by helix 2A formed by residues 41-53 in S100A7, residues 43-49 in S100A8 or residues 47-55 in S100A9.

The antibody does not mimic the epitope defined by residues 43-78 on S100B.

For example, the antibody binds to the V region of the RAGE receptor, formed by residues 23-116 in human RAGE, mimicking the interaction between the RAGE V-region and helix A2 in S100A7, S100A8 or S100A9. The antibody preferably may not bind to the V region of the RAGE receptor, formed by residues 23-116 in human RAGE, in a manner which mimics the interaction between RAGE and S100B residues 43-78.

In another aspect, the invention provides an antibody specific for a RAGE ligand selected from S100A7, S100A8, S100A9 and/or Calprotectin, and preferably may not bind to the RAGE ligands HMGB1 or S 100B. In one embodiment, the antibody according to this aspect of the invention mimics the RAGE epitope, which is targeted by S100A7, S100A8, S100A9 and/or Calprotectin.

In one embodiment, the antibody binds to a linear epitope, which is selected from the linear epitope defined by residues 41-93 in S100A7; residues 36-89 in S100A8; or residues 45-98 in S100A9. The antibody preferably may not bind to the S100B epitope defined by residues 43-78 on S100B.

In another embodiment, the antibody binds to the conformational epitope represented by helix A2 formed by residues 41-53 in S100A7, residues 43-49 in S100A8, residues 47-55 in S100A9 or residues 40-53 in S100A15. The antibody preferably may not bind to the conformational epitopes formed on HMGB1 or S100B.

In certain embodiments, any of the aforementioned antibody/antibodies may be selected from, but not limited to, an IgG, IgA, or an antigen binding antibody fragment selected from an antibody single variable domain polypeptide, dAb, FAb, F(ab')2, an scFv, an Fv, a V_{HH} domain (such as a Nanobody® or other camelized immunoglobulin domain) or a disulfide-bonded Fv. In certain embodiments, any of the above antibody types or fragments thereof may be prepared from one or more of a mammalian species selected from, but not limited to mouse, rat, rabbit, human. But of course, such antibodies should be humanized for use in humans. In certain embodiments, any of the above antibody types or fragments thereof may be provided as heterocinjugates, bispecific, single-chain, chimeric or humanized molecules having affinity for one or more specific S100 family members/ligands that induce pre-cachexia and/or cachexia.

In certain embodiments, any of the hereinmentioned antibody/antibodies binds to a RAGE receptor polypeptide or an S100A7, A8, A9 or A15 polypeptide, with a dissociation coefficient of 100nM or less, 75nM or less, 50nM or less, 25nM or less, such as 10nM or less, 5nM or less, 1nM or less, or in embodiments 500pM or less, 100pM or less, 50pM or less or 25pM or less.

In certain embodiments, any of the hereinmentioned antibody/antibodies may be provided as polyclonal and/or monoclonal antibodies, including any mixture thereof.

Accordingly, the term "antibody", unless the context so requires, includes polyclonal antisera and antibody cocktails as well as monoclonal antibodies. Antibodies may be monospecific, with narrow or broad specificity; or multispecific, such as bispecific, such that they possess two distinct epitope specificities in a single antibody molecule. Cocktails of antibodies may be targeted at two or more specific epitopes. Polyclonal antisera can be raised in a conventional manner against one or more antigens, and may include IgG, IgM and other antibody classes. In certain embodiments, antisera may be modified to comprise only IgG or only IgM. Antibody cocktails may be prepared by admixture of one or more monoclonal antibodies. In one embodiment, an antibody cocktail contains two, three, four or more monoclonal antibodies each of which binds to or mimics S100A7, S100A8 and/or S100A9, so binds to S100A15, as set out herein, but preferably may not bind to or mimic HMGB1 or S100B. Alternativley, the foregoing antigens may be targeted by multispecific antibodies and/ore polyclonal antisera.

In one embodiment, the antibody is monoclonal and binds a unique structural motif shared by S100A7/ A8/ A9/ A15 or a bi or multi-valent antibody that binds to any combination of S100A7/ A8/ A9/ A15.

In one aspect, the antibody or antibodies of the invention are formulated for intravenous (iv) or intramuscular (im) administration. Antibodies administered iv should extravasate from the circulation in order to enter the interstitial tissue space and bind to their cognate target. It is an advantage of the present invention that the anti-cachexia antibodies do not need to be targeted to solid tumors, which are resistant to extravasation as they exert a higher tissue fluid pressure which retards convective transport of antibodies from blood vessels into tissue.

The antibody, in one embodiment, is an antibody fragment such as a scFv, dAb or V_{HH} antibody. Small antibody fragments are extravasated much more readily into tissue, and for this reason can perform better than IgG or other larger antibodies. However, smaller fragments are also cleared faster from the circulation. A compromise must be struck between tissue accessibility and clearance. For example, see Wang et al., Clinical pharmacology & Therapeutics, 84:5, 2008, 548-558. Several antibody conjugates have been described which have extended half-life using a variety of strategies, for example through conjugation to albumin (such as human serum albumin). See Kontermann et al., BioDrugs April 2009, Volume 23, Issue 2, pp 93-109.

In one aspect the invention provides a vector system comprising nucleic acid molecule(s) encoding and expressing therapeutic, engineered protein/peptide compositions comprising e.g., antibodies, to target a RAGE receptor directly and/or via differential competition with one or more pre-cachexia and/or cachexia-associated RAGE ligands or markers.

In one aspect the invention provides a viral vector system (*e.g*., AAV, retroviral, lentiviral) comprising nucleic acid molecule(s) encoding and expressing therapeutic, engineered protein/peptide compositions comprising e.g., antibodies, to target a RAGE receptor directly and/or via differential competition with one or more pre-cachexia and/or cachexia-associated RAGE ligands or markers.

In one aspect the invention provides a method for viral delivery comprising administering to a subject at risk of developing pre-cachexia and/or cachexia or a subject suffering from pre-cachexia and/or cachexia, a viral vector system comprising nucleic acid molecule(s) encoding and expressing therapeutic, engineered protein/peptide compositions comprising e.g., antibodies, to target a RAGE receptor directly and/or via differential competition with one or more pre-cachexia and/or cachexia-associated RAGE ligands or markers. In certain embodiments the viral vector system is an AAV system or a lentivral system. Therapetuic compositions may also be delievered uing a modified RNA system (Zangi et al., Nature Biotechnology 31, 898-907 (2013)).

In another aspect, the invention features a method of inhibiting the loss of myosin heavy chain in a myocyte, the method involving contacting the myocyte with an effective amount of one or more agents that is an agent that inhibits RAGE activity, that is any one or more of an anti-RAGE antibody, a RAGE blocking peptide or small molecule that inhibits binding of RAGE to ligands, as defined in the foregoing aspects of the invention.

In another aspect, the invention features a method of inhibiting lipolysis in an adipocyte, the method involving contacting the adipocyte with an effective amount of one or more agents that is an agent that inhibits RAGE activity, that is any one or more of an anti-RAGE antibody, a RAGE blocking peptide or small molecule that inhibits binding of RAGE to ligands, as defined in the foregoing aspects of the invention.

In another aspect, the invention features a method of inhibiting atrophy in a cell, the method involving contacting the adipocyte, myocyte, or cardiomyocyte with an effective amount of one or more agents that is an agent that inhibits RAGE activity, that is an anti-RAGE antibody, as defined in the foregoing aspects of the invention. In another aspect, the disclosure features a method of inhibiting atrophy in a cell, the method involving contacting the adipocyte, myocyte, or cardiomyocyte with an effective amount of one or more agents that is an agent that inhibits RAGE activity, that is any one or more of an anti-RAGE antibody, a RAGE blocking peptide or small molecule that inhibits binding of RAGE to ligands, as defined in the foregoing aspects of the invention.

In various embodiments of the above aspects, the myocyte, adipocyte, cardiomyocyte or hepatocyte cell is *in vitro* or *in vivo.* In other embodiments of the above aspects, the myocyte, adipocyte, or hepatocyte cell is present in a subject identified as having at least one cancer. In still other embodiments of the above aspects, the cancer includes but is not limited to one or more carcinomas and sarcomas and cancers of the skin, pancreas, stomach, colon, thorax, liver, gallbladder, musculoskeletal system, breast, lung, ovary, uterus, endometrium, prostrate, colon, skin, mouth, salivary, esophagus, head and neck, plus other tumors of the gastrointestinal tract.

In another aspect, the invention features a method of treating pre-cachexia, the method involving administering to the subject an effective amount of one or more agents that is an agent that inhibits RAGE activity, that is any one or more of an anti-RAGE antibody, a RAGE blocking peptide or small molecule that inhibits binding of RAGE to ligands, as defined in the foregoing aspects of the invention.

In another aspect, the invention features a method of inhibiting the progression of precachexia to cachexia in a subject, the method involving administering to the subject an effective amount of one or more agents that is an agent that inhibits RAGE activity, that is an anti-RAGE antibody, as defined in the foregoing aspects of the invention. In another aspect, the disclosure features a method of inhibiting the progression of precachexia to cachexia in a subject, the method involving administering to the subject an effective amount of one or more agents that is an agent that inhibits RAGE activity, that is any one or more of, a RAGE blocking peptide or small molecule that inhibits binding of RAGE to ligands, as defined in the foregoing aspects of the invention.

In another aspect, the invention features a method of treating or preventing undesirable muscle or fat loss in a cancer patient, the method involving administering to the subject an effective amount of one or more agents that is an agent that inhibits RAGE activity, that is anti-RAGE antibody, as defined in the foregoing aspects of the invention. In another aspect, the disclosure features a method of treating or preventing undesirable muscle or fat loss in a cancer patient, the method involving administering to the subject an effective amount of one or more agents that is an agent that inhibits RAGE activity, that is any one or more of a RAGE blocking peptide or small molecule that inhibits binding of RAGE to ligands, as defined in the foregoing aspects of the invention.

In various embodiments of the above aspects, the subject is pre-selected as having a molecular signature indicative of pre-cachexia or cachexia by detecting an alteration in at least three markers that is any one or more of S100A2 or S100A4; S100A8 or S100A9; and S100A7; detecting an alteration in at least four markers that is any one or more of: S100A2 or S100A4; S100A8 or S100A9; S100A7; and S100A14; and/or measuring the level of at least five markers that is any one or more of: S100A2 or S100A4; S100A8 or S100A9; S100A7; S100A14; and S100P, thereby pre-selecting the patient as having a molecular signature indicative of pre-cachexia or cachexia. Although certain of these markers are not causative of cachexia, many markers are coordinately expressed and hence provide diagnostic potential even if not specifically causative of cachexia.

In another aspect, the disclosure features a method of enhancing cancer sensitivity to chemotherapy, the method involving administering gemcitabine in combination with an anti-RAGE therapy that is any one or more of an anti-RAGE antibody, a RAGE blocking peptide, an antibody targeted to a RAGE ligand such as S100 A7/ A8/ A9 or A15, or a small molecule that inhibits binding of RAGE to ligands, as described in the preceding aspects of the invention, thereby enhancing cancer sensitivity to chemotherapy. Also included are antibodies specific to the exposed V-domain of RAGE, as explained herein. Gemcitabine is a cytotoxic chemotherapeutic agent, which is used most often in combination with other agents (such as platinum compounds or combinations of drugs such as FOLFOX). Part of chemoresistance to Gemcitabine is mediated though increased cellular autophagy, which is mitigated by anti-RAGE therapy thereby recalibrating chemotherapeutic sensitivity. In one embodiment, the cancer is that is any one or more of one or more carcinoma or sarcoma, including but not limited to cancers of the skin, pancreas, stomach, colon, thorax, liver, gallbladder, musculoskeletal system, breast, lung, ovary, uterus, endometrium, prostrate, colon, skin, mouth, salivary, esophagus, head and neck, plus other tumors of the gastrointestinal tract.

Accordingly, anti-RAGE therapy can be delivered in combination with chemotherapy, which can achieve synergistic and/or curative results. Chemotherapeutic agents can be drugs or cytotoxic agents that inhibit or prevens the function of cells and/or causes destruction of cells. The drugs or cytotoxic agents may be targeted, or systemically administered. Examples of cytotoxic agents include radioactive isotopes, chemotherapeutic agents, and toxins such as small molecule toxins or enzymatically active toxins of bacterial, fungal, plant or animal origin, including synthetic analogues and derivatives thereof. The cytotoxic agent may be selected from the group consisting of an auristatin, a DNA minor groove binding agent, a DNA minor groove alkylating agent, an enediyne, a lexitropsin, a duocarmycin, a taxane, a puromycin, a dolastatin, a maytansinoid and a vinca alkaloid or a combination of two or more thereof.

In one embodiment the drug is a chemotherapeutic agent selected from the group consisting of a topoisomerase inhibitor, an alkylating agent (*eg*. nitrogen mustards; ethylenimes; alkylsulfonates; triazenes; piperazines; and nitrosureas), an antimetabolite (*eg* mercaptopurine, thioguanine, 5-fluorouracil), an antibiotics (*eg*. anthracyclines, dactinomycin, bleomycin, adriamycin, mithramycin. dactinomycin) a mitotic disrupter (*eg*. plant alkaloids - such as vincristine and/or microtubule antagonists - such as paclitaxel), a DNA intercalating agent (eg carboplatin and/or cisplatin), a DNA synthesis inhibitor, a DNA-RNA transcription regulator, an enzyme inhibitor, agene regulator, a hormone response modifier, a hypoxia-selective cytotoxin (*eg.* tirapazamine), an epidermal growth factor inhibitor, an anti-vascular agent (*eg.* xanthenone 5,6-dimethylxanthenone-4-acetic acid), a radiation-activated prodrug (*eg*. nitroarylmethyl quaternary (NMQ) salts) or a bioreductive drug or a combination of two or more thereof.

The chemotherapeutic agent may selected from the group consisting of Erlotinib (TARCEVA®), Bortezomib (VELCADE®), Fulvestrant (FASLODEX®), Sutent (SU11248), Letrozole (FEMARA®), Imatinib mesylate (GLEEVEC®), PTK787/ZK 222584, Oxaliplatin (Eloxatin®.), 5-FU (5-fluorouracil), Leucovorin, Rapamycin (Sirolimus, RAPAMUNE®.), Lapatinib (GSK572016), Lonafarnib (SCH 66336), Sorafenib (BAY43-9006), and Gefitinib (IRESSA®.), AG1478, AG1571 (SU 5271; Sugen) or a combination of two or more thereof.

The chemotherapeutic agent may be an alkylating agent - such as thiotepa, CYTOXAN® and/or cyclosphosphamide; an alkyl sulfonate - such as busulfan, improsulfan and/or piposulfan; an aziridine - such as benzodopa, carboquone, meturedopa and/or uredopa; ethylenimines and/or methylamelamines - such as altretamine, triethylenemelamine, triethylenepbosphoramide, triethylenethiophosphoramide and/or trimethylomelamine; acetogenin - such as bullatacin and/or bullatacinone; camptothecin; bryostatin; callystatin; cryptophycins; dolastatin; duocarmycin; eleutherobin; pancratistatin; sarcodictyin; spongistatin; nitrogen mustards - such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide and/or uracil mustard; nitrosureas - such as carmustine, chlorozotocin, fotemustine, lomustine, nimustine, and/or ranimnustine; dynemicin; bisphosphonates - such as clodronate; an esperamicin; a neocarzinostatin chromophore; aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN®. doxorubicin - such as morpholino-doxorubicin, cyanomorpholino-doxorubicin, 2-pyrrolino-doxorubicin and/or deoxydoxorubicin, epirubicin, esorubicin, idarubicin, marcellomycin, mitomycins - such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites - such as methotrexate and 5-fluorouracil (5-FU); folic acid analogues - such as denopterin, methotrexate, pteropterin, trimetrexate; purine analogues - such as fludarabine, 6-mercaptopurine, thiamiprine, thioguanine; pyrimidine analogues - such as ancitabine, azacitidine, 6-azauridine, carmofur, cytarabine, dideoxyuridine, doxifluridine, enocitabine, floxuridine; androgens - such as calusterone, dromostanolone propionate, epitiostanol, mepitiostane, testolactone; anti-adrenals - such as aminoglutethimide, mitotane, trilostane; folic acid replenisher - such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; macrocyclic depsipeptides such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; razoxane; rhizoxin; sizofiran; spirogermanium; tenuazonic acid; triaziquone; 2,2',2"-trichlorotriethylamine; trichothecenes - such as verracurin A, roridin A and/or anguidine; urethan; vindesine; dacarbazine; mannomustine; mitobronitol; mitolactol; pipobroman; gacytosine; arabinoside; cyclophosphamide; thiotepa; taxoids - such as TAXOL®. paclitaxel, abraxane, and/or TAXOTERE®, doxetaxel; chloranbucil; GEMZAR® gemcitabine; 6-thioguanine; mercaptopurine; methotrexate; platinum analogues - such as cisplatin and carboplatin; vinblastine; platinum; etoposide; ifosfamide; mitoxantrone; vincristine; NAVELBINE®, vinorelbine; novantrone; teniposide; edatrexate; daunomycin; aminopterin; xeloda; ibandronate; topoisomerase inhibitor RFS 2000; difluoromethylomithine (DMFO); retinoids - such as retinoic acid; capecitabine; and pharmaceutically acceptable salts, acids, derivatives or combinations of two or more of any of the above.

The drug may be a tubulin disruptor including but are not limited to: taxanes - such as paclitaxel and docetaxel, vinca alkaloids, discodermolide, epothilones A and B, desoxyepothilone, cryptophycins, curacin A, combretastatin A-4-phosphate, BMS 247550, BMS 184476, BMS 188791; LEP, RPR 109881A, EPO 906, TXD 258, ZD 6126, vinflunine, LU 103793, dolastatin 10, E7010, T138067 and T900607, colchicine, phenstatin, chalcones, indanocine, T138067, oncocidin, vincristine, vinblastine, vinorelbine, vinflunine, halichondrin B, isohomohalichondrin B, ER-86526, pironetin, spongistatin 1, spiket P, cryptophycin 1, LU103793 (cematodin or cemadotin), rhizoxin, sarcodictyin, eleutherobin, laulilamide, VP-16 and D-24851 and pharmaceutically acceptable salts, acids, derivatives or combinations of two or more of any of the above.

The drug may be a DNA intercalator including but are not limited to: acridines, actinomycins, anthracyclines, benzothiopyranoindazoles, pixantrone, crisnatol, brostallicin, CI-958, doxorubicin (adriamycin), actinomycin D, daunorubicin (daunomycin), bleomycin, idarubicin, mitoxantrone, cyclophosphamide, melphalan, mitomycin C, bizelesin, etoposide, mitoxantrone, SN-38, carboplatin, cis-platin, actinomycin D, amsacrine, DACA, pyrazoloacridine, irinotecan and topotecan and pharmaceutically acceptable salts, acids, derivatives or combinations of two or more of any of the above.

The drug may be an anti-hormonal agent that acts to regulate or inhibit hormone action on tumours - such as anti-estrogens and selective estrogen receptor modulators, including, but not limited to, tamoxifen, raloxifene, droloxifene, 4-hydroxytamoxifen, trioxifene, keoxifene, LY117018, onapristone, and/or fareston toremifene and pharmaceutically acceptable salts, acids, derivatives or combinations of two or more of any of the above. The drug may be an aromatase inhibitor that inhibits the enzyme aromatase, which regulates estrogen production in the adrenal glands - such as, for example, 4(5)-imidazoles, aminoglutethimide, megestrol acetate, AROMASIN®. exemestane, formestanie, fadrozole, RIVISOR®. vorozole, FEMARA®. letrozole, and ARIMIDEX® and/or anastrozole and pharmaceutically acceptable salts, acids, derivatives or combinations of two or more of any of the above.

The drug may be an anti-androgen - such as flutamide, nilutamide, bicalutamide, leuprolide, goserelin and/or troxacitabine and pharmaceutically acceptable salts, acids, derivatives or combinations of two or more of any of the above.

The drug may be a protein kinase inhibitor, a lipid kinase inhibitor or an anti-angiogenic agent.

The drug could also be a cytokine (e.g., an interleukin, a member of the TNF superfamily, or an interferon).

In a preferred embodiment, the drug is a maytansinoid, in particular DM1, or a tubulin disruptor.

### Definitions

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Sp ringer Verlag (1991); and Hale & Marham, The Harper Collins Dictionary of Biology (1991). As used herein, the following terms have the meanings ascribed to them below, unless specified otherwise.

"Pre-cachexia" is currently and clearly defined as a pathological state necessitating intervention (e.g., International Consensus Definition Classification 2011-present Lancet Onc, 2011, Vol. 12(5), 489-495). Accordingly, pre-cachexia encompasses the early pathophysiologic state of normal tissue wasting or atrophy which does not yet meet the clinical criteria for cachexia. For example, a subject may be pre-cachectic when their weight is stable or when their weight loss is about 1%, 2% or 3% of their body mass. As used with respect to the invention described herein, at least a subset of patients with pre-cachexia may be characterized by an increase in one or more S100 RAGE ligands and/or a decrease in soluble RAGE relative to a reference level without unintended weight loss of at least 5% or more of body weight. In this application, Applicants' have determined pre-cachexia to be characterized by an alteration in biomarker or specific component levels in cells or body tissue like RAGE ligands (e.g. S100A7, S100A8 and S100A9), Myosin Heavy Chain (MYH) (e.g. myosin, heavy chain 3, skeletal muscle, embryonic (MYH3); myosin, heavy chain 6, cardiac muscle, alpha (MYH6); myosin, heavy chain 2, skeletal muscle, adult (MYH1, MYH2); ; myosin, heavy chain 7, cardiac muscle, beta (MYH7); myosin, heavy chain 7B, cardiac muscle, beta (MYH7B) etc), Soluble Insulin Receptor (INSR) and Branched Chain Amino Acids (BCAA). In embodiments of the invention, the levels of biomarkers may be assayed in the plasma or other fluids (which include but are not limited to serum, urine, saliva, ascites, pleural fluid etc) or cells via biopsy samples- fresh or fixed. In further embodiments, elevated mRNA levels of specific RAGE ligands or INSR have been assessed (most likely as cleavage products) is another diagnostic approach. An alteration in the levels of the biomarkers may be an increase in level or a decrease in level of the biomarkers as compared to a baseline reference value level (e.g. a reference value of the biomarker level in a healthy or control subject or individual). Loss of lipid content in cells and cell atrophy are also changes that are observed in a pre-cachexia or cachexia state. Loss of lipid content may be associated with increased levels of lipid metabolites like sphingomyelins, lysophospholipids, di-acyl-glycerides, triacyl glycerides, cholesterol esters, and/ or phospholipids. In a preferred embodiment of the invention, increase in the plasma levels of one or more of S100A7, MYH3, MYH6, INSR and BCAA are correlated to pre-cachexia and cachexia. In further embodiments, alteration in the levels of biomarkers MYH1, MYH2, MYH7, MYH7B, MYH6, and MYH3 are related to skeletal or cardiac muscle cachexia. Applicants have also determined that increase in the plasma levels of one of more of S100A4, S100A6, S100A11, S100A12, S100P and HMGB1 are anti-correlated to pre-cachexia and cachexia.

"Cachexia" is a condition often but not exclusively associated with cancer, which is manifested in weight loss, of at least 5% or more of body weight. A universal definition of cachexia is provided in Fearon et al., Lancet Oncol 2011; 12: 489-95. In general, cachexia refers to the progressive loss of lean body mass (particularly of muscle mass) that typically is associated with gross body weight loss that is at least 5, 6, 7, 8, 9, 10% or more. Muscle and adipose tissue loss, indicative of cachexia, may be detected by a computed tomography (CT) scan (Martin et al., J Clin Oncol 31:1539-1547 (2013)), though this method has not been validated as sufficient to formally diagnose the condition as there are numerous conditions that result in similar findings by imaging studies alone. Currently, there is no molecular biomarker(s) for this condition, as the pathogenesis of cancer-induced cachexia remains to be elucidated.

By "cancer-induced cachexia" is meant cachexia associated with the presence of a cancer or tumor.

By "disease-induced cachexia" is meant cachexia associated with the presence of a disease that is not due to the presence of at least one cancer or tumor. Aspects of the invention may relate to tissue or muscle cachexia which may not only be associated with disease states but also with injury and acute or chronic stress. As used herein, at least a subset of patients with disease-induced cachexia may be characterized by an increase in one or more S100 RAGE ligands and/or a decrease in soluble RAGE relative to a reference level.

"Cardiac cachexia" is a form of cachexia that has long been recognized as a serious complication of heart disease which includes but is not limited to chronic heart failure (CHF), myocardial infarction, congestive heart failure etc. Cardiac cachexia is associated with poor prognosis, independently of functional disease severity, age, measures of exercise capacity, and left ventricular ejection fraction. Patients with cardiac cachexia suffer from generalised loss of lean tissue, fat tissue, as well as bone tissue. Cachectic CHF patients are weaker and fatigue earlier. This is due to both reduced skeletal muscle mass and impaired skeletal muscle quality.While cardiac cachexia is linked to raised plasma levels of tumor necrosis factor alpha (TNFα) and other inflammatory cytokines and increased concentration of epinephrine, norepinephrine, and cortisol, as well as high plasma renin activity and increased plasma aldosterone level, much is still to be understood in the progression from pre-cachexia to cachexia in cardiac patients. Particualrly, studies of entanercept, a TNF-receptor blocker and TNF antibodies in patients with congestive failure did not improve outcomes, as described in Anker SD & Sharma R, The syndrome of cardiac cachexia, Int J Cardiol. 2002 Sep;85(1):51-66, and Morley et al., Cachexia: pathophysiology and clinical relevance, Am J Clin Nutr. 2006 Apr;83(4):735-4,. An aspect of the invention encompasses treating cardiac cachexia with a medicament comprising an antibody to S100A7, S100A8 and/or S100A9. In a preferred embodiment, cardiac pre-cachexia is associated with an increased level of the biomarkers MYH6, MYH7 and MYH7B.

"COPD cachexia" refers to the disproportionate loss of fat free mass (FFM) in chornic obstructive pulmonary disease (COPD), which is a lung disease characterized by irreversible chronic airflow limitation with or without alveolar wall destruction. Weight loss and particularly loss of FFM were shown to adversely affect respiratory and peripheral muscle function and exercise capacity as described in Broekhuizen et al., Pulmonary cachexia, systemic inflammatory profile, and the interleukin 1β -511 single nucleotide polymorpbism1,2,3, Am J Clin Nutr. 2005 Nov;82(5): 1059-64 and Remels et al., The mechanisms of cachexia underlying muscle dysfunction in COPD, J Appl Physiol (1985). 2013 May;114(9): 1253-62. An aspect of the invention encompasses treating COPD cachexia with a medicament comprising an antibody to S100A7, S100A8 S100A9 and/or Calprotectin. In a preferred embodiment, cardiac pre-cachexia is associated with an increased level of the biomarkers MYH1, MYH2 and MYH3.

As used herein, "cellular differentiation" or "differentiation" is the process by which a less specialized cell becomes a more specialized cell type.

"High-throughput screening" (HTS) refers to a process that uses a combination of modern robotics, data processing and control software, liquid handling devices, and/or sensitive detectors, to efficiently process a large amount of (e.g., thousands, hundreds of thousands, or millions of) samples in biochemical, genetic or pharmacological experiments, either in parallel or in sequence, within a reasonably short period of time (e.g., days). Preferably, the process is amenable to automation, such as robotic simultaneous handling of 96 samples, 384 samples, 1536 samples or more. A typical HTS robot tests up to 100,000 to a few hundred thousand compounds per day. The samples are often in small volumes, such as no more than 1 mL, 500 µl, 200 µl, 100 µl, 50 µl or less. Through this process, one can rapidly identify active compounds, small molecules, antibodies, proteins or polynucleotides which modulate a particular biomolecular/genetic pathway. The results of these experiments provide starting points for further drug design and for understanding the interaction or role of a particular biochemical process in biology. Thus "high-throughput screening" as used herein does not include handling large quantities of radioactive materials, slow and complicated operator-dependent screening steps, and/or prohibitively expensive reagent costs, etc.

As used herein, a therapeutic that "prevents" a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample.

As used herein, a "subject" means a human or animal (in the case of an animal, more typically a mammal, and can be, but is not limited to, a non-human animal or mammal). In one aspect, the subject is a human. A "subject" mammal can include, but is not limited to, a human or non-human mammal, such as a primate, bovine, equine, canine, ovine, feline, or rodent; and, it is understood that an adult human is typically about 70 kg, and a mouse is about 20g, and that dosing from a mouse or other non-human mammal can be adjusted to a 70 kg human by a skilled person without undue experimentation.

The term "treating" is art-recognized and includes administration to the host or patient or subject of one or more of the subject compositions, e.g., to diminish, ameliorate, or stabilize the existing unwanted condition or side effects thereof. In aspects of the invention, treatment is for the patient or subject in need thereof.

By "agent" is meant a peptide, nucleic acid molecule, or small compound.

By "ameliorate" is meant decrease, suppress, attenuate, diminish, arrest, or stabilize the development or progression of a disease.

By "alteration" is meant a change (increase or decrease) in the expression levels or activity of a gene or polypeptide as detected by standard art known methods such as those described herein. As used herein, an alteration includes a 10% change in expression levels, preferably a 25% change, more preferably more than a 30% change, a 35% change, a 40% change, and most preferably a 50% or greater change in expression levels. In a more preferred embodiment of the invention, the upregulation or increase in biomarker levels is at least greater than a 30% increase over baseline or normal population reference standards.

"Detect" refers to identifying the presence, absence or amount of the analyte to be detected.

By "disease" is meant any condition or disorder that damages or interferes with the normal function of a cell, tissue, or organ. In one embodiment, the disease is pre-cachexia, cachexia, or refractory cachexia.

By "effective amount" is meant the amount of a required to ameliorate the symptoms of a disease relative to an untreated patient. The effective amount of active compound(s) used to practice the present invention for therapeutic treatment of a disease varies depending upon the manner of administration, the age, body weight, and general health of the subject. Ultimately, the attending physician or veterinarian will decide the appropriate amount and dosage regimen. Such amount is referred to as an "effective" amount.

The invention provides a number of targets that are useful for the development of highly specific drugs to treat or a disorder characterized by the methods delineated herein. In addition, the methods of the invention provide a facile means to identify therapies that are safe for use in subjects. In addition, the methods of the invention provide a route for analyzing virtually any number of compounds for effects on a disease described herein with high-volume throughput, high sensitivity, and low complexity.

By "fragment" is meant a portion of a polypeptide or nucleic acid molecule. This portion contains, preferably, at least 10%, 20%, 30%, 40%, 50%, 60%, 70%, 80%, or 90% of the entire length of the reference nucleic acid molecule or polypeptide. A fragment may contain 10, 20, 30, 40, 50, 60, 70, 80, 90, or 100, 200, 300, 400, 500, 600, 700, 800, 900, or 1000 nucleotides or amino acids.

The terms "isolated," "purified," or "biologically pure" refer to material that is free to varying degrees from components which normally accompany it as found in its native state. "Isolate" denotes a degree of separation from original source or surroundings. "Purify" denotes a degree of separation that is higher than isolation. A "purified" or "biologically pure" protein is sufficiently free of other materials such that any impurities do not materially affect the biological properties of the protein or cause other adverse consequences. That is, a nucleic acid or peptide of this invention is purified if it is substantially free of cellular material, viral material, or culture medium when produced by recombinant DNA techniques, or chemical precursors or other chemicals when chemically synthesized. Purity and homogeneity are typically determined using analytical chemistry techniques, for example, polyacrylamide gel electrophoresis or high performance liquid chromatography. The term "purified" can denote that a nucleic acid or protein gives rise to essentially one band in an electrophoretic gel. For a protein that can be subjected to modifications, for example, phosphorylation or glycosylation, different modifications may give rise to different isolated proteins, which can be separately purified.

By "isolated polynucleotide" is meant a nucleic acid (e.g., a DNA) that is free of the genes which, in the naturally-occurring genome of the organism from which the nucleic acid molecule of the invention is derived, flank the gene. The term therefore includes, for example, a recombinant DNA that is incorporated into a vector; into an autonomously replicating plasmid or virus; or into the genomic DNA of a prokaryote or eukaryote; or that exists as a separate molecule (for example, a cDNA or a genomic or cDNA fragment produced by PCR or restriction endonuclease digestion) independent of other sequences. In addition, the term includes an RNA molecule that is transcribed from a DNA molecule, as well as a recombinant DNA that is part of a hybrid gene encoding additional polypeptide sequence.

The term "polynucleotide" used herein refers to a polymer of deoxyribonucleotide or ribonucleotide that exists as a single-stranded or double-stranded form. The polynucleotide includes RNA genome sequences, DNA (gDNA and cDNA), and RNA sequences transcribed therefrom, and includes analogues of natural polynucleotides, unless specifically mentioned.

The polynucleotide also includes nucleotide sequences encoding the amino acid sequences of the heavy and light chain variable regions of an antibody disclosed herein, and nucleotide sequences complementary thereto. The complementary sequences include completely complementary sequences and substantially complementary sequences. For example, substantially complementary sequences are sequences that may be hybridized with nucleotide sequences encoding the amino acid sequences of the heavy or light chain variable regions of an antibody disclosed herein under stringent conditions known in the art. Stringent conditions mean, for example, hybridization to DNA in 6×SSC at about 45°C, followed by one or more washes in 0.2×SSC/0.1% SDS at about 50°C - 65°C.

By an "isolated polypeptide" is meant a polypeptide of the invention that has been separated from components that naturally accompany it. Typically, the polypeptide is isolated when it is at least 60%, by weight, free from the proteins and naturally-occurring organic molecules with which it is naturally associated. Preferably, the preparation is at least 75%, more preferably at least 90%, and most preferably at least 99%, by weight, a polypeptide of the invention. An isolated polypeptide of the invention may be obtained, for example, by extraction from a natural source, by expression of a recombinant nucleic acid encoding such a polypeptide; or by chemically synthesizing the protein. Purity can be measured by any appropriate method, for example, column chromatography, polyacrylamide gel electrophoresis, or by HPLC analysis.

By "marker","biomarker" or "biological marker" is meant any clinical indicator, protein, metabolite, or polynucleotide having an alteration associated with a disease or disorder or a measurable indicator of some biological state or condition. Biomarkers are often measured _and evaluated (e.g. whether their levels are increased or decreased or remain unchanged) to examine normal biological processes, pathogenic processes, or pharmacologic responses to a therapeutic intervention.. In one embodiment, an alteration in body mass, lean body mass, metabolism, or a metabolite, as well as RAGE ligands (e.g. S100A7, S100A8 and S100A9), Myosin Heavy Chain (MYH) (e.g. myosin, heavy chain 3, skeletal muscle, embryonic (MYH3) or myosin, heavy chain 6, cardiac muscle, alpha (MYH6), myosin, heavy chain 2, skeletal muscle, adult (MYH1 and MYH2) etc), Soluble Insulin Receptor (INSR) and Branched Chain Amino Acids (BCAA) are markers or biomarkers (e.g., clinical indicator) of disease state (e.g., pre-cachexia or cachexia).

By "Soluble Insulin Receptor" (INSR) is meant the soluble form of the insulin receptor (IR), a tetrameric transmembrane protein located on the surface of target cells, that is present in plamsa, e.g. human plasma.

By "Branched Chain Amino Acid" (BCAA) is meant an amino acid having aliphatic side-chains with a branch (a central carbon atom bound to three or more carbon atoms). Among the proteinogenic amino acids, there are three BCAAs which include but are not imited to leucine, isoleucine and valine. Non-proteinogenic BCAAs include but are not limited to norvaline and 2-aminoisobutyric acid. Validation of the elevation of circulating BCAA in human plasma as a early event in human pancreatic adenocarcinoma development is further described in Mayers et al., Elevation of circulating branched-chain amino acids is an early event in human pancreatic adenocarcinoma development, Nat Med. 2014 Oct;20(10): 1193-8. In aspects of the invention, levels of BCAA are initially found to increase in pre-cachexia but then the levels decrease in late stage cachexia, as further confirmed by Mayers et al.

By "metabolic profile" is meant alterations in the level or activity of one or more amino acid, small molecule cellular metabolite, polypeptide or lipid metabolites.

As used herein, "obtaining" as in "obtaining an agent" includes synthesizing, purchasing, or otherwise acquiring the agent.

By "reduces" is meant a negative alteration of at least 10%, 25%, 50%, 75%, or 100%.

By "reference" is meant a standard or control condition.

A "reference sequence" is a defined sequence used as a basis for sequence comparison. A reference sequence may be a subset of or the entirety of a specified sequence; for example, a segment of a full-length cDNA or gene sequence, or the complete cDNA or gene sequence. For polypeptides, the length of the reference polypeptide sequence will generally be at least about 16 amino acids, preferably at least about 20 amino acids, more preferably at least about 25 amino acids, and even more preferably about 35 amino acids, about 50 amino acids, or about 100 amino acids. For nucleic acids, the length of the reference nucleic acid sequence will generally be at least about 50 nucleotides, preferably at least about 60 nucleotides, more preferably at least about 75 nucleotides, and even more preferably about 100 nucleotides or about 300 nucleotides or any integer thereabout or therebetween.

It should be understood that proteins, including antibodies of the invention may associate with a specified region through various interactions to form ligand-receptor complexes. These interactions include but are not limited to electrostatic forces, such as hydrogen-bonding and Van der Waal forces, dipole-dipole interactions, hydrophobic interactions, pi-pi stacking, and so on. Other associations which describe more specific types of interactions include covalent bonds, electronic and conformational rearrangements, steric interactions, and so on. Thus, as used herein the term "associate" generally relates to any type of force which connects an antibody to a specified region. As used herein the term "interacts" generally relates to a more specific and stronger connection of an antibody to a specified region. As used herein the term "sterically blocks" is a specific type of association which describes an antibody interacting with a specific region and preventing other ligands from associating with that region through steric interactions. The terms "binds" or "specifically binds" as used throughout this application may be interpreted to relate to the terms "associates", "interacts" or "sterically blocks" as required.

By "specifically binds" is meant a compound or antibody that recognizes and binds a polypeptide of the invention, but which does not substantially recognize and bind other molecules in a sample, for example, a biological sample, which naturally includes a polypeptide of the invention.

Nucleic acid molecules useful in the methods of the invention include any nucleic acid molecule that encodes a polypeptide of the invention or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a double-stranded nucleic acid molecule. Nucleic acid molecules useful in the methods of the invention include any nucleic acid molecule that encodes a polypeptide of the invention or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a double-stranded nucleic acid molecule. By "hybridize" is meant pair to form a double-stranded molecule between complementary polynucleotide sequences (e.g., a gene described herein), or portions thereof, under various conditions of stringency. (See, e.g., Wahl, G. M. and S. L. Berger (1987) Methods Enzymol. 152:399; Kimmel, A. R. (1987) Methods Enzymol. 152:507).

For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and more preferably at least about 50% formamide. Stringent temperature conditions will ordinarily include temperatures of at least about 30° C, more preferably of at least about 37° C, and most preferably of at least about 42° C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In a preferred: embodiment, hybridization will occur at 30° C in 750 mM NaCl, 75 mM trisodium citrate, and 1% SDS. In a more preferred embodiment, hybridization will occur at 37° C in 500 mM NaCl, 50 mM trisodium citrate, 1% SDS, 35% formamide, and 100 .mu.g/ml denatured salmon sperm DNA (ssDNA). In a most preferred embodiment, hybridization will occur at 42° C in 250 mM NaCl, 25 mM trisodium citrate, 1% SDS, 50% formamide, and 200 µg/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25 °C, more preferably of at least about 42° C, and even more preferably of at least about 68 °C. In a preferred embodiment, wash steps will occur at 25° C in 30 mM NaCl, 3 mM trisodium citrate, and 0.1% SDS. In a more preferred embodiment, wash steps will occur at 42 °C in 15 mM NaCl, 1.5 mM trisodium citrate, and 0.1% SDS. In a more preferred embodiment, wash steps will occur at 68° C in 15 mM NaCl, 1.5 mM trisodium citrate, and 0.1% SDS. Additional variations on these conditions will be readily apparent to those skilled in the art. Hybridization techniques are well known to those skilled in the art and are described, for example, in Benton and Davis (Science 196:180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York.

By "substantially identical" is meant a polypeptide or nucleic acid molecule exhibiting at least 50% identity to a reference amino acid sequence (for example, any one of the amino acid sequences described herein) or nucleic acid sequence (for example, any one of the nucleic acid sequences described herein). Preferably, such a sequence is at least 60%, more preferably 80% or 85%, and more preferably 90%, 95% or even 99% identical at the amino acid level or nucleic acid to the sequence used for comparison.

Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program may be used, with a probability score between e⁻³ and e⁻¹⁰⁰ indicating a closely related sequence.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

As used herein, the terms "treat," treating," "treatment," and the like refer to reducing or ameliorating a disorder and/or symptoms associated therewith. It will be appreciated that, although not precluded, treating a disorder or condition does not require that the disorder, condition or symptoms associated therewith be completely eliminated.

Unless specifically stated or obvious from context, as used herein, the term "or" is understood to be inclusive. Unless specifically stated or obvious from context, as used herein, the terms "a", "an", and "the" are understood to be singular or plural.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

The recitation of a listing of chemical groups in any definition of a variable herein includes definitions of that variable as any single group or combination of listed groups. The recitation of an embodiment for a variable or aspect herein includes that embodiment as any single embodiment or in combination with any other embodiments or portions thereof.

Any compositions or methods provided herein can be combined with one or more of any of the other compositions and methods provided herein.

### BRIEF DESCRIPTION OF THE DRAWINGS

Figure 1 is a graph showing that S100B has little or no activity in inducing cachexia in the assay.
Figure 2 is a graph conmparing S 100A7, S 100A8/9 and HMGB1 in a cachexia assay.
Figure 3 is a graph comparing S100A12, S100A13, S100A15, S100A5 and S100P in a cachexia assay.

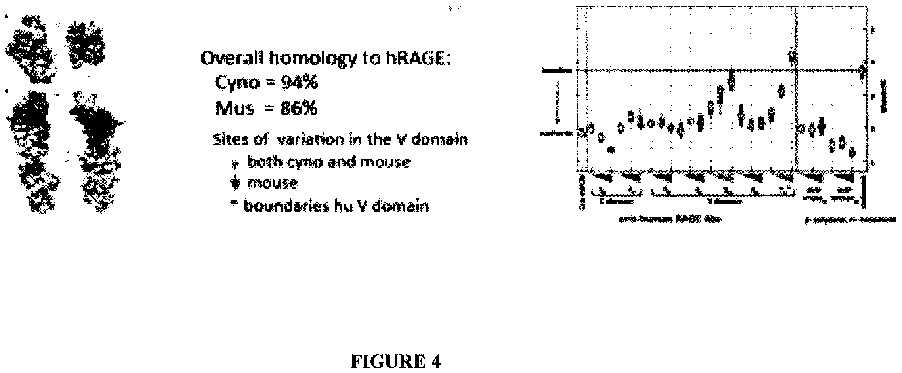

Figure 4 shows the key associated with the sequences in Figure 5.
Figure 5 is a comparison of early epitope mapping of anti-RAGE mAb's define inhibitory epitope.
Figure 6 illustrates RAGE antibodies which have different cachexia-inhibiting activities.
Figure 7 illustrates human RAGE antibody which has cachexia-inhibiting activity in mouse skeletal muscle.

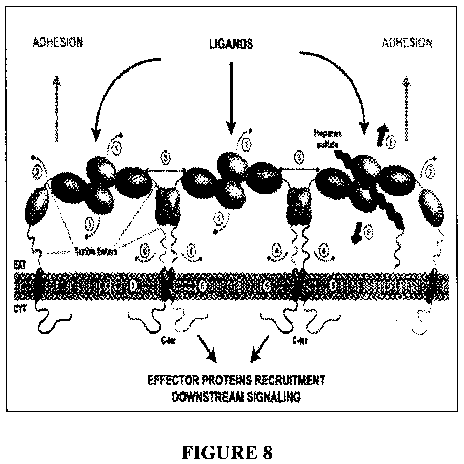

Figure 8 illustrates that RAGE signaling is dependent on V-V domain dimerization. X-ray crystallography based models indicate that V-V domain dimerization is not a typical lock and key, receptor-ligand model but is rather based on hydrophibic interactions which are further enhanced by ligand binding implying imteractions based on a mass action, equilibrium state.

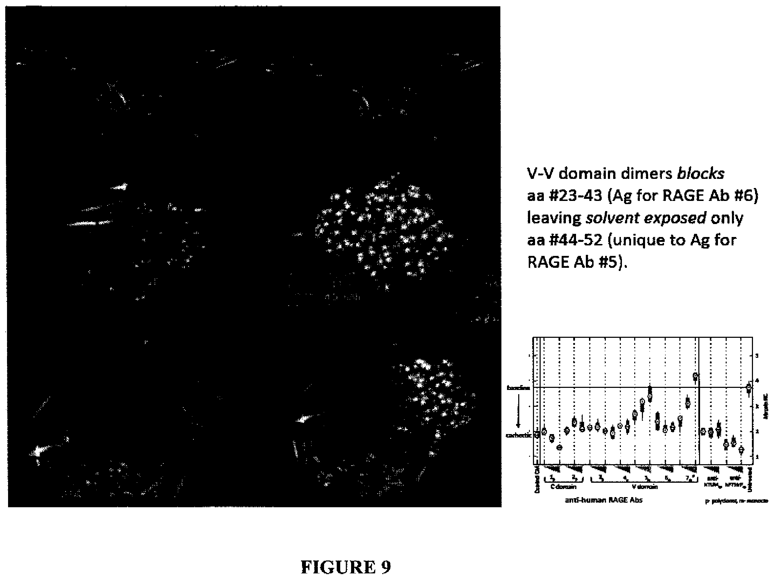

Figure 9 illustrates the structural epitope mapping of inhibitory huRAGE mAb and shows that the V-V dimerization exposes aa # 44-52 and thus an epitope herein identified comprises aa 44-52.

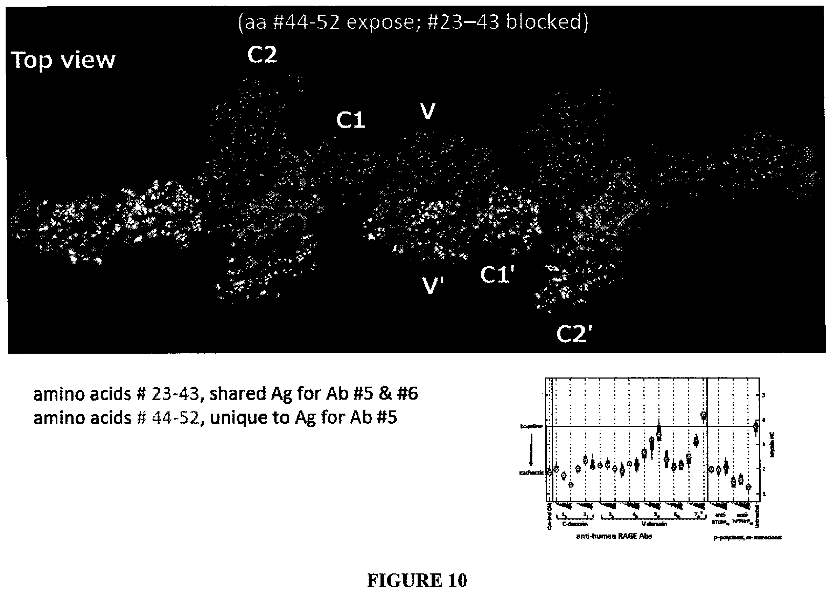

Figure 10 illustrates the structural epitope mapping of inhibitory huRAGE mAb and shows the top view of the molecule.

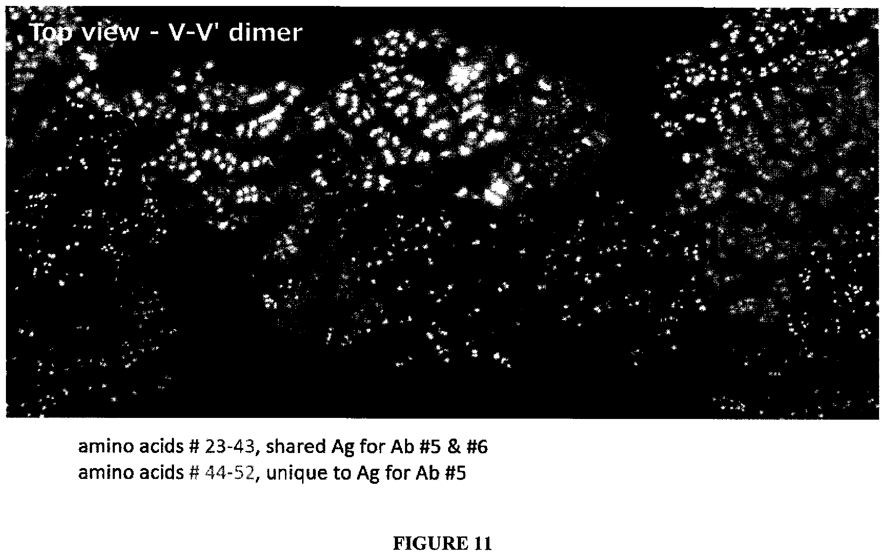

Figure 11 illustrates the structural epitope mapping of inhibitory huRAGE mAb and shows the top view of the V-V dimer.

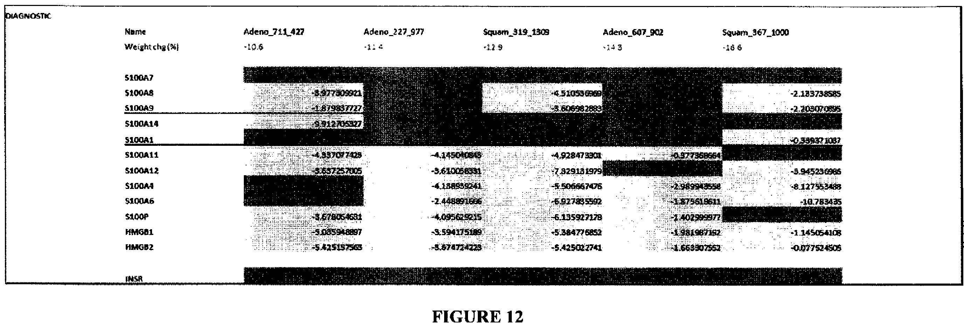

Figure 12 is a tabulation of diagnostic and pharmacodynamic biomarkers in pre-cachexia/cachexia based on deep proteomic analyses of case-controlled patient plasma.

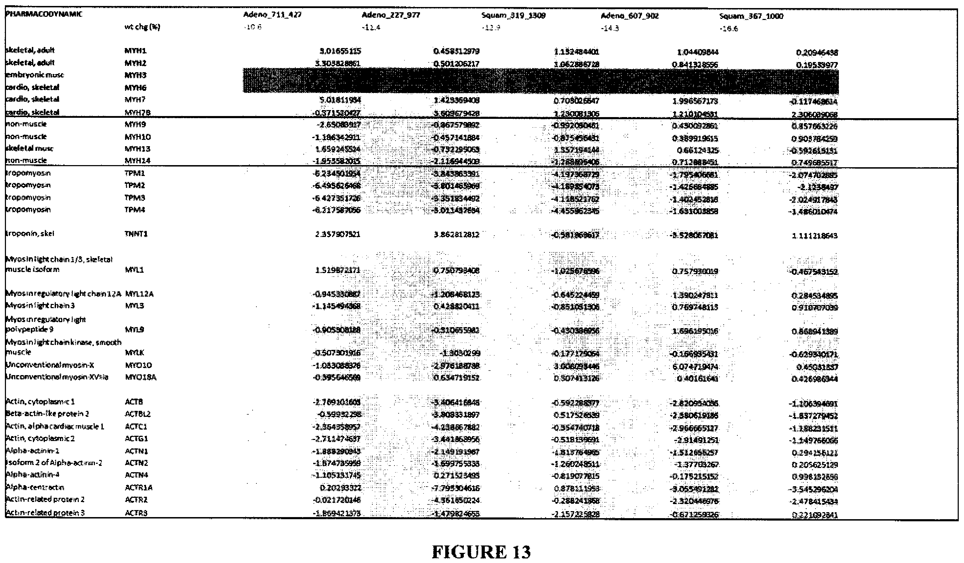

Figure 13 is a more detailed tabulation of diagnostic and pharmacodynamics biomarkers in pre-cachexia/cachexia based on deep proteomic analyses of case-controlled plasma.

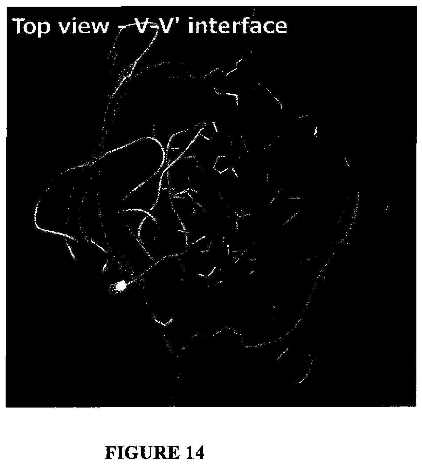

Figure 14 shows the hydrophobic core between the V domains of two hRAGE molecules. The following is a list of hydrophobic residues from each monomer involved in the interactions: P33, V35, L79, P80, F85, P87, A88. In addition a direct interaction between charged residues E32-K43 can be observed. However, large number of hydrophobic residues constituting the interface between V domains of hRAGE suggests that the dimerization is hydrophobically driven.

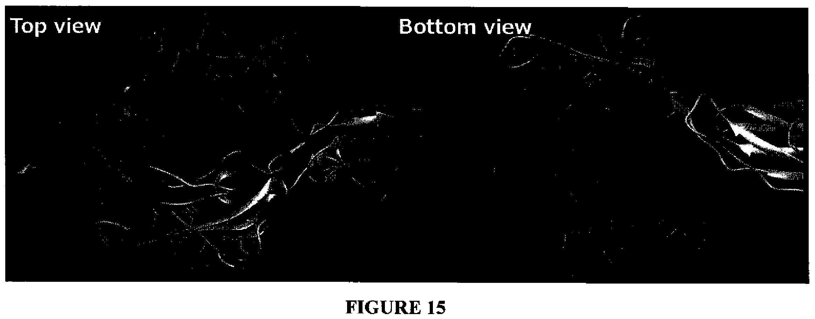

Figure 15 shows a dimer of hRAGE proteins (PDB ID: 4LP4 [Yatime, L., and Andersen, G.R. (2013). Structural insights into the oligomerization mode of the human receptor for advanced glycation end-products supported by FEBS J. 280, 6556-6568.]) with surface representation of the solvent accessible area in antigens of two RAGE antibodies: Cachexia inhibiting (green + red residues) and non-inhibiting (red). The area represented in red faces a cellular membrane, and is inaccessible for antibody binding. The amino acids represented in green (within residues 44 to 54) are exposed to the solvent and fully accessible for binding. The top and bottom view on a dimer of V domains in two hRAGE proteins is shown in the figure. The green surface represents solvent accessible area in antigens of two RAGE antibodies. The cellular membrane is in the plane of the figure and behind the screen in the first panel (not shown explicitly).
Figure 16 shows a surface representation of hRAGE with a cluster of molecular solvents (yellow) with the highest number of members (24, with the second one consisting of 15 members) which indicates that the position of the binding site is located between residues 45-49, 66-67, and 78-82 in V domain of hRAGE.
Figure 17 illustrates hot spot for small molecule ligand binding with detailed specification of surrounding residues in V domain of hRAGE.

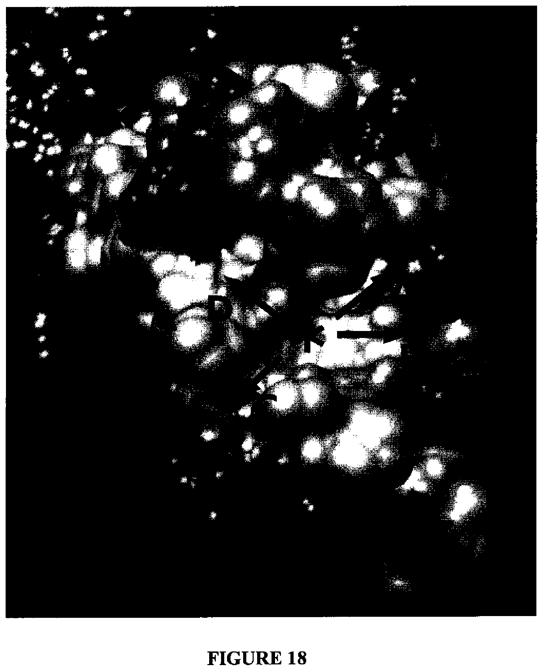

Figure 18 shows surface representation of the V domain. Asterisk indicates position of the binding site (*) in the V domain of hRAGE with four groves (A-D) that allow for customization of a potential drug. Hydrophobic residues are in grey, positively charged in dark blue, negatively charged in red, and all other types of residues are in orange. The C1 domain of hRAGE in the background is colored in purple. It can be noticed that the binding site is mostly hydrophobic and there are four groves on the surface of V domain that project from the binding site and can accommodate extensions of a drug bound to the site and be used to fine tune properties of the drug.

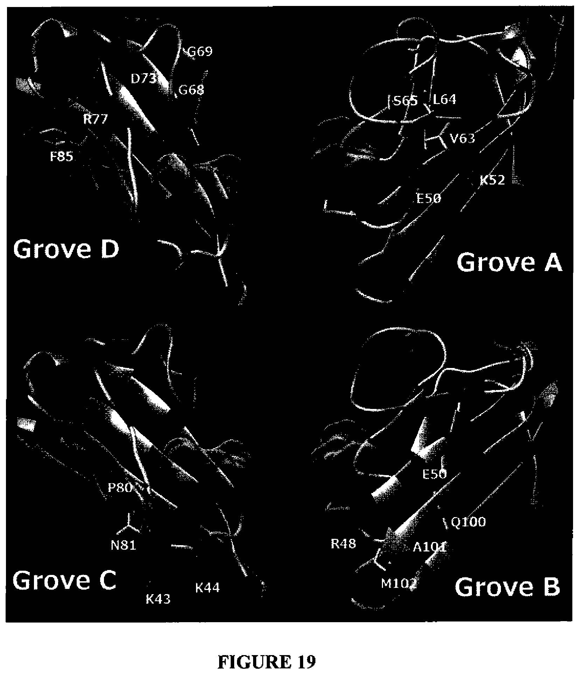

Figure 19 illustrates a detailed representation of residues forming the four groves extending from the main binding site.

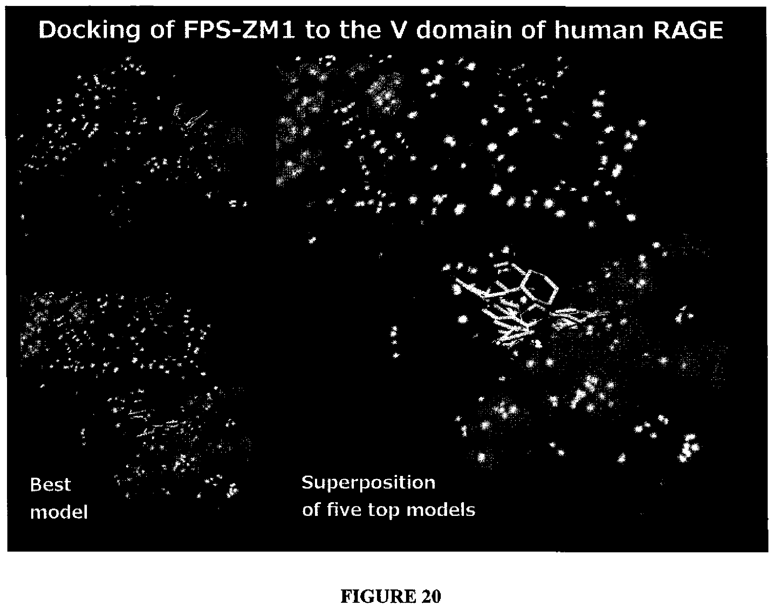

Figure 20 illustrates results of docking of FPS-ZM1 to the V domain of hRAGE (purple). Green and red color indicate antigen of two hRAGE antibodies.
Figure 21 illustrates results of docking of Azeliragon (TTP-488) to the V domain of hRAGE (purple). Green and red color indicate antigen of two hRAGE antibodies.
Figure 22 illustrates results of docking of FPS3 to the V domain of hRAGE (purple). Green and red color indicate antigen of two hRAGE antibodies.

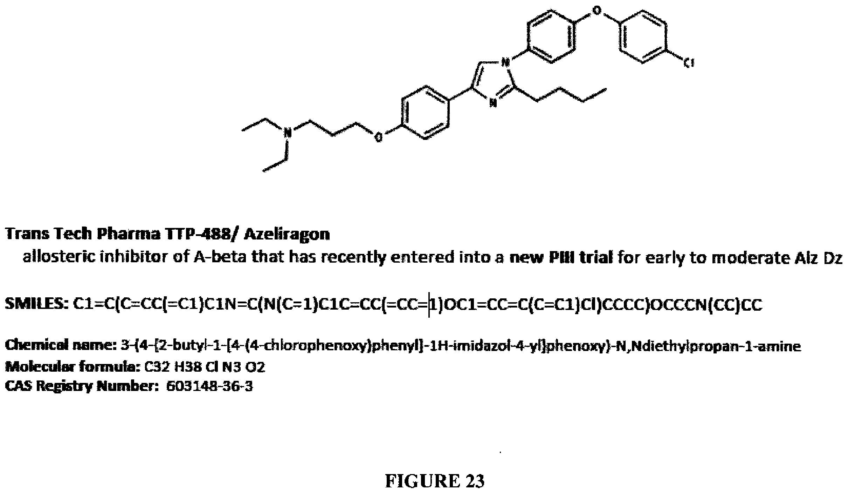

Figure 23 shows series 1 of allosteric inhibiotrs of RAGE V-domain: Aβ

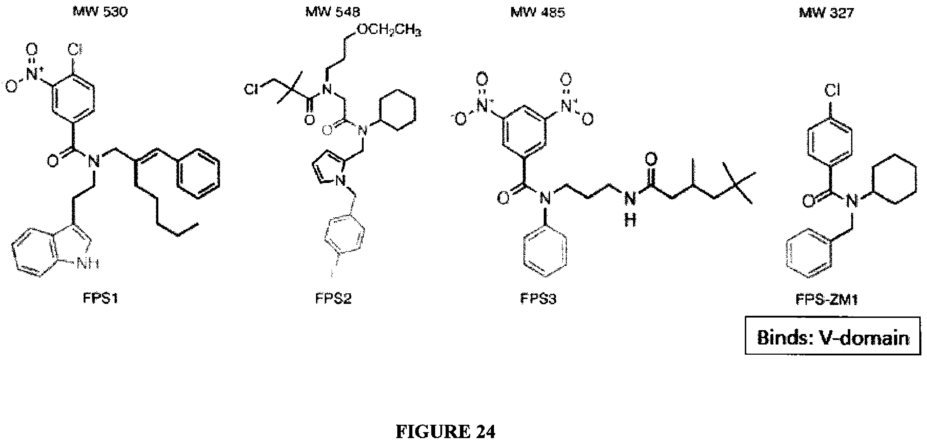

Figure 24 shows series 2 of allosteric inhibiotrs of RAGE V-domain: Aβ. Structure of new high-affinity Aβ/RAGE blockers. Each structure shows the tertiary amide group (red or the CONH₂) and its functional moieties, hydrophobic group (blue), electron-rich aromatic group (green), and electron-poor group (pink), as decribed in Deane R, J Clin Invest 2012.
Figure 25 illustrates the functional testing of FPS3 in Cachexia Assay. The RAGE V-domain small molecule inhibitor of Aβ also inhibits cachexia phenotype in HSkM functional assay. This implies that the modeled Ab epitope is likely a functional focused target for Ab therapy and that this is the basis for further small molecule therapeutic development for cancer cachexia and other RAGE mediated diseases.

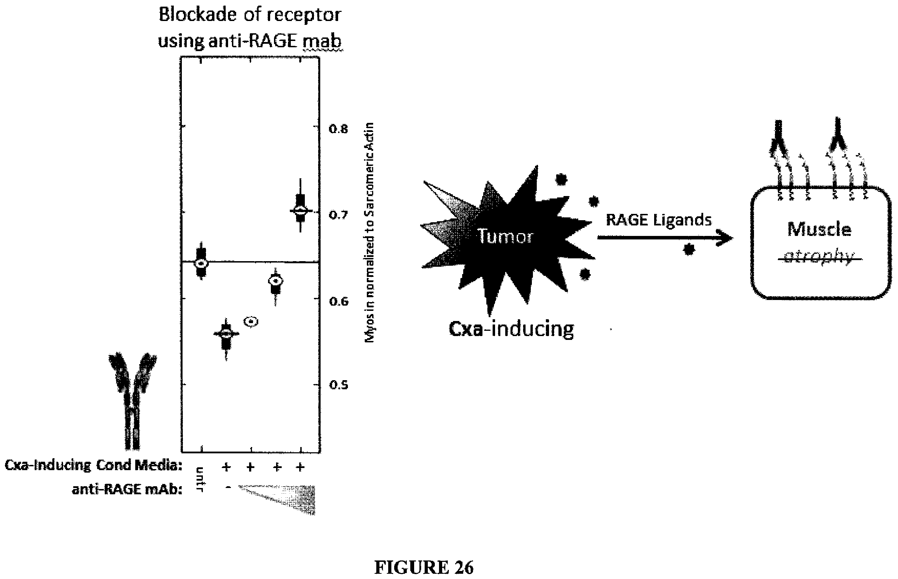

Figure 26 shows inhibitory receptor antibody blocks cachexia in skeletal myocytes.
Figure 27 shows that antibody inhibition of cachexia-inducing S100 proteins has a comparable efficiency in blocking cachexia.

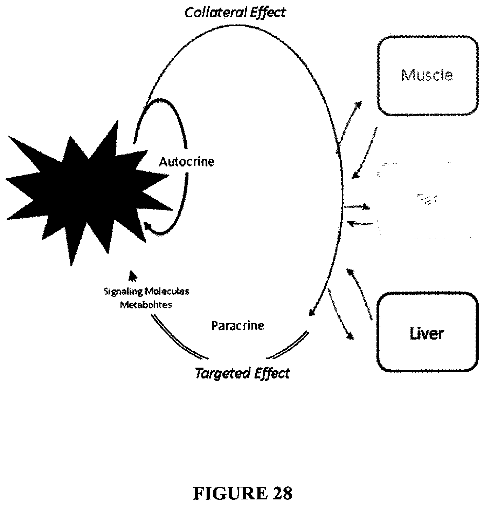

Figure 28 is a schematic that illustrates cancer cachexia signaling vs. targeted and/or collateral effects on muscle, fat, and liver.

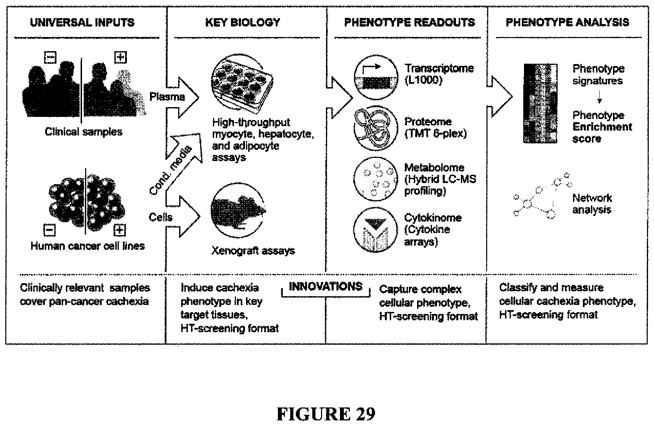

Figure 29 is a schematic of a clinical sample derived phenotypic screening platform for cancer cachexia mediator discovery (Reverse Translational Medicine).

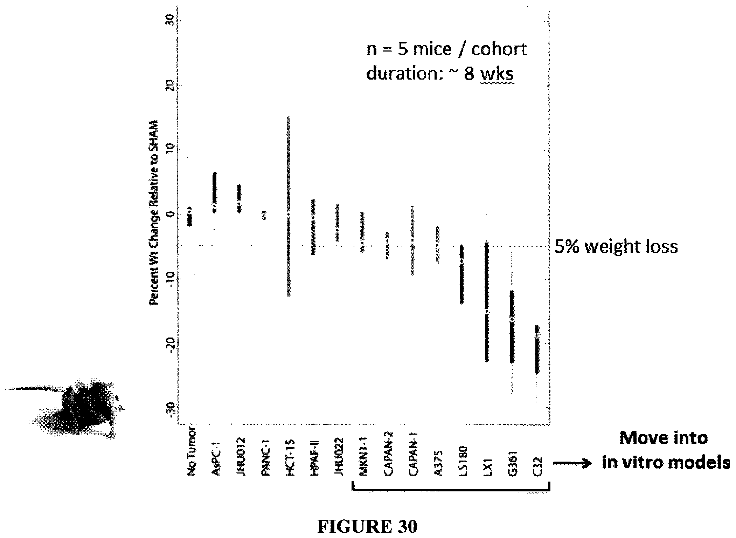

Figure 30 is a graph showing the mean tumor-free weight change in cohorts of xenograft mice (n=5 mice per cohort). Human Cancer Cell Lines are classified for Cachexia-inducing Activity *in vivo* and the most cachexia-inducing cell lines over the 8-week period are shown in red.
Figure 31 shows that the *in vitro* system recapitulates cachexia phenotype in mouse and human muscle. Patient Plasma *in vitro* muscle signature vs. cachectic patient muscle biopsy signature was compared and the specificity for clinical cachexia is greater than the 99.9%ile. The analysis was performed across 4,815 genetic and small molecule perturbations in Connectivity Map. See text below in examples for a listing of the labels shown under the "gene_symbol" section for the heat panels.

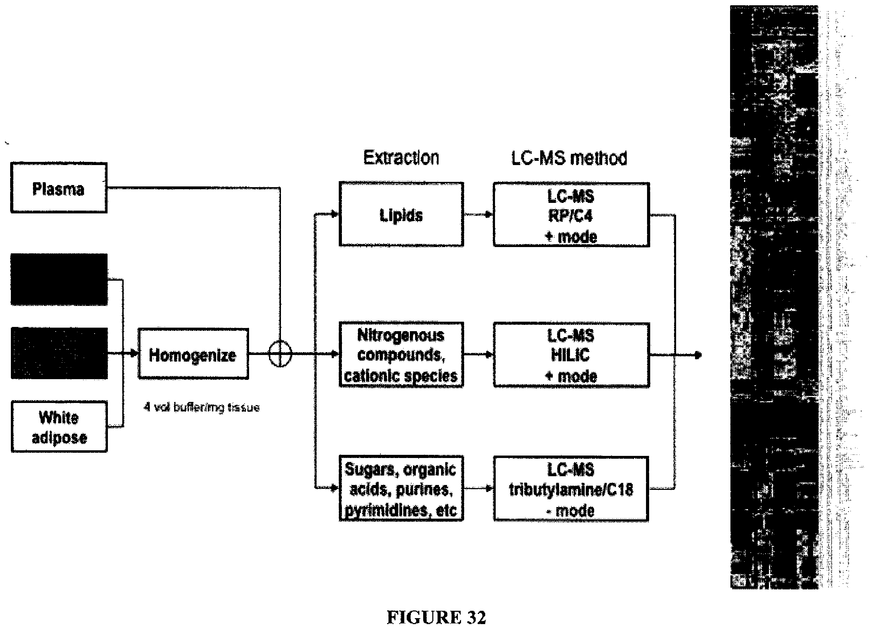

Figure 32 shows a schematic of Metabolomic Profiling with global high-resolution readout. Metabolomic Profiling Platform analyzes > 320 lipid, cationic, and anionic metabolites / sample.

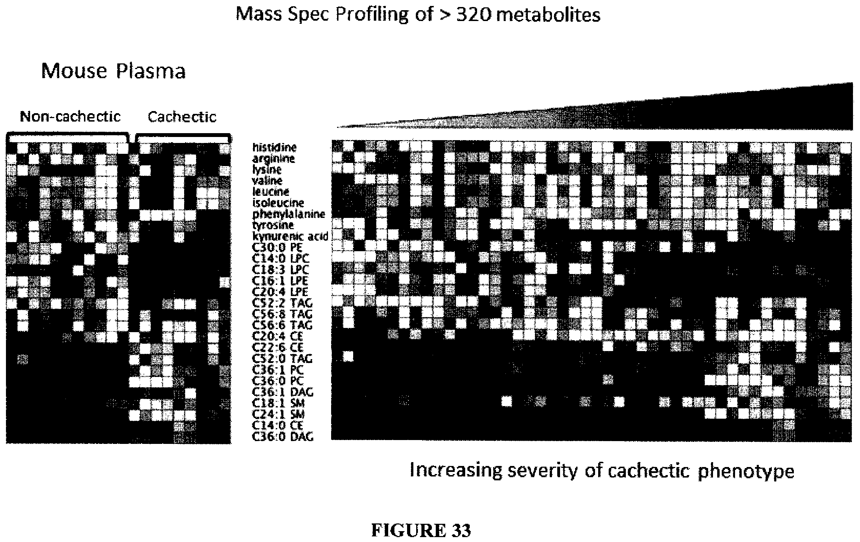

Figure 33 shows the Metabolite profiling of mouse plasma shows loss of branched chain AA (an occurrence in late stage cachexia), increased DAGs/TAGs seen clinically. The profiling was conducted via Mass Spectrometry of > 320 metabolites.

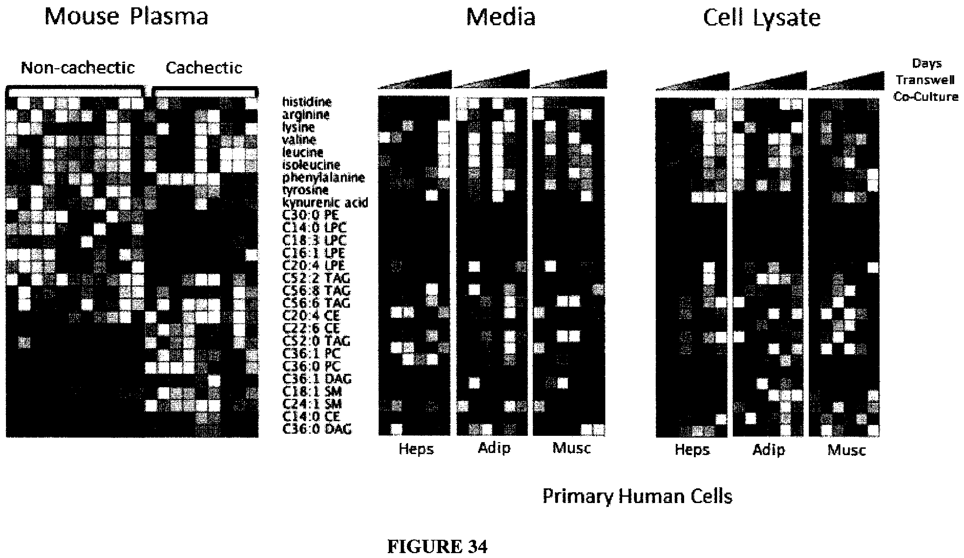

Figure 34 shows that the Metabolite profiling of *in vitro* models recapitulates many of the features of the *in vivo* response.
Figure 35 shows that a Drug Screen using Cachexia Gene Expression Signature identified a signaling pathway critical to the cachexia phenotype.
Figure 36 shows that MEK and ERK inhibitors block cachexia-inducing activity of conditioned media.
Figure 37 shows that RAGE ligands are more abundant in cachexia-inducing conditioned media.
Figure 38 illustrates that *in vitro* adipocytes generate relevant lipid metabolites in response to cachexia-inducing conditioned media. This indicates that the induction of cachexia may be targeted to providing certain tumors with the necessary metabolites to sustain growth independent of certain aspects of the cellular machinery establishing a feebback or sustenance cycle.

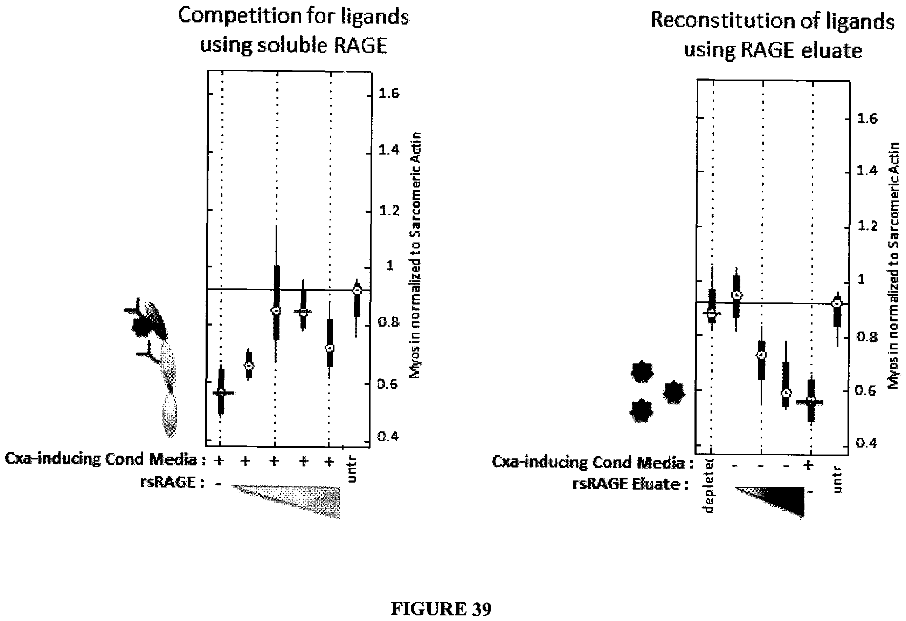

Figure 39 illustrates that soluble RAGE receptor depletes conditioned media of cachexia activity and the eluate reconstitutes cachexia activity further establishing a role for RAGE ligands in pre-cachexia and cachexia.

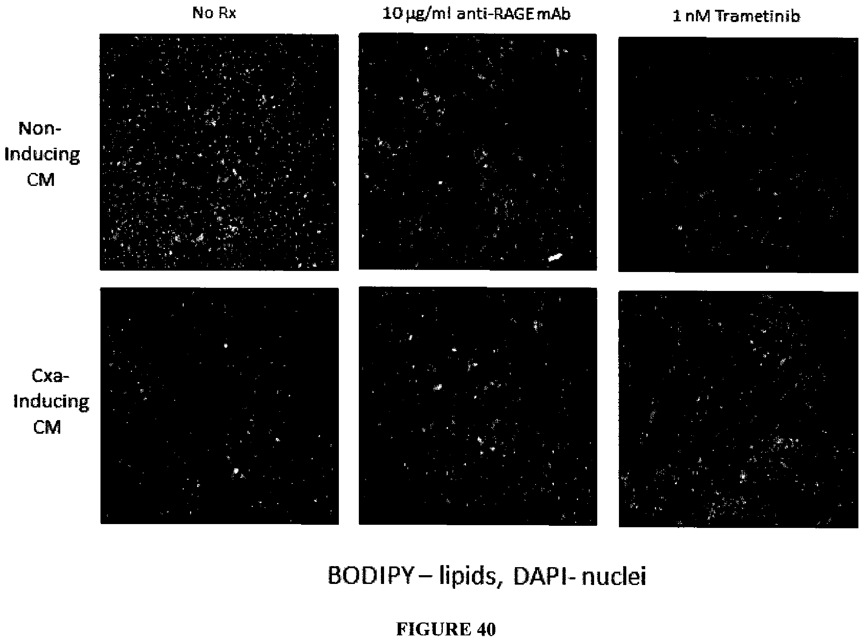

Figure 40 shows that inhibition of RAGE signaling inhibits lipid loss in adipocytes. Inhibition of RAGE signaling is carried out using and anti-RAGE mAb as well as Trametinib (a MEK inhibitor drug).
Figure 41 shows schematics for the *in vivo* validation of the RAGE pathway in cachexia, e.g. cancer cachexia, by targeting the RAGE Receptor by treatment with soluble RAGE receptor to out compete the endogenous receptor or by knocking out the RAGE receptor using an AAV-SaCas9 system.
Figure 42 shows schematics for the *in vivo* validation RAGE pathway in cachexia, e.g. cancer cachexia, by targeting the RAGE Ligands like the S100 ligands or by using an HdTVI transposon expression.

### DETAILED DESCRIPTION OF THE INVENTION

Disclosed herein are Applicants' novel findings as to the role of particular RAGE ligands in pre-cachexia and/or cachexia. Applicants have demonstrated for the first time the key involvement of specific RAGE ligands of the S100 family in inducing precachexia and/or cachexia in the context of several pathologies, and in particular cancer. Previous claims of cachexia as an indication for other anti-RAGE therapeutics have lumped cachexias as a treatment indication for anti-RAGE therapies based on (i) the incorrect assumption that cachexia is an inflammatory disease and (ii.) the assumption that any/all RAGE ligands cause cachexia. Key points include herein and discussed are:
- Clinically, cachexia does not manifest any of the cardinal symptoms of inflammation (e.g. swelling, erythema, pain, etc.)
- Clinical trials of proven anti-inflammatory therapies have all failed to even significantly ameliorate cachexia
- Applicants assayed over 300 human cytokines in cachectic vs non-cachectic patient plasma samples, and did not find significant correlations among inflammatory cytokines and cachexia (data not shown)
- Applicants prospectively tested 300 human cytokines individually and in combination and did not recapitulate the cachexic disease phenotype (data not shown)

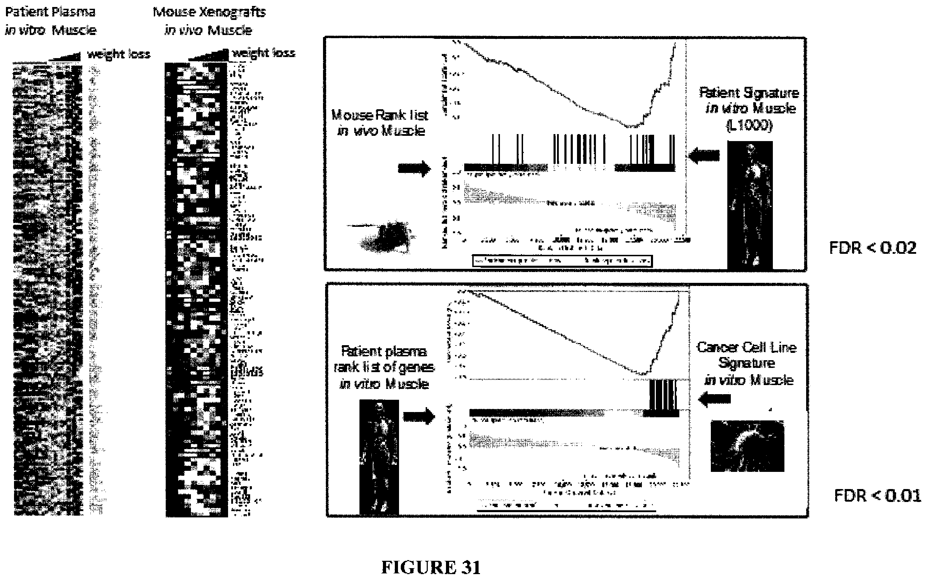

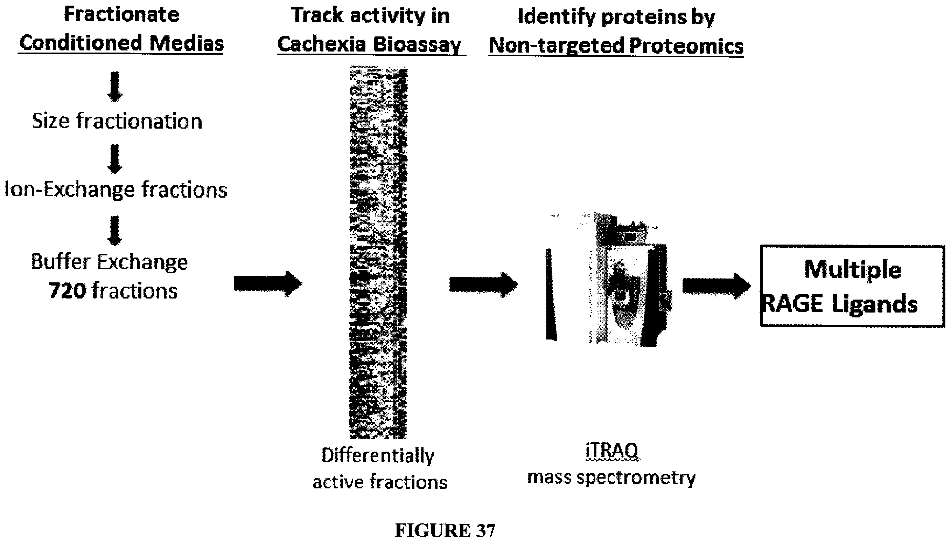

To date there has been little specificity defined for RAGE ligands and the receptor's numerous pleiotropic effects (mostly through HMGB1). Previously filed patents often base the effectiveness of their anti-RAGE therapeutics based on the demonstrated inhibition of RAGE binding to HMGB1, S100P, and/ or S100B. Applicants have shown that the RAGE ligands S100A7, S100A8, S100A9, and homodimers, heterodimers and heterotetramers of S100A8 and S100A9 (e.g., Calprotectin) are key effector molecules in inducing precachexia and/or cachexia. Applicants have discovered that S100A7, S100A8, and S100A9 are differentially secreted by cachexia-inducing cancer cells compared to cachexia non-inducing matched cancer cells (see **Figure** 37). Applicants have also discovered that RAGE gene expression is up-regulated in response to plasma from cachectic cancer patients but not to the plasma from non-cachectic cancer patients (see **Figure 31**).

Importantly, Applicants have discovered that high mobility group box 1 (HMGB1) and S100B do not induce precachexia and/or cachexia and that S100A7 and S100A8/A9 dimers directly bind to RAGE in direct immunoprecipitation experiments, which was not previously known. Applicants' novel findings open the door for effectively targeting RAGE in the context of precachexia and cachexia. Applicants disclose herein, *inter alia*, targeted and efficacious therapeutic engineered biologic compositions for specifically targeting RAGE in a subject at risk of developing pre-cachexia and/or cachexia or a subject suffering from pre-cachexia and/or cachexia. More specifically, the invention disclosed herein provides therapeutic, engineered protein/peptide compositions comprising e.g., anti-RAGE antibodies to target a RAGE receptor (including soluble forms thereof) directly and/or via differential competition with one or more pre-cachexia and/or cachexia-associated RAGE ligands or markers (e.g., a RAGE ligand of the S100 family chosen from S100A7, S100A8, and S100A9).

Notably, patents and published patent publications disclosing anti-RAGE antibodies, some of which are in clinical stage trials, appear to predominantly target HMGB1. Interestingly, a recent publication showed that HMGB1-RAGE signaling is actually important in maintaining muscle homeostasis. (Riuzzi et al. HMGB1-RAGE regulates muscle satellite cell homeostasis through p38-MAPK- and myogenin-dependent repression of Pax7 transcription. *J Cell Sci* 2012;125 1440-1454). As noted above, Applicants' functional data shows that HMGB1 is not cachexogenic in muscle. Applicants' gene signature of cachexia generated by treating primary human skeletal muscle with plasma from case-matched controlled cachectic vs non-cachectic cancer patients is highly specific (99.9 percentile) for the gene expression state analyzed from clinical muscle biopsies from cachectic vs non-cachectic cancer patients. This cachexia gene signature was also used successfully as the screen for drugs that could block and reverse cachexia through MEK/ERK inhibition (see **Figure 31**). It was demonstrated that ERK activation is downstream of RAGE activation. This demonstration by Applicants underscores the key need in the art for and the critical importance for the therapeutic biologic compositions (e.g., anti-RAGE antibodies, anti-RAGE T-cell receptors) disclosed herein, which inhibit a target RAGE receptor directly and/or via differential competition with one or more pre-cachexia and/or cachexia-associated RAGE ligands or markers RAGE ligands (e.g., S100A7, S100A8, S100A9, including homodimers, heterodimers and heterotetramers of S 100A8 and S 100A9).

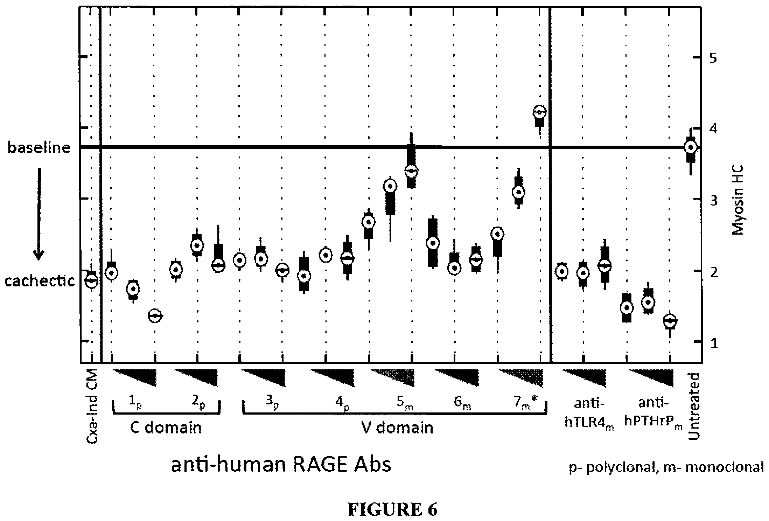

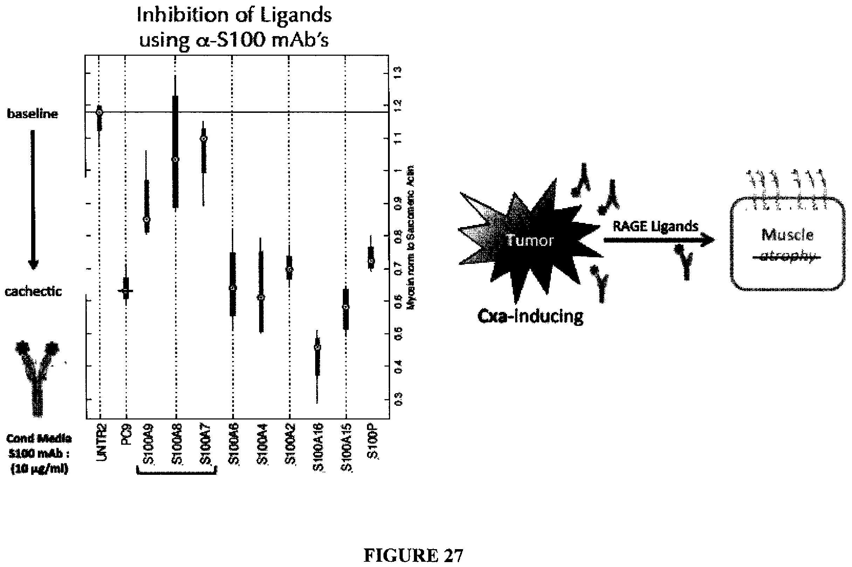

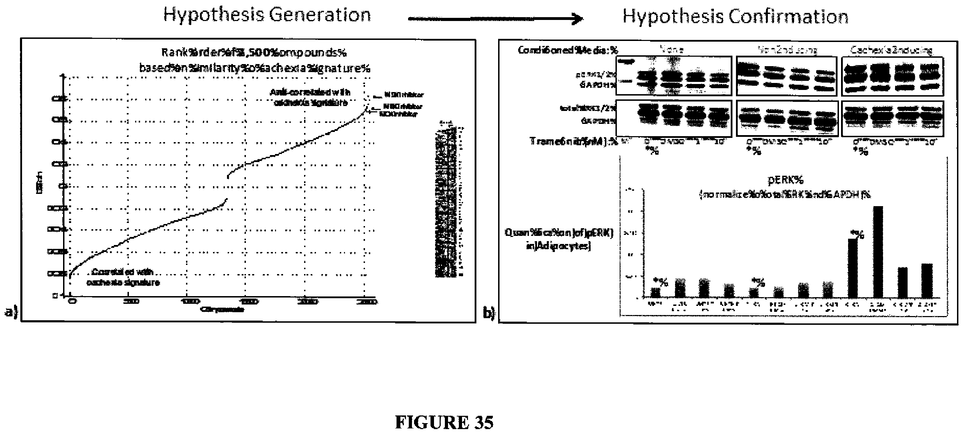

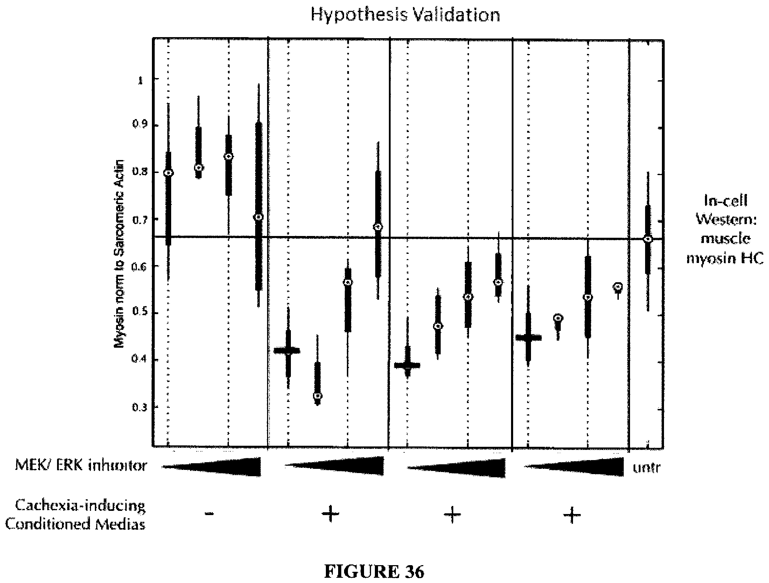

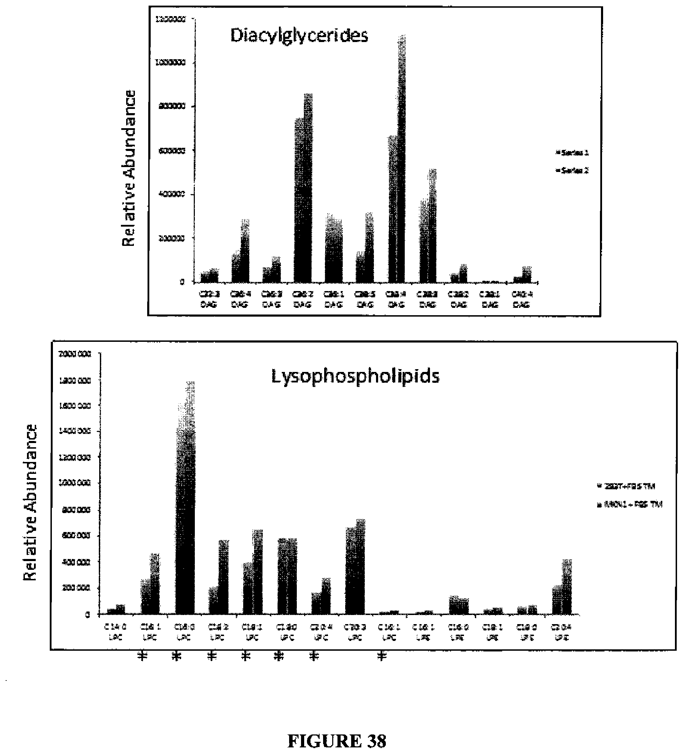

Applicants have determined that transcription of S100A7, S100A8, and S100A9 is highly coordinately regulated in primary cancers from patient biopsy samples (see a TCGA heat map known in the art, see also **Figure 27**). It was discovered that specific inhibition of S100A7, S100A8/A9 by neutralizing Abs, but not neutralizing Abs against other S100 proteins inhibited the disease phenotype (see **Figure 6**). Applicants demonstrated that specifically only recombinant S100A7 or S100A8/A9 (Calprotectin) are capable of inducing the cachexia phenotype while other active recombinant S100 and HMGB1 proteins cannot (see **Figure 27**). Applicants discovered that only specific anti-RAGE Abs (in total 4 out of 48 high affinity binding Abs) block the cachexia disease phenotype (see **Figure 6**; see **Figure 38**). Applicants discovered, discussed herein, using the cachexia gene signature that pathological MEK/ERK signaling is activated by RAGE, and the cachexia disease phenotype can be blocked and reversed with careful MEK/ERK inhibition (see **Figures 35****,** **36****,** and **38**.)S100 proteins are small (9-13 kDa) acidic regulatory metal-binding proteins and belong to a protein family consisting of at least 24 members functionally distributed into three main subgroups, (see e.g., Donato R. et al. Curr. Mol. Med. 2013 Jan;13(1):24-57. Functions of S100 proteins; Marenholz I. et al. Biochem. Biophys. Res. Comm. 2004 Oct 1;322(4):1111-22. S100 proteins in mouse and man: from evolution to function and pathology (including an update of the nomenclature)). The functional diversity of S100 proteins is extremely borad, partly due to their ability to adapt to various targets, (see e.g., Permyakov S.E. et al., Mol. BioSyst. 2011 Jul;7(7):2164-80. Intrinsic disorder in S100 proteins). The crystal structures of several S100 proteins are known. (see e.g., Brodersen D. E. et al. Biochemistry 1999 Feb 9;38(6):1695-704. Zinc-binding site of an S100 protein revealed. Two crystal structures of Ca2+-bound human psoriasin (S100A7) in the Zn2+-loaded and Zn2+-free states; Itou H. et al. J. Mol. Biol. 2002 Feb 15;316(2):265-76. The crystal structure of human MRP14 (S100A9), a Ca(2+)-dependent regulator protein in inflammatory process; Ishikawa K. et al., J. Acta Crystallographica Section D Biological Crystallography 2000 May;56(Pt 5):559-66. The structure of human MRP8, a member of the S100 calcium-binding protein family, by MAD phasing at 1.9 A resolution; Korndörfer I. P. et al. J. Mol. Biol. 2007 Jul 27;370(5):887-98. Epub 2007 May 3. The crystal structure of the human (S100A8/S100A9) heterotetramer, calprotectin, illustrates how conformational changes of interacting alpha-helices can determine specific association of two EF-hand proteins; Zhang H. et al. Mol. Biol. 2003 Jan 24;325(4):785-94. The crystal structure at 2A resolution of the Ca2+ - binding protein S100P.) Several S100 proteins bind and activate RAGE. (see e.g., Leclerc E. et al. Biochimica et Biophysica Acta 2009 Jun;1793(6):993-1007. Binding of S100 proteins to RAGE: an update; Koch M. et al. Structure 2010 Oct 13;18(10):1342-52. Structural basis for ligand recognition and activation of RAGE.).

Anti-RAGE antibodies are known; see, e.g., WO2008137552, WO2007109747, WO2010019656, WO2009136382, WO 2011053707, WO2011042548, US8,420,083. Anti-RAGE monoclonal antibodies are known; see, e.g., US 8,420,083, WO2011042548 and WO2008137552. However, these antibodies and monoclonal antibodies compete for binding with a range of RAGE ligands including HMGB1-contrary to and teaching away from, the instant invention. For instance, WO2008137552 specifically involves antibodies that inhibit binding between RAGE and HMGB1. As noted above, Applicant's functional data shows that HMGB1 in not cachexogenic in muscle and therefore HMGB1 is NOT a relevant target RAGE ligand for treating, preventing, alleviating or addressing pre-cachexia and/or cachexia. Accordingly, the anti-RAGE antibodies and monoclonal antibodies of the prior art: are not useful in the practice of the instant invention; fail to teach or suggest the present invention; and moreover, expressly teach away from the instant invention. Again, in embodiments, the present invention provides antibody(ies) specific for a RAGE receptor, which antibody(ies) competes for binding to the RAGE receptor with RAGE ligand(s) S100A7, S100A8, S100A9 and/or Calprotectin, but preferably may not compete for binding to the RAGE receptor with RAGE ligand HMGB1; for example, antibody(ies) that binds to epitope(s) on the RAGE receptor selected from epitope(s) which is / are bound by S100A7, S100A8, S100A9 or Calprotectin, but preferably may not bind to an epitope which is bound by the RAGE ligand HMGB1. The prior art teaches and directs towards antibodies that compete for binding to the RAGE receptor with RAGE ligand HMBG1, and hence, teach away from the instant invention; and the prior art does not teach or suggest antibody(ies) competes for binding to the RAGE receptor with RAGE ligand(s) S100A7, S100A8, S100A9 and/or Calprotectin, but preferably may not compete for binding to the RAGE receptor with RAGE ligand HMGB 1, and hence does not teach or suggest the instant invention.

Thus, as herein discussed, the invention involves an antibody specific for a RAGE receptor, which antibody competes for binding to the RAGE receptor with a RAGE ligand selected from S100A7, S100A8, S100A9 and/or Calprotectin, but preferably may not compete for binding to the RAGE receptor with RAGE ligand HMGB1. As noted above, Applicants disclose herein that S100A7 and S100A8/A9 dimers directly bind to RAGE in direct immunoprecipitation experiments. The antibody can be is selected from an IgG, IgA, or an antigen binding antibody fragment selected from an antibody single variable domain polypeptide, dAb, FAb, F(ab')₂, an scFv, an Fv, or a disulfide-bonded Fv. The antibody can binds to a RAGE receptor polypeptide with a dissociation coefficient of 1µm or less. The antibody can bind to a RAGE receptor polypeptide with a dissociation coefficient of 1nm or less. The antibody can bind to an epitope on the RAGE receptor selected from an epitope, which is bound by S100A7, S100A8, S100A9 or Calprotectin, but preferably may not bind to an epitope which is bound by the RAGE ligand HMGB1. The antibody can bind to a RAGE epitope bound by S100A7, S100A8 or S100A9 and/or calprotectin by mimicking the linear epitope defined by any one of residues 41-93 on S100A7, residues 36-89 on S100A8, or residues 45-98 on S100A9 (see Figure 6); or by mimicking the conformational epitope represented by helix A2 formed by residues 41-53 in S100A7, residues 43-49 in S100 A8 or residues 47-55 in S100A9; or which does not bind to the epitope defined by residues 43-78 in (S100B). The antibody can bind to residues 41-93 in (S100A7), residues 36-89 in (S100A8) or residues 45-98 in (S100A9), S100A15 or Calprotectin. The antibody binds to a linear epitope which is selected from the linear epitope defined by residues 41-93 in (S100A7), residues 36-89 in (S100A8), residues 45-98 in (S100A9) or residues 43-78 in (S100B), and does not bind to the S100B epitope defined by residues 43-78 in (S100B), or which binds to the conformational epitope formed by residues 41-53 on S100A7, residues 43-49 on S100A8, residues 47-55 on S100A9 or residues 40-53 on S100A15 and does not bind to the conformational epitope formed by residues 43-78 on S100B.

As referred to herein, an "epitope" can be a linear epitope, comprising a sequence of contiguous amino acids, or conformational, formed by the three-dimensional juxtaposition of amino acids and/or glycosyl groups in the tertiary structure of a protein. An epitope can comprise or consist of as few as at least 6 amino acids that are unique to a polypeptide or protein sequence. An epitope can be longer; for example, an epitope can comprise, consist essentially of or consist of at least 6 or at least 8, or at least 10, or at least 12, or at least 15 or at least 20 amino acids.

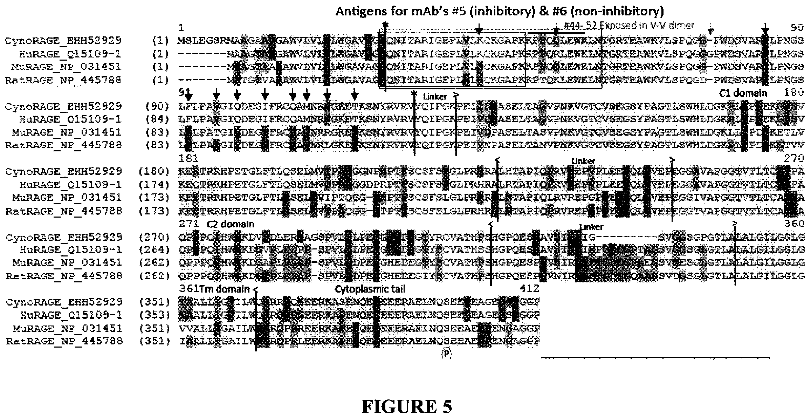

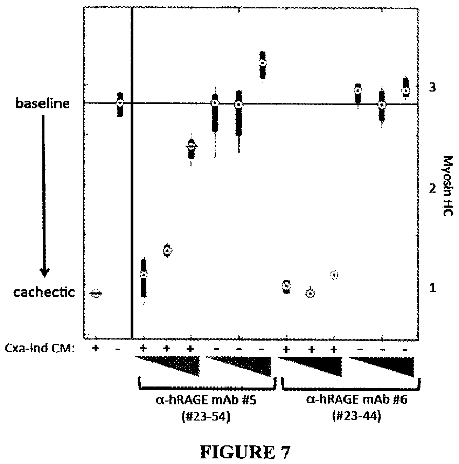

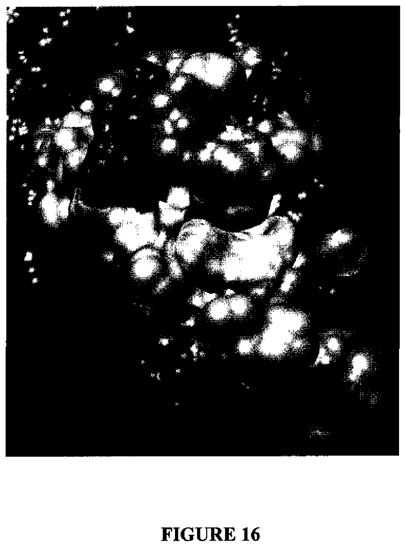

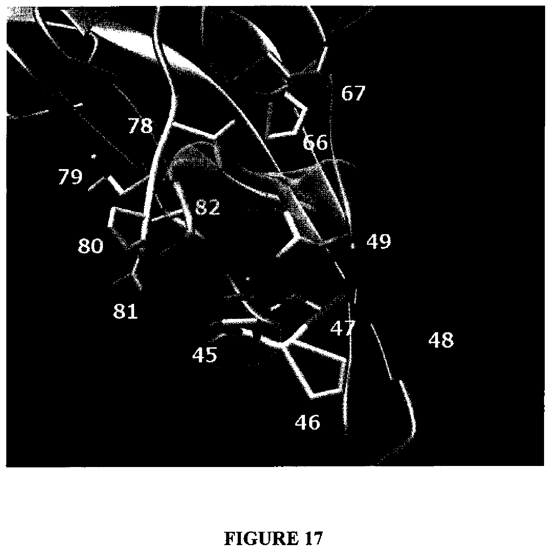

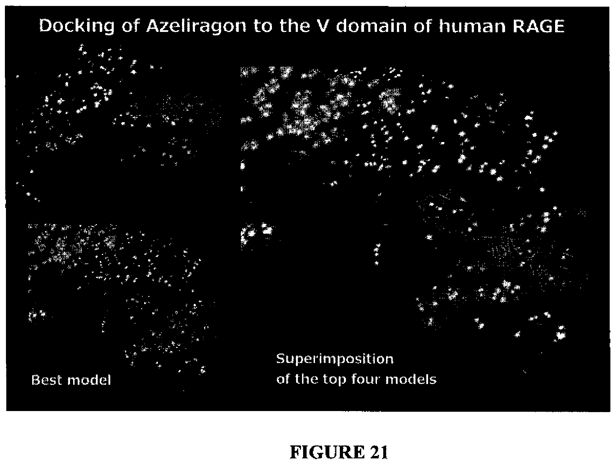

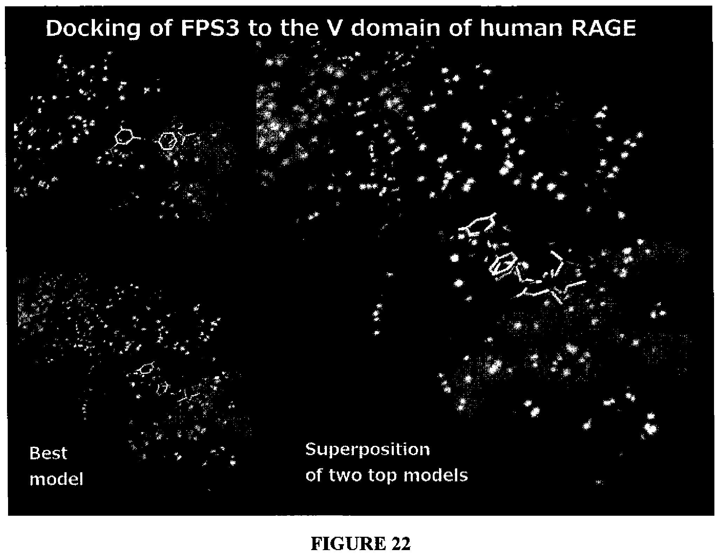

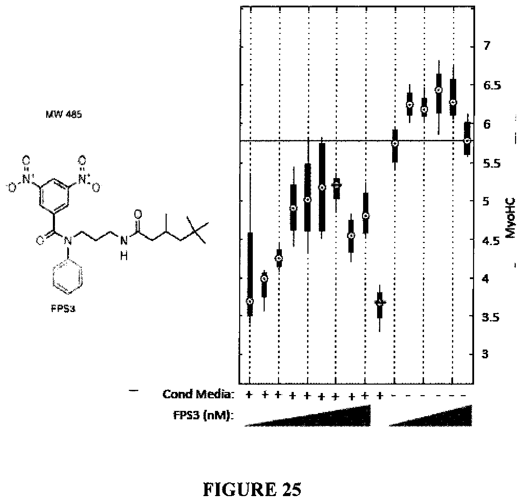

The mechanism by which RAGE signaling is activated in cachexia was previously unknown. Applicants have defined the epitope on RAGE that when inhibited by RAGE Abs or RAGE small molecule compounds/ligands results in the inhibition of the cachexia phenotype by functional determination of anti-RAGE Abs and structural mapping (see **Figure 7**.) As shown in **Figures 16** and **17**, Applicants have determined that the inhibitory epitope contains a small molecule binding "hot spot." Consequently, Applicants have discovered several small molecule "drugs" known to bind preferentially to Applicants' defined inhibitory epitope of RAGE (see **Figure 21** and **Figure 22**) and results in functional inhibit of cachexia (see **Figure 25**). We have determined the preferential binding sites of S100A7 and S100A8/A9 overlap on RAGE and are unique compared with other S100 proteins (see **Figure 5**)

S100, including S100A7, S100A8, S100A9 and S100B, is, in embodiments of the invention, human S100. Likewise, HMBG1 is, in embodiments of the invention, human HMGB1. Sequences of human S100 proteins, as well as HMBG1, can be located in the UniProt database under accession numbers P31151, P05109, P06702, P33763, P06703, P29034, P25815, P23297, P04271, Q99584 and P80511.

The invention also encompasses sequences which are homologous the the database sequences recited above. In one embodiment, the sequences according to the invention are at least 95% homologous the sequences referred to above. In other embodiments, they are at least 96%, 97%, 98%, 99% or 100% homologous. Homology can be assessed using any suitable sequence alignment technique. Optimal alignment may be detemined with the use of any suitable algorithm for aligning sequences, non-limiting examples of which include the Smith-Waterman algorithm, the Needleman-Tunsch algorithm, algorithms based on the Burrows-Wheeler Transfom (e.g. the Burrcnvs Wheeler Aligner), ClustalW, Clustal X, BLAST, Novoalign (Novocraft Technologies, ELAND (Illumina, San Diego, CA), SOAP (available at soap.genomics.org.cn), and Maq (available at maq.sourceforge.net).

The invention comprises a method of treating pre-cachexia and providing treatment to patients in need thereof, the method comprising administering to the subject an effective amount of one or more agents that is an anti-RAGE antibody, a RAGE blocking peptide or small molecule that inhibits binding of RAGE to ligands.

The current invention includes antibodies to the RAGE exposed V-domain for the prevention and or treatment of pre-cachexia. In line with the inventions of the application, various combinations of 1) antibodies to the RAGE exposed V-domain, 2) antibodies to the S100A7, A8, A9, A14, A1, and 3) antibodies to the INSR/MYH are possible and effective for the prevention and or treatment of pre-cachexia. Different patients may have different combinations of these diagnostic and pharmacodynamics biomarkers. Aspects of the invention encompass identifying patients as pre-cachetic or cachectic based on these different combinations and providing them with subsequent treatment. The diagnostioc biomarkers include S100A7/8/9 (which may be considered the "driver" biomarkers), S100A1 and A14 (which may be considered as passenger or highly correlated biomarkers) and the pharmacodynamics biomarkers include MYH1/2/3/6/7s in addition to metabolomic profile of BCAA/ lipid metabolites. Any one of the above antibodies may be also combined with antibodies to MYH1, MYH2, MYH7 and MYH7B for the prevention and/or treatment of pre-cachexia related to skeletal or cardiac muscle cachexia.. Of these, S100A7 is important, as is S100A8 and S00A9. Antibodies to S100A7 can be combined with Antibodies to S100A8 and/or S100A9. S100A14 and S100A1 are of interest. And hence antibodies against S100A7 with antibiodies against S100A1 and/or S100A14, alone or in combination with antibodies against S100A8 and/or S100A9. Also, it is observed that as to pre-cachexia and cachexia, the Soluble Insulin Receptor (INSR) is increased across the board. From the foregoing, increased levels of S100A7, especially with increased levels of S100A8 and/or S100A9 may be important markers for observing pre-cachexia. Increased levels of INSR and/or MYH6 may be important markers too. Pre-cachexia patients may be observed though a combination of increased levels of S100A7, INSR, and MYH6 or S100A7, S100A8, INSR, and MYH6 or S100A7, S100A8, S100A9, INSR, and MYH6. With these can be increased levels of S100A1 and/or S100A14. Accordingly, pre-cachexia may be defined by upregulation of S100A7, S100A8, S100A9, S100A14 and INSR and optionally also MYH6, alone or in further combination with upregulation of S100A1 and/or S100A14.

The invention thus envisions S100A7 and INSR, and combinations thereof, for diagnosis of pre-cachexia. The invention also envisions S100A7 and S100A8/A9 dimers directly bind to RAGE. Patients may be assayed for biomarker levels, wherein the biomarkers may be different combinations of S100A7/8/9, S100A1 and A14, and MYH1/2/3/6/7s further to the metabolomic profile of BCAA/ lipid metabolites. Diagnosis includes diagnosis in the context of in vitro assays. The invention also envisions S100A7, INSR, MYH6, MYH7, MYH7B for diagnosis of cardiac pre-cachexia. The invention further comprehends upregulation of at least two of S100A7, S100A8, S100A9, MYH1, MYH2, MYH3, MYH6, MYH7, MYH7B and/or INSR, alone or in further combination with S100A1 and/or S100A14 for diagnosis of pre-cachexia. Diagnosis includes diagnosis in the context of in vitro assays. For example, the antibodies or small molecules of the invention may be used for the prevention and or treatment of cardiac pre-cachexia, wherein cardiac pre-cachexia is defined by upregulation of S100A7, INSR, and cardio specific MYH6 in plasma. The antibodies or small molecules of the invention may be used for the prevention and or treatment of cardiac pre-cachexia, wherein cardiac pre-cachexia is defined by upregulation of S100A7, INSR, and cardio specific MYH6.

The antibodies or small molecules of the invention may be used for the prevention and or treatment of pre-cachexia, wherein pre-cachexia is defined by upregulation of at least two of S100A7, S100A8, S100A9, MYH1, MYH2, MYH3, MYH6, MYH7, MYH7B and/or INSR. The antibodies or small moleucles of the invention may be used for the prevention and or treatment of pre-cachexia, wherein pre-cachexia is defined by upregulation of S100A7, S100A8, S100A9, S100A14, and/or INSR. The antibodies or small moleucles of the invention may be used for the prevention and or treatment of pre-cachexia, wherein pre-cachexia is defined by upregulation of at least two of S100A7, S100A8, S100A9, MYH1, MYH2, MYH3, MYH6, MYH7, MYH7B and/or INSR and optionally also S100A14 and/or S100A1. A practioner in applying any aspects of the invention may consider an increase in the level of at least two biomarkers in conjunction with evidence of pre-cachexia (unexpected weight loss %, MYH release or degradation, lipolysis or increased BCAA levels).

The markers for determining or identifying pre-cachexia discussed herein, e.g., upregulation of at least two of S100A7, S100A8, S100A9, MYH1, MYH2, MYH3, MYH6, MYH7, MYH7B and/or INSR, alone or in further combination with upregulation of S100A1 and/or S100A14, can also be combined with other diagnostic means for determining or identifying whether a patient may have a cardiac or heart condition; and, the treatments herein as to treating or inhibiting the progression of pre-cachexia can be used in combination with treatments for cardiac or heart conditions, especially in view of cardiac cachexia. Likewise, markers for determining or identifying pre-cachexia discussed herein, e.g., upregulation of at least two of S100A7, S100A8, S100A9, MYH1, MYH2, MYH3, MYH6, MYH7, MYH7B and/or INSR, alone or in further combination with upregulation of S100A1 and/or S100A14, can also be combined with other diagnostic means for determining or identifying whether a patient may have a cancer; and, the treatments herein as to treating or inhibiting the progression of pre-cachexia can be used in combination with treatments for cancer, especially in view of cancer cachexia. Similarly, markers for determining or identifying pre-cachexia discussed herein, e.g., upregulation of at least two of S100A7, S100A8, S100A9, MYH1, MYH2, MYH3, MYH6, MYH7, MYH7B and/or INSR, alone or in further combination with upregulation of S100A1 and/or S100A14, can also be combined with other diagnostic means for determining or identifying whether a patient may have COPD; and, the treatments herein as to treating or inhibiting the progression of pre-cachexia can be used in combination with treatments for COPD, especially in view of cancer cachexia. Accordingly, if upregulation of at least two of S100A7, S100A8, S100A9, MYH1, MYH2, MYH3, MYH6, MYH7, MYH7B and/or INSR, alone or in further combination with upregulation of S100A1 and/or S100A14 is observed in a patient, and the patient otherwise does not present symptoms as to a cardiac or heart condition, cancer, COPD or any other condition associated with cachexia, the patient may be further screened to determine whether the patient is prone or susceptible to or has a yet undiagnosed cardiac or heart condition, cancer, COPD or other condition associated with cachexia; and thus, the markers herein- upregulation of at least two of S100A7, S100A8, S100A9, MYH1, MYH2, MYH3, MYH6, MYH7, MYH7B and/or INSR, alone or in further combination with upregulation of S100A1 and/or S100A14 observed in a patient-may be sign of an individual prone or susceptible to or having a yet undiagnosed cardiac or heart condition, cancer, COPD or other condition associated with cachexia, and the treatments herein discussed may be administered to such a patient, e.g., to treat or prevent or inhibit progression of cachexia as well as to treat or prevent or inhibit progression of cardiac or heart condition, cancer, COPD or other condition associated with cachexia.

The invention comprehends a method of inhibiting the progression of pre-cachexia to cachexia in a subject, the method comprising administering to the subject an effective amount of one or more agents that is an anti-RAGE antibody, a RAGE blocking peptide or small molecule that inhibits binding of RAGE to ligands.

The invention comprehends a method of treating or preventing undesirable muscle or fat loss in a cancer patient in need of treatment thereof, the method comprising administering to the subject an effective amount of one or more agents that is an anti-RAGE antibody, a RAGE blocking peptide or small molecule that inhibits binding of RAGE to ligands.

The method can involve wherein: the subject is pre-selected as having a molecular signature indicative of pre-cachexia or cachexia by detecting an alteration in at least three markers selected from the group consisting of S100A2 or S100A4; S100A8 or S100A9; and S100A7; detecting an alteration in at least four markers selected from the group consisting of: S100A2 or S100A4; S100A8 or S100A9; S100A7; and S100A14; and/or measuring the level of at least five markers selected from the group consisting of : S100A2 or S100A4; S100A8 or S100A9; S100A7; S100A14; and S100P, thereby pre-selecting the patient as having a molecular signature indicative of pre-cachexia or cachexia. The anti-RAGE antibody can be an antibody which competes for binding to the RAGE receptor with a RAGE ligand selected from S100A7, S100A8, S100A9 and Calprotectin, but preferably may not compete for binding to the RAGE receptor with RAGE ligand HMGB1. The anti-RAGE antibody is an antibody as herein disclosed.

The invention also comprehends a method of inhibiting the loss of myosin heavy chain in a myocyte, the method comprising contacting the myocyte with an effective amount of one or more agents that is an anti-RAGE antibody, a RAGE blocking peptide or small molecule that inhibits binding of RAGE to ligands.

The invention also comprehends a method of inhibiting lipolysis in an adipocyte, the method comprising contacting the adipocyte with an effective amount of one or more agents that is an anti-RAGE antibody, a RAGE blocking peptide or small molecule that inhibits binding of RAGE to ligands.

The invention also involves a method of inhibiting atrophy in a cell, the method comprising contacting the adipocyte, myocyte, or cardiomyocyte with an effective amount of one or more agents that is an anti-RAGE antibody, a RAGE blocking peptide or small molecule that inhibits binding of RAGE to ligands.

In inventive methods, the myocyte, adipocyte, or cell is *in vitro* or *in vivo.* The myocyte, adipocyte, or cell can be present in a subject identified as having at least one cancer. The cancer includes but is not limited to one or more carcinomas and sarcomas and cancers of the skin, pancreas, stomach, colon, thorax, liver, gallbladder, musculoskeletal system, breast, lung, ovary, uterus, endometrium, prostrate, colon, skin, mouth, salivary, esophagus, head and neck, plus other tumors of the gastrointestinal tract.

The anti-RAGE antibody can be an antibody, which competes for binding to the RAGE receptor with a RAGE ligand selected from S100A7, S100A8, S100A9 and Calprotectin, but preferably may not compete for binding to the RAGE receptor with RAGE ligand HMGB1 or A100B. The anti-RAGE antibody is an antibody as herein disclosed.

The disclosure additionally involves a method of enhancing cancer sensitivity to chemotherapy, the method comprising administering gemcitabine in combination with an anti-RAGE therapy selected from the group consisting of an anti-RAGE antibody, a RAGE blocking peptide, or a small molecule that inhibits binding of RAGE to ligands, thereby enhancing cancer sensitivity to chemotherapy. The cancer can be selected from the group consisting of one or more carcinoma or sarcoma, including but not limited to cancers of the skin, pancreas, stomach, colon, thorax, liver, gallbladder, musculoskeletal system, breast, lung, ovary, uterus, endometrium, prostrate, colon, skin, mouth, salivary, esophagus, head and neck, plus other tumors of the gastrointestinal tract.

The invention also comprehends a non-naturally-occurring or engineered B-cell that expresses an antibody of the invention.

The invention also comprehends a vector expressing an antibody or binding fragment thereof of the invention.

In inventive methods the anti-RAGE antibody can be administered by administering a vector that contains nucleic acid molecule(s) encoding and expresses the antibody or a binding fragment thereof.

The disclosure comprehends a method of identifying pre-cachexia in a subject wherein an epitope surface binds to a compound of Formula (I): 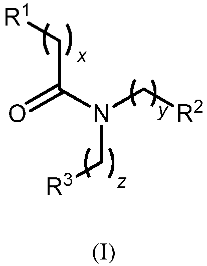
wherein R¹ is selected from the group consisting of -(CH₂)ₐ-NR⁵R⁶, -(CH₂)ₐ-NR⁵C(O)R⁶, a three to twelve-membered aromatic ring, a three- to twelve-membered heterocyclic ring;
each of which may be optionally substituted from 1 to 3 substituents independently selected from -Cl, -Br, -NO₂, -C(O)H, -COOH, -COOR⁴, C(O)R⁴,-C(O)Cl, -CF₃, -CCl₃, -CN, - SO₃H, -NH₃⁺, -N(R⁴)₃³⁺;
R⁴ is selected from a group consisting of C₁-C₆ alkyl, -(C₁-C₆ alkyl)-O-(C₁-C₆ alkyl), -(C₁-C₆ alkyl)-;
R² is selected from the group consisting of a C₃-C₇ cycloalkyl, C₁-C₆ alkyl, -(CH₂)ₐ-NR⁵R⁶, -NHC(O)-Y-R⁵, -NHC(O)CHR⁵R⁶, -CHR⁵R⁶;
Y is selected form a covalent bond, O, NH, and C₁-C₆ alkyl;
R³ is selected from the group consisting of a three- to twelve-membered aromatic ring, a three- to twelve-membered heterocyclic ring,
each of which may be optionally substituted from 1 to 3 substituents independently selected from -C₁-C₆ alkyl, -F, -OC(O)alkyl, -CH=CH₂, -(C₁-C₆ alkyl)-O-(C₁-C₆ alkyl), -OH, - NH₂, -NR⁵R⁶, -(CH₂)ₐ-NR⁵R⁶, -Y-R⁵;
Y is selected from a covalent bond, O, NH, and C₁-C₆ alkyl;
R⁵ and R⁶ are independently selected from the group consisting of -H, -C₁-C₈ straight chain alkyl, -C₁-C₈ branched alkyl, -C₂-C₈ alkenyl, C₂-C₈ alkynyl, -(C₁-C₆ alkyl)-O-(C₁-C₆ alkyl) each of which is optionally substituted by a halogen, ether, vinyl group, allylic group, -NH₂, or - CN, -(CH₂)ₐNR⁵⁵R⁵⁶, -(CH₂)ₐ-C(O)NR⁵⁵R⁵⁶, an aromatic group, heteroaromatic group, C₃-C₇ cycloalkyl, a three to twelve membered heterocyclic having up to 3 heteroatoms each of which preceding cyclic group is optionally substituted from 1 to 3 substituents independently selected from a halogen, C₁-C₆ alkyl, C₂-C₆ alkenyl, -O(C₁-C₆ alkyl), -C(O)-, -OH, -NH₂, -CN, and C₁-C₃ perfluoro alkyl;
or R⁵ and R⁶ may be taken together to form a three to twelve membered heterocyclic having up to 3 heteroatoms which is optionally substituted from 1 to 3 substituents independently selected from a halogen, C₁-C₆ alkyl, C₂-C₆ alkenyl, -O(C₁-C₆ alkyl), -C(O)-, -OH, -NH₂, -CN, and C₁-C₃ perfluoro alkyl;
R⁵⁵ and R⁵⁶ are independently selected from the group consisting of -H, -C₁-C₈ straight chain alkyl, -C₁-C₈ branched alkyl, -C₂-C₈ alkenyl, C₂-C₈ alkynyl, -(C₁-C₆ alkyl)-O-(C₁-C₆ alkyl);
a is independently any integer between 0 and 6;
x is independently any integer between 0 and 6;
y is independently any integer between 0 and 6;
z is independently any integer between 0 and 6.

In another embodiment, the method identifies pre-cachexia according to any one of claims X in a subject wherein an epitope surface binds to a compound of Formula (I):

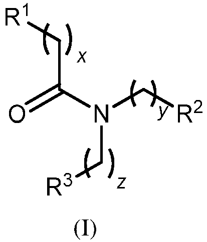

wherein R¹ is selected from the group consisting of -(CH₂)ₐ-NR⁵C(O)R⁶, a three to twelve-membered aromatic ring, a three- to twelve-membered heterocyclic ring;
each of which may be optionally substituted from 1 to 3 substituents independently selected from -Cl, -Br, -NO₂, -C(O)H, -COOH, -COOR⁴, C(O)R⁴,-C(O)Cl, -CF₃, -CCl₃, -CN, - SO₃H, -NH₃⁺, -N(R⁴)₃³⁺;
R⁴ is selected from a group consisting of C₁-C₆ alkyl, -(C₁-C₆ alkyl)-O-(C₁-C₆ alkyl), -(C₁-C₆ alkyl)-;
R² is selected from the group consisting of a C₃-C₇ cycloalkyl, -(CH₂)ₐ-NR⁵R⁶, - NHC(O)-Y-R⁵, -NHC(O)CHR⁵R⁶;
Y is selected form a covalent bond, O, NH, and C₁-C₆ alkyl;
R³ is selected from the group consisting of a three- to twelve-membered aromatic ring, a three- to twelve-membered heterocyclic ring,
each of which may be optionally substituted from 1 to 3 substituents independently selected from -C₁-C₆ alkyl, -F, -OC(O)alkyl, -CH=CH₂, -(C₁-C₆ alkyl)-O-(C₁-C₆ alkyl), -OH, - NH₂, -NR⁵R⁶, -(CH₂)ₐ-NR⁵R⁶, -Y-R⁵;
Y is selected from a covalent bond, O, NH, and C₁-C₆ alkyl;
R⁵ and R⁶ are independently selected from the group consisting of -H, -C₁-C₈ straight chain alkyl, -C₁-C₈ branched alkyl, -C₂-C₈ alkenyl, C₂-C₈ alkynyl, -(C₁-C₆ alkyl)-O-(C₁-C₆ alkyl) each of which is optionally substituted by a halogen, ether, vinyl group, allylic group, -NH₂, or - CN, -(CH₂)ₐNR⁵⁵R⁵⁶, -(CH₂)ₐ-C(O)NR⁵⁵R⁵⁶, an aromatic group, heteroaromatic group, C₃-C₇ cycloalkyl, a three to twelve membered heterocyclic having up to 3 heteroatoms each of which preceding cyclic group is optionally substituted from 1 to 3 substituents independently selected from a halogen, C₁-C₆ alkyl, C₂-C₆ alkenyl, -O(C₁-C₆ alkyl), -C(O)-, -OH, -NH₂, -CN, and C₁-C₃ perfluoro alkyl;
or R⁵ and R⁶ may be taken together to form a three to twelve membered heterocyclic having up to 3 heteroatoms which is optionally substituted from 1 to 3 substituents independently selected from a halogen, C₁-C₆ alkyl, C₂-C₆ alkenyl, -O(C₁-C₆ alkyl), -C(O)-, -OH, -NH₂, -CN, and C₁-C₃ perfluoro alkyl;
R⁵⁵ and R⁵⁶ are independently selected from the group consisting of -H, -C₁-C₈ straight chain alkyl, -C₁-C₈ branched alkyl, -C₂-C₈ alkenyl, C₂-C₈ alkynyl, -(C₁-C₆ alkyl)-O-(C₁-C₆ alkyl);
a is independently any integer between 0 and 6;
x is independently any integer between 0 and 6;
y is independently any integer between 0 and 6;
z is independently any integer between 0 and 6.

In an embodiment, a method for treating treating pre-cachexia is provided in a subject in need thereof with a therapeutically effective amount of at least one compound according to the Formula I which associates with an epitope on a RAGE receptor or a pharmaceutically acceptable salt thereof.

In a further embodiment, the method further provides a compound according to Formula I wherein the compound associates with a V domain of the RAGE receptor.

In an embodiment, the method provides a compound according to Formula I wherein the V domain is exposed on V-V dimerization of the RAGE receptor.

In a further embodiment, the method further provides a compound wherein the compound associates with amino resides 44-52 corresponding to the sequence numbering of human RAGE receptor.

In another embodiment, the method provides a compound wherein the compound associates with amino resides KPPQRLEWK of human RAGE receptor.

### Anti-RAGE antibodies

The present invention provides antibodies that bind specifically to RAGE, including soluble RAGE and endogenous secretory RAGE, and competitively inhibit binding of RAGE to one or more specific RAGE ligands, in particular RAGE ligands of the S100 family. Antibodies can be expressed in various ways, as herein discussed. Phage display and yeast systems, as well as other vector systems, e.g., vectors for *in vivo* expression, are amongst the ways to produce antibodies. See generally "Therapeutic Monoclonal Antibodies: From Bench to Clinic," edited by Zhiqiang An (Wiley 2009) (available from Google Books), e.g., chapters 8, 9, 10, 11, 12 concerning phage, yeast, ribosomal, bacterial and mammalian antibody display systems (for instance sections 9.2, 9.4 9.6, 9.6, 9.6.1.1, 9.6.1.2 concerning yeast display, tumor applications, and intracellular applications), and the documents cited therein.

The invention includes anti-RAGE antibodies that bind specifically to RAGE-expressing cells *in vitro* and in *vivo*, and antibodies that bind to human RAGE with a dissociation constant (Kd) in the range of from at least about 1 x 10⁻⁷ M to about 1 x 10⁻¹⁰ M. Also Included are anti-RAGE antibodies of the invention that bind specifically to the V domain of human RAGE, and anti-RAGE antibodies that block the binding of RAGE to an S100 RAGE ligand.

Also included are anti-RAGE antibodies of the invention that bind specifically to the V domain of human RAGE. The invention defines particularly useful antibodies which exhibit specific binding to amino acids of the V domain which are unexpectedly, exposed on V-V dimerization (exposed V-domain in V-V dimer). For example, antibodies which exhibit specific binding to the linear epitope of amino acids 44 - 52 of RAGE (Cyno RAGE: KPPQQLEWK; HuRAGE: KPPQRLEWK; MuRAGE KPPQQLEWK; RatRAGE: KPTQKLEWK). Likewise, antibodies which exhibit specific binding to the conformational epitope of amino acids 44 - 52 of RAGE (Cyno RAGE: KPPQQLEWK; HuRAGE: KPPQRLEWK; MuRAGE KPPQQLEWK; RatRAGE: KPTQKLEWK). Such antibodies provide the unexpected result of specific inhibition of ligand binding to the V domain.. Such specific inhibition results in treatment of pre-cachexia and/or cachexia. Preferred are monoclonal antibodies which exhibit specific binding to the RAGE 44 - 52 conformational epitope, and in particular human RAGE 44 - 52. In aspects of the invention, the V-V domain dimers of RAGE block aa#23-43 leaving only aa#44-52 solvent exposed for binding to the antibodies and small molecules of the invention.

Also included in the invention is an antibody that binds specifically to RAGE and blocks the binding of RAGE to RAGE ligands such as S100A7, S100A8, S100A9 and/or Calprotectin.

Also included in the invention are antibodies which result in a return of values to baseline levels in an appropriate cachexia *in vitro* assay, as well as an assay to identify such antibodies. Appropriate cachexia *in vitro* assays are any in vitro assays which reflect inducible cachetic conditions *in vitro* (e.g. cachexia-inducing media, cachetic patient plasma; S100A7, S100A8, S100A9). Exemplary cell types for such *in vitro* assays are hepatocytes, apidocytes, or muscle cells. Preferred are primary skeletal muscle cells, e.g. HsKM cells. A preferred marker for *in vitro* cachetic condition is the loss of myosin heavy chain; a preferred marker for demonstrating a return to baseline. Cachetic conditions may be defined as conditions reflective of changes observed in cells *in vitro* in response to cachexia inducing factors (e.g., the cachectic gene expression signature, loss of myosin heavy chain, loss of lipid content, and cell atrophy). For example levels which are no more than 75%, no more than 70%, no more than 65% of control values may be considered as an indicator of a cachetic condition *in vitro.* Preferred is no more than 70%. Most preferred is no more than 65%. Preferred control is loss of myosin heavy chain. A return to baseline is reflective of changes observed in cells *in vitro* in response to cachexia inducing factors which are then reversed to conditions which are comparable to conditions of non-induced controls (e.g., the cachectic gene expression signature, loss of myosin heavy chain, loss of lipid content, and cell atrophy). A return to baseline levels include values of at least 75%, at least 80%, at least 90%, at least 95% and 100% as compared to the control (i.e. not cachetic induced). Preferred is at least 90%, more preferred is at least 95% return to baseline. In terms of upregulation or increase in biomarker levels, more than a 30% increase over baseline or normal population reference standard may indicate a pre-cachextic or cachextic state.

For example, primary skeletal muscle cells may be cultured *in vitro.* These cells may be exposed to cachectic inducing conditions (e.g. S100A7). Control cells are those which are not exposed to such conditions. Cachetic conditions may be reached when there is a 75% decrease / loss of the myosin heavy chain as compared to the control baseline. Once applying an antibody of the invention, the 75% decrease / loss of the myosin heavy chain as compared to the control baseline may be reversed and the myosin heavy chain levels return to levels of at least 90% of the original baseline levels.

An example of an antibody of the invention is that which binds to the RAGE V domain, in particular the RAGE exposed V domain when there is V-V dimerization. These criteria may be combined to further define the antibodies of the invention (e.g. binding of the antibody to the RAGE V domain while at the same time demonstrating a return of myosin heavy chain levels to 90% of the baseline). The same applies for small molecules of the invention (i.e. application of the small molecule and a return of the myosin heavy chain values to baseline; small molecules that bind to the RAGE V domain, in particular the RAGE exposed V domain when there is V-V dimerization; the combination thereof).

The antibodies of the invention are further specifically defined as exhibiting specific binding to the RAGE V domain while also exhibiting a return to baseline levels in an in vitro cachetic assay. For example, the antibodies exhibit specific binding to the RAGE V domain while also exhibiting a return of loss of myosin heavy chain to baseline levels in an *in vitro* assay using S100A7 to induce cachetic conditions.

The antibodies of the invention are further specifically defined as exhibiting specific binding to the RAGE exposed V domain in V-V dimer while also exhibiting a return to baseline levels in an in vitro cachetic assay. For example, the antibodies exhibit specific binding to the RAGE exposed V domain in V-V dimer while also exhibiting a return of loss of myosin heavy chain to baseline levels in an in vitro assay using S100A7 or credentialed cachexia-inducing human cancer cell line conditioned media (based on cachectic gene expression signature or spefic loss of MYH in primary human in vitro muscle to induce cachetic conditions.

The anti-RAGE antibodies of the invention include an anti-RAGE antibody as described above, which binds to RAGE or a RAGE ligand and has the features set forth in the Summary section above or otherwise in this description, or a RAGE-binding fragment which is selected from the group consisting of a chimeric antibody, a humanized antibody, a single chain antibody, a tetrameric antibody, a tetravalent antibody, a multispecific antibody, a domain-specific antibody, a domain-deleted antibody, a fusion protein, an Fab fragment, an Fab' fragment, an F(ab')2 fragment, an Fv fragment, an ScFv fragment, an Fd fragment, a single domain antibody, a dAb fragment, a V_{HH} domain such as a Nanobody™ (see Mulydermans et al., Annual Review of Biochemistry (2013) Vol. 82: 775-797; and an Fc fusion protein (i.e., an antigen binding domain fused to an immunoglobulin constant region). These antibodies can be coupled with a cytotoxic agent, a radiotherapeutic agent, or a detectable label.

Antibodies of the invention that specifically bind to RAGE also include variants of any of the antibodies described herein, which may be readily prepared using known molecular biology and cloning techniques.

Antibodies of the invention may also comprise a label attached thereto and able to be detected, (e.g. the label can be a radioisotope, fluorescent compound, enzyme or enzyme co-factor).

Proteins or polypeptides referred to herein as "recombinant" are proteins or polypeptides produced by the expression of recombinant nucleic acids.

The term "antibody" is used interchangeably with the term "immunoglobulin" herein, and includes intact antibodies, fragments of antibodies, e.g., Fab, F(ab')2 fragments, and intact antibodies and fragments that have been mutated either in their constant and/or variable region (e.g., mutations to produce chimeric, partially humanized, or fully humanized antibodies, as well as to produce antibodies with a desired trait, e.g., enhanced IL 13 binding and/or reduced FcR binding). The term "fragment" refers to a part or portion of an antibody or antibody chain comprising fewer amino acid residues than an intact or complete antibody or antibody chain. Fragments can be obtained via chemical or enzymatic treatment of an intact or complete antibody or antibody chain. Fragments can also be obtained by recombinant means. Exemplary fragments include Fab, Fab', F(ab')2, Fabc, Fd, dAb, V_{HH} and scFv and/or Fv fragments.

The antibody specifically binding to RAGE, or the antigen binding fragments thereof, may include variants of amino acid sequences disclosed herein within a range retaining the ability to specifically recognize RAGE. For example, to enhance the binding affinity and/or other biological properties of the antibody, the amino acid sequences of the antibody may be mutated. For example, such mutations include deletion, insertion, and/or substitution of amino acid sequence residues of the antibody. An amino acid mutation is made based on the relative similarity of the amino acid side chain substituents, for example, with respect to hydrophobic properties, hydrophilic properties, charges, or sizes. For example, arginine, lysine, and histidine are each a positively charged residue; alanine, glycine, and serine have a similar size; and phenylalanine, tryptophan, and tyrosine have a similar shape. Therefore, based on the considerations described above, arginine, lysine, and histidine may be biological functional equivalents; alanine, glycine, and serine may be biological functional equivalents; and phenylalanine, tryptophan, and tyrosine may be biological functional equivalents.

Amino acid substitution in a protein in which the activity of the molecule is not completely changed is well known in the art. Typical substitutions include Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly substitutions. Considering mutations with biologically equivalent activity, an antibody specifically binding to RAGE or the antigen-binding fragments thereof may also include sequences substantially identical to sequences disclosed herein. In this regard, a substantially identical amino acid sequence may be a sequence with at least 60% homology, at least 70% homology, at least 80% homology, at least 90%, at least 95% homology or 100% homology to a sequence disclosed herein, when the amino acid sequences are aligned to correspond to each other as much as possible. The aligned amino acid sequences are analyzed using an algorithm known in the art. Alignment methods for sequence comparison are well known to one of ordinary skill in the art. For example, a sequence analysis program available on the Internet at the NCBI Basic Local Alignment Search Tool (BLAST) home page, such as blastp, blastx, tblastn, or tblastx, may be used.

As used herein, a preparation of antibody protein having less than about 50% of non-antibody protein (also referred to herein as a "contaminating protein"), or of chemical precursors, is considered to be "substantially free." 40%, 30%, 20%, 10% and more preferably 5% (by dry weight), of non-antibody protein, or of chemical precursors is considered to be substantially free. When the antibody protein or biologically active portion thereof is recombinantly produced, it is also preferably substantially free of culture medium, i.e., culture medium represents less than about 30%, preferably less than about 20%, more preferably less than about 10%, and most preferably less than about 5% of the volume or mass of the protein preparation.

The term "antigen-binding fragment" refers to a polypeptide fragment of an immunoglobulin or antibody that binds antigen or competes with intact antibody (i.e., with the intact antibody from which they were derived) for antigen binding (i.e., specific binding). As such these antibodies or fragments thereof are included in the scope of the invention, provided that the antibody or fragment binds specifically to RAGE, and neutralizes or inhibits one or more RAGE-associated activities (e.g., inhibits binding of RAGE binding ligands to RAGE).

It is intended that the term "antibody" encompass any Ig class or any Ig subclass (e.g. the IgG1, IgG2, IgG3, and IgG4 subclassess of IgG) obtained from any source (e.g., humans and non-human primates, and in rodents, lagomorphs, caprines, bovines, equines, ovines, etc.).

The term "Ig class" or "immunoglobulin class", as used herein, refers to the five classes of immunoglobulin that have been identified in humans and higher mammals, IgG, IgM, IgA, IgD, and IgE. The term "Ig subclass" refers to the two subclasses of IgM (H and L), three subclasses of IgA (IgA1, IgA2, and secretory IgA), and four subclasses of IgG (IgG1, IgG2, IgG3, and IgG4) that have been identified in humans and higher mammals. The antibodies can exist in monomeric or polymeric form; for example, IgM antibodies exist in pentameric form, and IgA antibodies exist in monomeric, dimeric or multimeric form.

The term "IgG subclass" refers to the four subclasses of immunoglobulin class IgG - IgG1, IgG2, IgG3, and IgG4 that have been identified in humans and higher mammals by the heavy chains of the immunoglobulins, VI - γ4, respectively. The term "single-chain immunoglobulin" or "single-chain antibody" (used interchangeably herein) refers to a protein having a two-polypeptide chain structure consisting of a heavy and a light chain, said chains being stabilized, for example, by interchain peptide linkers, which has the ability to specifically bind antigen. The term "domain" refers to a globular region of a heavy or light chain polypeptide comprising peptide loops (e.g., comprising 3 to 4 peptide loops) stabilized, for example, by β pleated sheet and/or intrachain disulfide bond. Domains are further referred to herein as "constant" or "variable", based on the relative lack of sequence variation within the domains of various class members in the case of a "constant" domain, or the significant variation within the domains of various class members in the case of a "variable" domain. Antibody or polypeptide "domains" are often referred to interchangeably in the art as antibody or polypeptide "regions". The "constant" domains of an antibody light chain are referred to interchangeably as "light chain constant regions", "light chain constant domains", "CL" regions or "CL" domains. The "constant" domains of an antibody heavy chain are referred to interchangeably as "heavy chain constant regions", "heavy chain constant domains", "CH" regions or "CH" domains). The "variable" domains of an antibody light chain are referred to interchangeably as "light chain variable regions", "light chain variable domains", "VL" regions or "VL" domains). The "variable" domains of an antibody heavy chain are referred to interchangeably as "heavy chain constant regions", "heavy chain constant domains", "VH" regions or "VH" domains).

The term "region" can also refer to a part or portion of an antibody chain or antibody chain domain (e.g., a part or portion of a heavy or light chain or a part or portion of a constant or variable domain, as defined herein), as well as more discrete parts or portions of said chains or domains. For example, light and heavy chains or light and heavy chain variable domains include "complementarity determining regions" or "CDRs" interspersed among "framework regions" or "FRs", as defined herein.

The term "conformation" refers to the tertiary structure of a protein or polypeptide (e.g., an antibody, antibody chain, domain or region thereof). For example, the phrase "light (or heavy) chain conformation" refers to the tertiary structure of a light (or heavy) chain variable region, and the phrase "antibody conformation" or "antibody fragment conformation" refers to the tertiary structure of an antibody or fragment thereof.

"Specific binding" of an antibody means that the antibody exhibits appreciable affinity for a particular antigen or epitope and, generally, does not exhibit significant crossreactivity. The term "anti-RAGE antibody" as used herein refers to an antibody that binds specifically to a RAGE. "Appreciable" binding includes binding with an affinity of at least 25µM. Antibodies with affinities greater than 1 x 10⁷ M⁻¹ (or a dissociation coefficient of 1µm or less or a dissociation coefficient of 1nm or less) typically bind with correspondingly greater specificity. Values intermediate of those set forth herein are also intended to be within the scope of the present invention and antibodies of the invention bind to RAGE with a range of affinities, for example, 100nM or less, 75nM or less, 50nM or less, 25nM or less, for example 10nM or less, 5nM or less, 1nM or less, or in embodiments 500pM or less, 100pM or less, 50pM or less or 25pM or less. An antibody that "does not exhibit significant crossreactivity" is one that will not appreciably bind to an entity other than its target (e.g., a different epitope or a different molecule). For example, an antibody that specifically binds to RAGE will appreciably bind RAGE but will not significantly react with non-RAGE proteins or peptides. An antibody specific for a particular epitope will, for example, not significantly crossreact with remote epitopes on the same protein or peptide. Specific binding can be determined according to any art-recognized means for determining such binding. Preferably, specific binding is determined according to Scatchard analysis and/or competitive binding assays.

As used herein, the term "affinity" refers to the strength of the binding of a single antigen-combining site with an antigenic determinant. Affinity depends on the closeness of stereochemical fit between antibody combining sites and antigen determinants, on the size of the area of contact between them, on the distribution of charged and hydrophobic groups, etc. Antibody affinity can be measured by equilibrium dialysis or by the kinetic BIACORE™ method. The dissociation constant, Kd, and the association constant, Ka, are quantitative measures of affinity.

As used herein, the term "monoclonal antibody" refers to an antibody derived from a clonal population of antibody-producing cells (e.g., B lymphocytes or B cells) which is homogeneous in structure and antigen specificity. The term "polyclonal antibody" refers to a plurality of antibodies originating from different clonal populations of antibody-producing cells which are heterogeneous in their structure and epitope specificity but which recognize a common antigen. Monoclonal and polyclonal antibodies may exist within bodily fluids, as crude preparations, or may be purified, as described herein.

The term "binding portion" of an antibody (or "antibody portion") includes one or more complete domains, e.g., a pair of complete domains, as well as fragments of an antibody that retain the ability to specifically bind to RAGE. It has been shown that the binding function of an antibody can be performed by fragments of a full-length antibody. Binding fragments are produced by recombinant DNA techniques, or by enzymatic or chemical cleavage of intact immunoglobulins. Binding fragments include Fab, Fab', F(ab')2, Fabc, Fd, dAb, Fv, single chains, single-chain antibodies, e.g., scFv, and single domain antibodies.

"Humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, FR residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin.

Dose or dosage levels for antibodies in suitable and/or preferred pharmaceutical formulations can be determined in view of the present disclosure and general knowledge of formulation technology, depending upon the intended route of administration, delivery format, and desired dosage. Typically, a physician will determine the actual dosage which will be most suitable for an individual subject.

The specific dose level and frequency of dosage for any particular patient or subject in need of treatment thereof may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. For example, the antibody compound or composition may be administered at a dose of between 2 mg/kg and 0.1 mg/kg, depending on its activity.

The anti-RAGE antibody or fragment thereof may be administered as a fixed dose, independent of a dose per subject weight ratio, or at an appropriate dose in mg/kg body weight with an approximate maximum of 200 mg/kg. The antibody or fragment thereof may be administered to a 70 kg individual in one or more separate, simultaneous or sequential doses of 14,000 mg or less of antibody or fragment thereof, 13,000 mg or less of antibody or fragment thereof, 12,000 mg or less of antibody or fragment thereof, 11,000 mg or less of antibody or fragment thereof, 10,000 mg or less of antibody or fragment thereof, 9000 mg or less of antibody or fragment thereof, 8000 mg or less of antibody or fragment thereof, 7000 mg or less of antibody or fragment thereof, 6000 mg or less of antibody or fragment thereof, 5000 mg or less of antibody or fragment thereof, 4500 mg or less of antibody or fragment thereof, 4000 mg or less of antibody or fragment thereof, 3700 mg or less of antibody or fragment thereof, 3500 mg or less of antibody or fragment thereof, mg or less of antibody or fragment thereof, 3200 mg or less of antibody or fragment thereof, 3000 mg or less of antibody or fragment thereof, 2700 mg or less of antibody or fragment thereof, 2500 mg or less of antibody or fragment thereof, 2200 mg or less of antibody or fragment thereof, or 2100 mg or less of antibody or fragment thereof, 1700 mg or less of antibody or fragment thereof, 1500 mg or less of antibody or fragment thereof, 1200 mg or less of antibody or fragment thereof, or 1100 mg or less of antibody or fragment thereof, 1000 mg or less of antibody or fragment thereof, 700 mg or less of antibody or fragment thereof, 500 mg or less of antibody or fragment thereof, 200 mg or less of antibody or fragment thereof, or 100 mg or less of antibody or fragment thereof. In another embodiment, the antibody or fragment is administered in one or more doses of at least 20 mg of antibody or fragment thereof. Since the total protein concentration in human plasma is 70,000 mg/l, and standard blood transfusion or plasma or albumin infusions routinely deliver tens or even hundreds of grams of protein intravenously, administration of the maximal doses of anti-RAGE antibody are safe and acceptable.

The anti-RAGE antibody compound or composition may be administered as a fixed dose, independent of a dose per subject weight ratio. The anti-RAGE antibody compound or composition may be administered in one or more separate, simultaneous or sequential parenteral doses of 100 mg or less, of 50 mg or less, 25 mg or less, or 10 mg or less. Alternatively, anti-RAGE antibody compound or composition may be administered in a dose per subject weight ratio as easily determined by one of skill in the art. The component(s) may be formulated into a pharmaceutical composition, such as by mixing with one or more of a suitable carrier, diluent or excipient, by using techniques that are known in the art.

### Vector delivery systems

One type of vector is a "plasmid," which refers to a circular double stranded DNA loop into which additional DNA segments can be inserted, such as by standard molecular cloning techniques. Another type of vector is a viral vector, wherein virally-derived DNA or RNA sequences are present in the vector for packaging into a virus (e.g. retroviruses, replication defective retroviruses, adenoviruses, replication defective adenoviruses, and adeno-associated viruses). Viral vectors also include polynucleotides carried by a virus for transfection into a host cell. Certain vectors are capable of autonomous replication in a host cell into which they are introduced (e.g. bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors." Common expression vectors of utility in recombinant DNA techniques are often in the form of plasmids.

Recombinant expression vectors can comprise a nucleic acid of the invention in a form suitable for expression of the nucleic acid in a host cell, which means that the recombinant expression vectors include one or more regulatory elements, which may be selected on the basis of the host cells to be used for expression, that is operatively-linked to the nucleic acid sequence to be expressed. Within a recombinant expression vector, "operably linked" is intended to mean that the nucleotide sequence of interest is linked to the regulatory element(s) in a manner that allows for expression of the nucleotide sequence (e.g. in an in vitro transcription/translation system or in a host cell when the vector is introduced into the host cell).

Preferably, delivery is in the form of a vector which may be a viral vector, such as a lenti- or baculo- or preferably adeno-viral/adeno-associated viral vectors, but other means of delivery are known (such as yeast systems, microvesicles, gene guns/means of attaching vectors to gold nanoparticles) and are provided. A vector may mean not only a viral or yeast system (for instance, where the nucleic acids of interest may be operably linked to and under the control of (in terms of expression, such as to ultimately provide a processed RNA) a promoter), but also direct delivery of nucleic acids into a host cell. While in herein methods the vector may be a viral vector and this is advantageously an AAV, other viral vectors as herein discussed can be employed, such as lentivirus. For example, baculoviruses may be used for expression in insect cells. These insect cells may, in turn be useful for producing large quantities of further vectors, such as AAV or lentivirus vectors adapted for delivery of the present invention. Also envisaged is a method of delivering the present compositions comprising nucleic acids encoding anti-RAGE antibodies of the invention comprising delivering to a cell mRNA encoding said anti-RAGE antibodies. AAV and lentiviral vectors are preferred. delivering a non-naturally occurring or engineered composition comprising an AAV or lentivirus vector system comprising one or more AAV or lentivirus vectors operably encoding a composition for expression thereof.

Use of viral vectors for antibody-based protection (commonly referred to as "vectored immunprophylaxis") is known in the art. For example, Balzas et al. discuss antibody-based protection against HIV infection by vectored immunoprophylaxis (see e.g., Balazs A. B. et al. Nature 2011 Nov 30;481(7379):81-4. Antibody-based protection against HIV infection by vectored immunoprophylaxis; Balazs A.B. et al. Nat. Med. 2014 Mar;20(3):296-300. Vectored immunoprophylaxis protects humanized mice from mucosal HIV transmission.).

The invention in some embodiments comprehends a method of preparing the AAV of the invention comprising transfecting plasmid(s) containing or consisting essentially of nucleic acid molecule(s) coding for the AAV into AAV-infected cells, and supplying AAV rep and/or cap obligatory for replication and packaging of the AAV. In some embodiments the AAV rep and/or cap obligatory for replication and packaging of the AAV are supplied by transfecting the cells with helper plasmid(s) or helper virus(es). In some embodiments the helper virus is a poxvirus, adenovirus, herpesvirus or baculovirus. In some embodiments the poxvirus is a vaccinia virus. In some embodiments the cells are mammalian cells. And in some embodiments the cells are insect cells and the helper virus is baculovirus. In other embodiments, the virus is a lentivirus.

As to AAV, the AAV can be AAV1, AAV2, AAV5 or any combination thereof. One can select the AAV of the AAV with regard to the cells to be targeted; e.g., one can select AAV serotypes 1, 2, 5 or a hybrid or capsid AAV1, AAV2, AAV5 or any combination thereof for targeting brain or neuronal cells; and one can select AAV4 for targeting cardiac tissue. AAV8 is useful for delivery to the liver. The above promoters and vectors are preferred individually. For example, for AAV, the route of administration, formulation and dose can be as in US Patent No. 8,454,972 and as in clinical trials involving AAV. For Adenovirus, the route of administration, formulation and dose can be as in US Patent No. 8,404,658 and as in clinical trials involving adenovirus. For plasmid delivery, the route of administration, formulation and dose can be as in US Patent No 5,846,946 and as in clinical studies involving plasmids. Doses may be based on or extrapolated to an average 70 kg individual, and can be adjusted for patients, subjects, mammals of different weight and species. Frequency of administration is within the ambit of the medical or veterinary practitioner (e.g., physician, veterinarian), depending on usual factors including the age, sex, general health, other conditions of the patient or subject and the particular condition or symptoms being addressed.

The viral vectors can be injected into the tissue of interest. The expression of anti-RAGE antibodies can be driven by a cell-type specific promoter. For example, liver-specific expression might use the Albumin promoter and neuron-specific expression might use the Synapsin I promoter.

In some embodiments, the viral vector is delivered to the tissue of interest by, for example, an intramuscular injection, while other times the viral delivery is via intravenous, transdermal, intranasal, oral, mucosal, or other delivery methods. Such delivery may be either via a single dose, or multiple doses. One skilled in the art understands that the actual dosage to be delivered herein may vary greatly depending upon a variety of factors, such as the vector chose, the target cell, organism, or tissue, the general condition of the subject to be treated, the degree of transformation/modification sought, the administration route, the administration mode, the type of transformation/modification sought, etc.

Such a dosage may further contain, for example, a carrier (water, saline, ethanol, glycerol, lactose, sucrose, calcium phosphate, gelatin, dextran, agar, pectin, peanut oil, sesame oil, etc.), a diluent, a pharmaceutically-acceptable carrier (e.g., phosphate-buffered saline), a pharmaceutically-acceptable excipient, an adjuvant to enhance antigenicity, an immunostimulatory compound or molecule, and/or other compounds known in the art. The adjuvant herein may contain a suspension of minerals (alum, aluminum hydroxide, aluminum phosphate) on which antigen is adsorbed; or water-in-oil emulsion in which antigen solution is emulsified in oil (MF-59, Freund's incomplete adjuvant), sometimes with the inclusion of killed mycobacteria (Freund's complete adjuvant) to further enhance antigenicity (inhibits degradation of antigen and/or causes influx of macrophages). Adjuvants also include immunostimulatory molecules, such as cytokines, costimulatory molecules, and for example, immunostimulatory DNA or RNA molecules, such as CpG oligonucleotides. Such a dosage formulation is readily ascertainable by one skilled in the art. The dosage may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include microcrystalline cellulose, carboxymethylcellulose sodium, polysorbate 80, phenylethyl alcohol, chlorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991).
In an embodiment herein the delivery is via an adenovirus, which may be at a single booster dose containing at least 1 x 10⁵ particles (also referred to as particle units, pu) of adenoviral vector. In an embodiment herein, the dose preferably is at least about 1 x 10⁶ particles (for example, about 1 x 106-1 x 10¹² particles), more preferably at least about 1 x 10⁷ particles, more preferably at least about 1 x 10⁸ particles (e.g., about 1 x 10⁸-1 x 10¹¹ particles or about 1 x 10⁸-1 x 10¹² particles), and most preferably at least about 1 x 10⁹ particles (e.g., about 1^{∗} x 109-1 x 10¹⁰ particles or about 1 x 10⁹-1 x 10¹² particles), or even at least about 1 x 10¹⁰ particles (e.g., about 1 x 10¹⁰-1 x 10¹² particles) of the adenoviral vector. Alternatively, the dose comprises no more than about 1 x 10¹⁴ particles, preferably no more than about 1 x 10¹³ particles, even more preferably no more than about 1 x 10¹² particles, even more preferably no more than about 1 x 10¹¹ particles, and most preferably no more than about 1 x 10¹⁰ particles (e.g., no more than about 1 x 10⁹ articles). Thus, the dose may contain a single dose of adenoviral vector with, for example, about 1 x 10⁶ particle units (pu), about 2 x 10⁶ pu, about 4 x 10⁶ pu, about 1 x 10⁷ pu, about 2 x 10⁷ pu, about 4 x 10⁷ pu, about 1 x 10⁸ pu, about 2 x 10⁸ pu, about 4 x 10⁸ pu, about 1 x 10⁹ pu, about 2 x 10⁹ pu, about 4 x 10⁹ pu, about 1 x 10¹⁰ pu, about 2 x 10¹⁰ pu, about 4 x 10¹⁰ pu, about 1 x 10¹¹ pu, about 2 x 10¹¹ pu, about 4 x 10¹¹ pu, about 1 x 10¹² pu, about 2 x 10¹² pu, or about 4 x 10¹² pu of adenoviral vector. See, for example, the adenoviral vectors in U.S. Patent No. 8,454,972 B2 to Nabel, et. al., granted on June 4, 2013, and the dosages at col 29, lines 36-58 thereof. In an embodiment herein, the adenovirus is delivered via multiple doses.

In an embodiment herein, the delivery is via an AAV. A therapeutically effective dosage for *in vivo* delivery of the AAV to a human is believed to be in the range of from about 20 to about 50 ml of saline solution containing from about 1 x 10¹⁰ to about 1 x 10¹⁰ functional AAV/ml solution. The dosage may be adjusted to balance the therapeutic benefit against any side effects. In an embodiment herein, the AAV dose is generally in the range of concentrations of from about 1 x 10⁵ to 1 x 10⁵⁰ genomes AAV, from about 1 x 10⁸ to 1 x 10²⁰ genomes AAV, from about 1 x 10¹⁰ to about 1 x 10¹⁶ genomes, or about 1 x 10¹¹ to about 1 x 10¹⁶ genomes AAV. A human dosage may be about 1 x 10¹³ genomes AAV. Such concentrations may be delivered in from about 0.001 ml to about 100 ml, about 0.05 to about 50 ml, or about 10 to about 25 ml of a carrier solution. Other effective dosages can be readily established by one of ordinary skill in the art through routine trials establishing dose response curves. See, for example, U.S. Patent No. 8,404,658 B2 to Hajjar, et al., granted on March 26, 2013, at col. 27, lines 45-60.

In an embodiment herein the delivery is via a plasmid. In such plasmid compositions, the dosage should be a sufficient amount of plasmid to elicit a response. For instance, suitable quantities of plasmid DNA in plasmid compositions can be from about 0.1 to about 2 mg, or from about 1 µg to about 10 µg.

The doses herein are based on an average 70 kg individual. The frequency of administration is within the ambit of the medical or veterinary practitioner (e.g., physician, veterinarian), or scientist skilled in the art.

### Lentivirus

Lentiviruses are complex retroviruses that have the ability to infect and express their genes in both mitotic and post-mitotic cells. The most commonly known lentivirus is the human immunodeficiency virus (HIV), which uses the envelope glycoproteins of other viruses to target a broad range of cell types.

Lentiviruses may be prepared as follows. After cloning pCasES10 (which contains a lentiviral transfer plasmid backbone), HEK293FT at low passage (p=5) were seeded in a T-75 flask to 50% confluence the day before transfection in DMEM with 10% fetal bovine serum and without antibiotics. After 20 hours, media was changed to OptiMEM (serum-free) media and transfection was done 4 hours later. Cells were transfected with 10 µg of lentiviral transfer plasmid (pCasES10) and the following packaging plasmids: 5 µg of pMD2.G (VSV-g pseudotype), and 7.5ug of psPAX2 (gag/pol/rev/tat). Transfection was done in 4mL OptiMEM with a cationic lipid delivery agent (50µL Lipofectamine 2000 and 100µl Plus reagent). After 6 hours, the media was changed to antibiotic-free DMEM with 10% fetal bovine serum. Lentivirus may be purified as follows. Viral supernatants were harvested after 48 hours. Supernatants were first cleared of debris and filtered through a 0.45um low protein binding (PVDF) filter. They were then spun in a ultracentrifuge for 2 hours at 24,000 rpm. Viral pellets were resuspended in 50µl of DMEM overnight at 4C. They were then aliquotted and immediately frozen at -80C.

In another embodiment, minimal non-primate lentiviral vectors based on the equine infectious anemia virus (EIAV) are also contemplated, especially for ocular gene therapy (see, e.g., Balagaan, J Gene Med 2006; 8: 275 - 285, Published online 21 November 2005 in Wiley InterScience (www.interscience.wiley.com). DOI: 10.1002/jgm.845). In another embodiment, RetinoStat®, an equine infectious anemia virus-based lentiviral gene therapy vector that expresses angiostatic proteins endostain and angiostatin that is delivered via a subretinal injection for the treatment of the web form of age-related macular degeneration is also contemplated (see, e.g., Binley et al., HUMAN GENE THERAPY 23:980-991 (September 2012)) may be modified for expressing antibody(ies) of the present invention.

In another embodiment, self-inactivating lentiviral vectors with an siRNA targeting a common exon shared by HIV tat/rev, a nucleolar-localizing TAR decoy, and an anti-CCR5-specific hammerhead ribozyme (see, e.g., DiGiusto et al. (2010) Sci Transl Med 2:36ra43) may be used/and or adapted for expressing antibody(ies) of the present invention. A minimum of 2.5 × 106 CD34+ cells per kilogram patient weight may be collected and prestimulated for 16 to 20 hours in X-VIVO 15 medium (Lonza) containing 2mML-glutamine, stem cell factor (100 ng/ml), Flt-3 ligand (Flt-3L) (100 ng/ml), and thrombopoietin (10 ng/ml) (CellGenix) at a density of 2 × 106 cells/ml. Prestimulated cells may be transduced with lentiviral at a multiplicity of infection of 5 for 16 to 24 hours in 75-cm2 tissue culture flasks coated with fibronectin (25 mg/cm2) (RetroNectin,Takara Bio Inc.).

Lentiviral vectors have been disclosed as in the treatment for Parkinson's Disease, see, e.g., US Patent Publication No. 20120295960 and US Patent Nos. 7303910 and 7351585, and for delivery of anti-αβ antibodies for the treatment of Alzheimers disease (Fukuchi et al., Neurobiol Dis. 2006 September ; 23(3): 502-511). Lentiviral vectors have also been disclosed for the treatment of ocular diseases, see e.g., US Patent Publication Nos. 20060281180, 20090007284, US20110117189; US20090017543; US20070054961, US20100317109. Lentiviral vectors have also been disclosed for delivery to the brain, see, e.g., US Patent Publication Nos. US20110293571; US20110293571, US20040013648, US20070025970, US20090111106 and US Patent No. US7259015. These vectors can be adapted to express the antibody(ies) of the invention, and can be administered in analogous amounts, albeit advantageously iv or im or to the tumor mass rather than to the brain or eye (unless, of course, the tumor is in the brain or eye).

### Pre-cachexia and Cachexia

The invention provides compositions and methods for identifying patients with pre-cachexia and/or cachexia; a culture system that reproduces the cachectic process in cells *in vitro;* and methods of using this system to identify markers associated with pre-cachexia and/or cachexia, as well as for the discovery of therapeutic agents useful for disrupting (slowing, reducing, reversing, or preventing) the progression of pre-cachexia to cachexia.

The invention is based, at least in part, on the discovery of a molecular signature associated with pre-cachexia and/or cachexia. Accordingly, the invention provides a panel of markers useful for identifying patients that are pre-cachectic. This panel of markers or biomarkers includes but is not limited to S100A7, S100A8, S100A9, skeletal or cardiac muscle MYH, INSR and/or BCAA. In identifying patients with pre-cachexia, e.g., cardiac pre-cachexia, the panel include the cardio specific MYH, MYH6, MYH7, MYH7B. This provides for the identification and treatment of subjects before they develop the progressive weight loss and muscle atrophy that define cachexia, and which has proven refractory to all attempted therapeutic interventions. The invention further provides markers useful for characterizing cachexia.

### Cachexia's Effect on Patient Prognosis

The development of new highly effective oncologic therapies has transformed many cancers into chronically managed diseases. The efficacy of these new treatment regimens does not guarantee an increase in survival. While surgery, radiation or chemotherapy may successfully reduce tumor size, this reduction does not always correlate with an increase in survival. In fact, for many patients, degree of tumor burden does not correlate with prognosis. As shown herein, the difference in survival time for patients with only modest tumor burden is only about eight months more than the survival time of patients with extensive tumor burden. This counter-intuitive result may be attributed, at least in part, to cachexia. The clinical manifestations of cachexia are complex: muscle and fat wasting, multi organ dysfunction (e.g., cardiac, pulmonary, gastrointestinal), and profound metabolic derangement.

More than twenty-five percent of cancer deaths are caused-not by cancer-but by cachexia. Cachexia associated deaths include death by respiratory failure, cardiac failure, and metabolic derangement. The devastating effects of cachexia are poignantly illustrated in a case study of a sixty-one year old patient with pancreatic cancer. The patient presented with worsening abdominal pain. Upon imaging, a small (3 x 3 x 2cm) tumor was observed in the head of the pancreas. The patient was diagnosed with stage III non-resectable pancreatic cancer. She also had cachexia, having lost 35 lbs (17%) of her body weight. The patient was treated with 5'-FU / Leucovorin / Irinotecan / Oxaliplatin. Treatment successfully reduced the size of the patient's tumor, as well as levels of CA-19.9 a serum marker associated with pancreatic cancer. Despite this positive response, the patient continued to lose weight, ultimately becoming so frail that anti-cancer therapy had to be discontinued.

While cachexia shares certain phenotypic similarities with food deprivation, in fact cachexia is distinct from starvation. Even where patients with cachexia are provided with total parenteral nutrition, weight loss, including loss of lean body mass, continues. These losses have proven refractory to all therapeutic interventions, except for a complete removal of the cancer, which remains elusive for the vast majority of cancer patients.

### In vitro Cachexia/Pre-Cachexia Model System

To date, progress in understanding cachexia has been hampered by the lack of an *in vitro* system that could reproduce the hallmarks of cachexia. The identification of such a system is critical to identifying factors that mediate the tumor-host interaction. In the simplest view of tumor-host interactions, the tumor produces a factor that mediates wasting/dysfunction of target tissues. While conventional wisdom has held that mice "do not get cancer cachexia". To the contrary, results provided herein demonstrate that human cancer cell lines induce a cachectic state in murine xenograft models. Conventional wisdom also holds to the notion that immune system is required to generate inflammatory cytokines that have been thought to drive cachexia. We have demonstrated that the tumor cells from multiple tumor types grown in isolation produce the mediator(s) that cause target cells (muscle, fat, and liver) to phenocopy clinical cachexia with tremendous specificity, thus the immune is not required. These observations support the premise that tumor cells secrete one or more factors that drive cachexia. The present invention provides an *in vitro* system that models pre-cachexia and/or cachexia and facilitates the identification of agents that drive cachexia vs. factors that merely contribute to the disease process.

The present invention provides a culture system, comprising a human target cell and a cachexia-inducing factor. In certain embodiments, the target cell is selected from a myocyte, an adipocyte, and a hepatocyte. In one embodiment, the target cell is a myocyte.

In certain embodiments, the cachexia-inducing factor is provided in (i) human plasma from a cachexia patient or (ii) cachexia-inducing conditioned media, such as human cancer cell conditioned media. In certain embodiments, the media is conditioned by a human cancer cell line selected from one or more of the following: AsPC-1, A375, CAPAN-1, CAPAN-2, C32, G361, HCT-15, HPAF-II, JHU012, JHU022, LS180, LX1, MKN1, PC9, HCC827 and PANC-1. In particular embodiments, a cachexia-inducing cell line is the human melanoma cell line A375 (ATCC® CRL1619™); the intestinal human colon adenocarcinoma cell line LS180 (ATCC® CL-187™); the squamous cel lung cancer cell line LX1 (Cell vol. 29, 161-169 (1982); the malignant melanoma human cell line G361 (ATCC® CRL-1424™), or the human malignant melanoma cell line C32 (ATCC® CRL-1585™). In certain embodiments, the media is conditioned by a human cancer cell isolated from a patient.

In certain embodiments, the culture is in a single or a multi-well plate format. In certain embodiments, the invention provides a multi-well plate suitable for use in a high-throughput screening system, the plate having a plurality of wells comprising the culture system described therein. The surface of the multi-well plate may be the surface of a culture well or glass slide, or any other suitable surface. In certain embodiments the multi-well plate contains 384 wells. Cultures can be contained in a multi-well plate having a 96-, 384-, 1536- or more than 1536-well format.

In certain embodiments, the invention further provides a method for producing the model of the present invention, in a single or multi-well plate format.

### Uses of the Cachexia/Pre-cachexia Model System

The present invention provides methods for characterizing an agent for the ability to induce pre-cachexia or cachexia in a target cell. The method generally involves exposing a target cell to a test agent, and characterizing the effect of the agent on the target cell relative to: (i) a control target cell not exposed to the test agent; or (ii) a transcriptional profile of a non-cachectic cell and/or a transcriptional profile of a cachectic cell, wherein the cell type of the target cell and the cachectic and/or non-cachectic cell is the same.

The present invention provides a method for predicting the effect of a test agent on a target cell of a patient *in vivo,* comprising culturing a target cell obtained from a patient in the system of the invention, exposing it to the test agent, and assaying for a pharmacological effect of the test agent on the target cell relative to: (i) a control target cell not treated with the test agent; or (ii) a transcriptional profile of a non-cachectic cell and/or a transcriptional profile of a cachectic cell, wherein the cell type of the target cell and the cachectic and/or non-cachectic cell is the same.

In certain embodiments, the target cell is isolated from a patient with cancer. "Cancer" is understood in the context it is used in the literature, e.g., Martin, L. et al., J Clin Oncol 31(12): 1539-1547 (2013).

In certain embodiments, the effect is selected from proliferation, viability, and differentiation, or combinations thereof.

In certain embodiments, the effect is detected by assessing a change in gene expression profile between the target cell and the control target cell of step (i); or the cachectic or non-cachectic cell of step (ii).

In certain embodiments, the target cell is selected from a myocyte, an adipocyte, and a hepatocyte. In a preferred embodiment, the target cell is a myocyte.

The cachectic culture system can be used to screen for test agents (such as solvents, small molecule drugs, peptides, and polynucleotides) or environmental conditions (such as culture conditions or manipulation) that affect the characteristics of cells. Two or more agents can be tested in combination (by exposing to the cells either simultaneously or sequentially), to detect possible drug-drug interactions and/or rescue effects (e.g., by testing a toxin and a potential anti-toxin). Agent(s) and environmental condition(s) can be tested in combination (by treating the cells with a drug either simultaneously or sequentially relative to an environmental condition), to detect possible agent-environment interaction effects.

In certain embodiments, the assay to determine the characteristics of cells is selected in a manner appropriate to the cell type and agent and/or environmental factor being studied as disclosed in WO 2002/04113. For example, changes in cell morphology may be assayed by standard light, or electron microscopy. Alternatively, the effects of treatments or compounds potentially affecting the expression of cell surface proteins may be assayed by exposing the cells to either fluorescently labeled ligands of the proteins or antibodies to the proteins and then measuring the fluorescent emissions associated with each cell on the plate. As another example, the effects of treatments or compounds which potentially alter the pH or levels of various ions within cells may be assayed using various dyes which change in color at determined pH values or in the presence of particular ions. The use of such dyes is well known in the art. For cells, which have been transformed or transfected with a genetic marker, such as the β-galactosidase, alkaline phosphatase, or luciferase genes, the effects of treatments or compounds may be assessed by assays for expression of that marker. In particular, the marker may be chosen so as to cause spectrophotometrically assayable changes associated with its expression.

Particular screening applications of this invention relate to the testing of pharmaceutical compounds in drug research. The reader is referred generally to the standard textbook In vitro Methods in Pharmaceutical Research, Academic Press, 1997, and U.S. Pat. No. 5,030,015. In certain aspects of this invention, the culture of the invention is used to grow and differentiate a cachectic target cell to play the role of test cells for standard drug screening and toxicity assays. Assessment of the activity of candidate pharmaceutical compounds generally involves combining the target cell (e.g., a myocyte, an adipocyte, a cardiomyocyte or a hepatocyte) with the candidate compound, determining any change in the morphology, marker phenotype, or metabolic activity of the cells that is attributable to the candidate compound (compared with untreated cells or cells treated with an inert compound, such as vehicle), and then correlating the effect of the candidate compound with the observed change. The screening may be done because the candidate compound is designed to have a pharmacological effect on the target cell, or because a candidate compound may have unintended side effects on the target cell. Alternatively, libraries can be screened without any predetermined expectations in hopes of identifying compounds with desired effects.

Cytotoxicity can be determined in the first instance by the effect on cell viability and morphology. In certain embodiments, toxicity may be assessed by observation of vital staining techniques, ELISA assays, immunohistochemistry, and the like or by analyzing the cellular content of the culture, e.g., by total cell counts, and differential cell counts or by metabolic markers such as MTT and XTT.

Additional further uses of the culture of the invention include, but are not limited to, its use in research e.g., to elucidate cachectic mechanisms leading to the identification of novel targets for cachectic therapies, and to generate genotype-specific cells for disease modeling, including the generation of new therapies customized to different genotypes. Such customization can reduce adverse drug effects and help identify therapies appropriate to the patient's genotype.

### Methods of Identifying a Patient as Having Pre-Cachexia or Cachexia

As reported herein, within days of contacting target cells with cachexia-inducing media changes in metabolism, metabolite profiles, the differential expression of markers, and changes in cell morphology are observed. Such changes closely phenocopy overt clinical cachexia as induced by cachectic patient plasma and seen in patient biopsy samples, and likely reproduce the alterations present in pre-cachectic humans and mice. Significantly, many of the changes observed in cells *in vitro* in response to cachexia inducing factors were reversible (e.g., the cachectic gene expression signature, loss of myosin heavy chain, loss of lipid content, and cell atrophy) with novel treatments identified with this discovery platform.

Accordingly, the invention provides methods for identifying subjects as pre-cachectic. Such methods are particularly advantageous given that treatment of pre-cachexia could slow or even reverse the progression of pre-cachexia to cachexia. Moreover, early identification of pre-cachectic patients could ensure that subjects are sufficiently strong to benefit from therapies delineated herein.

The invention provides for a transcriptional biomarker profile of a cachectic phenotype (e.g., cachexia, pre-cachexia) suitable for use in diagnosing and/or monitoring a patient with cachexia or pre-cachexia. Accordingly, the present invention provides a method for diagnosing a patient with cachexia or pre-cachexia, comprising (i) obtaining a target cell from the patient; (ii) determining the gene expression profile of that cell; and (iii) comparing the transcriptional profile of that target cell and the transcriptional profile of a cachectic cell, wherein the cell type of the target cell and the cachectic cell is the same. In a further preferred aspect of the invention, the method of diagnosing a patient with pre-cachexia and providing treatment to the patient in need thereof, comprises (i) obtaining plasma from the patient, (ii) determining the levels of a panel of biomarkers selected from the group consisting of S100A7, S100A8, S100A9, INSR, skeletal or cardiac muscle MYH and BCAA, (iii) comparing the levels of said panel of biomarkers to those obtained from a control or healthy subject, and (iv) if the level of the panel of biomarkers are increased then an effective amount of the antibodies or small molecules of the invention are provided to the patient.

The invention provides for a transcriptional biomarker profile of a cachectic phenotype suitable for use in diagnosing and/or monitoring a patient with cachexia or pre-cachexia. Accordingly, the present invention provides a method for diagnosing a patient with cachexia or pre-cachexia, comprising (i) obtaining a target cell from the patient; (ii) determining the gene expression profile of that cell; and (iii) comparing the transcriptional profile of that target cell and the transcriptional profile of a cachectic cell, wherein the cell type of the target cell and the cachectic cell is the same.

The present invention further provides a method for monitoring a patient with cachexia or pre-cachexia, comprising (i) obtaining a target cell from the patient; (ii) determining the gene expression profile of that cell; and (iii) comparing the transcriptional profile of that target cell and the transcriptional profile of a cachectic cell, wherein the cell type of the target cell and the cachectic cell is the same. Monitoring the cachectic transcriptional profile of a patient is useful, for example, to determine the patient's pharmacological response to a drug or disease progression.

The invention further provides protein-mediated cachexia-inducing factors, present in media conditioned by certain human cancer cell lines, that induce cellular consequences recapitulated by cachectic-patient-derived plasma samples. For instance, RAGE-based biomarkers were identified herein as associated with the cachexia phenotype. Soluble RAGE (a negative regulator of RAGE signaling that acts as a sink for RAGE ligands) was found to be elevated in non-cachexia-inducing conditioned media in comparison to cachexia-inducing conditioned media. In contrast, levels of certain RAGE ligands, such as S100 proteins, were found to be elevated in cachexia-inducing conditioned media in comparison to non-cachexia-inducing conditioned media. HMGB1 does not induce the cachectic phenotype in any of Applicants' studies, nor is it elevated in cachectic patient plasma samples compared to non-cachectic cancer patient controls (is elevated in cachexia-inducing conditioned media), nor do inhibitory anti-HMGB 1 Abs inhibit the cachectic phenotype.

The presence and/or level of the cachexia-inducing proteins, e.g. the factors listed in the following Table, in patient plasma may serve as cachectic biomarker(s) useful for diagnosing and/or monitoring a patient with cachexia. Accordingly, the present invention also provides a method for diagnosing a patient with cachexia, comprising (i) obtaining plasma from the patient; and (ii) detecting the presence or level of a cachexia-inducing factor(s) selected from the following Table. The present invention also provides a method for monitoring a patient with cachexia, comprising (i) obtaining plasma from the patient; and (ii) detecting the presence or level of a cachexia-inducing factor(s) selected from the following Table.

| **Cachexia-Inducing Factors** | |
|---|---|
| BCAM | NGF |
| BTC | PDGFA |
| CCL5 | PDGFB |
| CCL27 | |
| DKK3 | S100A7 |
| EGFR | S100A8 |
| Follistatin-related peptide 1 | S100A9 |
| | |

| | **sRAGE (soluble RAGE)*** |
|---|---|
| IGFBP-1 | TNFRSF10C |
| | TNFSF18 |
| IL6 | TYRO3 |
| Lipolysis-stimulated lipoprotein receptor | |
| BCAM | |

| | |
|---|---|
| (*anti-cachectogenic, and as such, a potential therapeutic) | |

In particular embodiments, at least one or more of IGFBP-1, CCL27, AXL, CSF1, ICAM2, PIGF, TMP2, FGF4, KDR and CSF3 are increased in cachexia.

In other embodiments, a marker of cachexia or pre-cachexia is any one or more of three markers selected from S100A2 or S100A4, S100A8 or S100A9, and S100A7; four markers selected from S100A2 or S100A4, S100A8 or S100A9, S100A7 and S100A14; five markers selected from S100A2 or S100A4, S100A8 or S100A9, S100A7, S100A14, and S100P, which are increased in pre-cachexia or cachexia.

In other embodiments, a marker of cachexia or pre-cachexia is Basal cell adhesion molecule (BCAM), Buchang-tang (BCT), Chemokine ligand (CCL)5, CCL28, Dickkopf-related protein 3 (DKK3), Epidermal Growth Factor Receptor (EGFR), FASLG (Fas ligand), Fibroblast growth factor 4 (FGF4), Follistatin-related peptide 1, intercellular adhesion molecule (ICAM2), High Mobility Group (HMG1), Insulin Growth Factor-2 (IGF-2), Insulin Growth Factor Binding Protein-2 (IGFBP-2), IGFBP-6, interleukin-6 (IL6, Kinase insert domain receptor (KDR), lipolysis-stimulated receptor (LSR), NM (NME/NM23 Nucleoside Diphosphate Kinase 1), Nerve Growth Factor (NGF), Platelet Derived Growth Factor-A (PDGFA), PDGFB, PlGF (placenta growth factor), tyrosine-protein kinase receptor (TYRO3), Plasminogen activator inhibitor 1, tissue inhibitor of metalloproteinases (TIMP2), soluble Receptor for Advanced Glycation Endproducts (sRAGE)*, Tumor necrosis factor receptor superfamily member 10C (TNFRSF10C), and tumor necrosis factor superfamily member 18 (TNFSF18). All of the aforementioned markers are increased, with the exception of sRAGE, which is decreased. In other embodiments, the method further involves measuring an increase in an S100 family member selected from HMGB1, S100P, S100A2, S100A3, S100A4, S100A5, S100A7, S100A7A, S100A8, S100A9, S100A11, S100A12, S100A13, S100A14, and S100A15.

In certain embodiments, the patient has cachexia associated with a cancer. In a preferred embodiment, the patient has cachexia associated with a cancer, e.g., an epithelial-derived cancer or a mesenchemal-derived cancer, such as carcinomas and sarcomas and cancers of the skin, pancreas, stomach, colon, thorax, liver, gallbladder, musculoskeletal system, breast, lung, ovary, uterus, endometrium, prostrate, colon, skin, mouth, salivary, esophagus, head and neck, plus other tumors of the gastrointestinal tract.

In other embodiments, the patient has age-related weight loss or has age-related sarcopenia. In the case of age-related sarcopenia, a patient may present with muscle wasting without evidence of unintended weight loss. As used herein, at least a subset of patients with age-related weight loss or has age-related sarcopenia may be characterized by an increase in one or more S100 RAGE ligands and/or a decrease in soluble RAGE relative to a reference level.

In other embodiments, the patient has a disease-related cachexia that is not associated with cancer, but that like cancer-induced cachexia fails to respond to treatment with nutritional support and anti-inflammatory therapy. Such disease-related cachexia may, for example, be associated with AIDS, chronic obstructive lung or pulmonary disease (COPD), or heart disease (e.g. congestive heart failure, myocardial infarction or chronic heart failure) and, like cancer-induced cachexia are characterized by an increase in one or more S100 RAGE ligands and/or a decrease in soluble RAGE relative to a reference level.

In certain embodiments, the target cell is selected from a myocyte, an adipocyte, and a hepatocyte. In a preferred embodiment, the target cell is a myocyte.

The presence or absence of the herein disclosed marker(s) is measured in a tissue (e.g., biopsy) or bodily fluid from a pre-cachectic or cachectic subject. Bodily fluids used to evaluate the presence or absence of the herein disclosed markers include without limitation blood, serum, plasma, urine, and or saliva. For example, levels of biomarker are measured in the blood or biopsy before and after treatment in a subject.

Biopsy refers to the removal of a sample of tissue for purposes of diagnosis. For example, a biopsy is from a muscle, fat, a cancer or tumor, including a sample of tissue from an abnormal area or an entire tumor.

The disclosed methods involve comparing the presence or levels of the disclosed markers in a sample from a subject identified as having cancer or a pre-cancerous condition to the levels of the same markers in a reference (e.g., levels present in a corresponding sample from a healthy control). It is understood a reference includes a concurrently run control, or a standard created by assaying one or more non-cancer cells and collecting the marker data. Thus, the control sample is optionally a standard that is created and used continuously. The standard includes, for example, the average level of a biomarker in a sample from a non-cancer control group.

Also provided is a method of predicting or monitoring the efficacy of an anti-cachectic agent in a subject. The method comprises acquiring a biological sample, such as tissue or bodily fluid, from the subject after administering the agent to the subject. F or example, the tissue or bodily fluid is collected from the subject 1 to 60 minutes, hours, days, or weeks after administering the agent to the subject. The method further comprises detecting levels of one or more biomarkers delineated herein (e.g., S100 family proteins). A decrease in level(s) of one or more biomarkers is evidence of treatment efficacy. Thus, a decline in said increase or time is evidence of decreasing efficacy. Thus, it is preferred that biological samples be systematically acquired over time to monitor changes in marker levels.

### Panels and Arrays for Characterizing Pre-cachexia or Cachexia

The invention provides panels for characterizing cachexia comprising the aforementioned markers. In one embodiment, the panel is present on a protein array. Protein arrays are solid-phase ligand binding assay systems using immobilized proteins on surfaces which include glass, membranes, microtiter wells, mass spectrometer plates, and beads or other particles. The assays are highly parallel (multiplexed) and often miniaturized (microarrays, protein chips). Their advantages include being rapid and automatable, capable of high sensitivity, economical on reagents, and giving an abundance of data for a single experiment. Bioinformatics support is important; the data handling demands sophisticated software and data comparison analysis. However, the software is adapted from that used for DNA arrays, as can much of the hardware and detection systems.

One of the chief formats for characterizing cachexia is the capture array, in which ligand-binding reagents, which are usually antibodies, but can also be alternative protein scaffolds, peptides or nucleic acid aptamers, are used to detect target molecules in mixtures such as plasma or tissue extracts. In diagnostics, capture arrays are used to carry out multiple immunoassays in parallel, both testing for several analytes in individual sera for example and testing many serum samples simultaneously. In proteomics, capture arrays are used to quantitate and compare the levels of proteins in different samples in health and disease, i.e. protein expression profiling. Proteins other than specific ligand binders are used in the array format for in vitro functional interaction screens such as protein-protein, protein-DNA, protein-drug, receptor-ligand, enzyme-substrate, etc. The capture reagents themselves are selected and screened against many proteins, optionally in a multiplex array format against multiple protein targets.

For construction of arrays, sources of proteins include cell-based expression systems for recombinant proteins, purification from natural sources, production in vitro by cell-free translation systems, and synthetic methods for peptides. Many of these methods are automated for high throughput production. For capture arrays and protein function analysis, it is important that proteins be correctly folded and functional; this is not always the case, e.g., where recombinant proteins are extracted from bacteria under denaturing conditions. Nevertheless, arrays of denatured proteins are useful in screening antibodies for cross-reactivity, and selecting ligand binding proteins.

Protein arrays have been designed as a miniaturization of familiar immunoassay methods such as ELISA and dot blotting, often utilizing fluorescent readout, and facilitated by robotics and high throughput detection systems to enable multiple assays to be carried out in parallel. Physical supports include glass slides, silicon, microwells, nitrocellulose or PVDF membranes, and magnetic and other microbeads. While microdrops of protein delivered onto planar surfaces are the most familiar format, alternative architectures include CD centrifugation devices based on developments in microfluidics (Gyros, Monmouth Junction, N.J.) and specialized chip designs, such as engineered microchannels in a plate (e.g., The Living Chip.TM., Biotrove, Woburn, Mass.) and tiny 3D posts on a silicon surface (Zyomyx, Hayward Calif.). Particles in suspension are also used as the basis of arrays, providing they are coded for identification; systems include color coding for microbeads (Luminex, Austin, Tex.; Bio-Rad Laboratories), semiconductor nanocrystals (e.g., QDOTS.TM., Quantum Dot, Hayward, Calif.), barcoding for beads (ULTRAPLEX.TM. beads, SmartBead Technologies Ltd, Babraham, Cambridge, UK) and multimetal microrods (e.g., NANOBARCODES.TM. particles, Nanoplex Technologies, Mountain View, Calif.). Beads are optionally assembled into planar arrays on semiconductor chips (LEAPS.TM. technology, BioArray Solutions, Warren, N.J.).

Immobilization of proteins involves both the coupling reagent and the nature of the surface being coupled to. A good protein array support surface is chemically stable before and after the coupling procedures, allows good spot morphology, displays minimal nonspecific binding, does not contribute a background in detection systems, and is compatible with different detection systems. The immobilization method used are reproducible, applicable to proteins of different properties (size, hydrophilic, hydrophobic), amenable to high throughput and automation, and compatible with retention of fully functional protein activity. Orientation of the surface-bound protein is recognized as an important factor in presenting it to ligand or substrate in an active state; for capture arrays the most efficient binding results are obtained with orientated capture reagents, which generally require site-specific labeling of the protein.

Both covalent and noncovalent methods of protein immobilization are used and have various pros and cons. Passive adsorption to surfaces is methodologically simple, but allows little quantitative or orientational control. It may or may not alter the functional properties of the protein, and reproducibility and efficiency are variable. Covalent coupling methods provide a stable linkage, are applied to a range of proteins and have good reproducibility. However, orientation is variable. Furthermore, chemical derivatization may alter the function of the protein and requires a stable interactive surface. Biological capture methods utilizing a tag on the protein provide a stable linkage and bind the protein specifically and in reproducible orientation, but the biological reagent must first be immobilized adequately, and the array may require special handling and have variable stability.

Several immobilization chemistries and tags have been described for fabrication of protein arrays. Substrates for covalent attachment include glass slides coated with amino- or aldehyde-containing silane reagents. In the VERSALINX.TM. system (Prolinx, Bothell, Wash.) reversible covalent coupling is achieved by interaction between the protein derivatised with phenyldiboronic acid, and salicylhydroxamic acid immobilized on the support surface. This also has low background binding and low intrinsic fluorescence and allows the immobilized proteins to retain function. Noncovalent binding of unmodified protein occurs within porous structures such as HYDROGEL.TM. (PerkinElmer, Wellesley, Mass.), based on a 3-dimensional polyacrylamide gel; this substrate is reported to give a particularly low background on glass microarrays, with a high capacity and retention of protein function. Widely used biological coupling methods are through biotin/streptavidin or hexahistidine/Ni interactions, having modified the protein appropriately. Biotin may be conjugated to a poly-lysine backbone immobilized on a surface such as titanium dioxide (Zyomyx, Inc., Hayward, Calif.) or tantalum pentoxide (Zeptosens, Witterswil, Switzerland).

Array fabrication methods include robotic contact printing, ink-jetting, piezoelectric spotting and photolithography. A number of commercial arrayers are available [e.g. Packard Biosciences, Affymetrix Inc. and Genetix] as well as manual equipment [e.g., V & P Scientific]. Bacterial colonies are optionally robotically gridded onto PVDF membranes for induction of protein expression in situ.

At the limit of spot size and density are nanoarrays, with spots on the nanometer spatial scale, enabling thousands of reactions to be performed on a single chip less than 1 mm square. BioForce Nanosciences Inc. and Nanolink Inc., for example, have developed commercially available nanoarrays.

Fluorescence labeling and detection methods are widely used. The same instrumentation as used for reading DNA microarrays is applicable to protein arrays. For differential display, capture (e.g., antibody) arrays are probed with fluorescently labeled proteins from two different cell states, in which cell lysates are directly conjugated with different fluorophores (e.g. Cy-3, Cy-5) and mixed, such that the color acts as a readout for changes in target abundance. Fluorescent readout sensitivity is amplified 10-100 fold by tyramide signal amplification (TSA) (PerkinElmer Lifesciences). Planar waveguide technology (Zeptosens) enables ultrasensitive fluorescence detection, with the additional advantage of no intervening washing procedures. High sensitivity is achieved with suspension beads and particles, using phycoerythrin as label (Luminex) or the properties of semiconductor nanocrystals (Quantum Dot). A number of novel alternative readouts have been developed, especially in the commercial biotech arena. These include adaptations of surface plasmon resonance (HTS Biosystems, Intrinsic Bioprobes, Tempe, Ariz.), rolling circle DNA amplification (Molecular Staging, New Haven, Conn.), mass spectrometry (Intrinsic Bioprobes; Ciphergen, Fremont, Calif.), resonance light scattering (Genicon Sciences, San Diego, Calif.) and atomic force microscopy [BioForce Laboratories].

Capture arrays form the basis of diagnostic chips and arrays for expression profiling. They employ high affinity capture reagents, such as conventional antibodies, single domains, engineered scaffolds, peptides or nucleic acid aptamers, to bind and detect specific target ligands in high throughput manner.

Antibody arrays have the required properties of specificity and acceptable background, and some are available commercially (BD Biosciences, San Jose, Calif.; Clontech, Mountain View, Calif.; BioRad; Sigma, St. Louis, Mo.). Antibodies for capture arrays are made either by conventional immunization (polyclonal sera and hybridomas), or as recombinant fragments, usually expressed in E. coli, after selection from phage or ribosome display libraries (Cambridge Antibody Technology, Cambridge, UK; BioInvent, Lund, Sweden; Affitech, Walnut Creek, Calif.; Biosite, San Diego, Calif.). In addition to the conventional antibodies, Fab and scFv fragments, single V-domains from camelids or engineered human equivalents (Domantis, Waltham, Mass.) are optionally useful in arrays.

The term scaffold refers to ligand-binding domains of proteins, which are engineered into multiple variants capable of binding diverse target molecules with antibody-like properties of specificity and affinity. The variants are produced in a genetic library format and selected against individual targets by phage, bacterial or ribosome display. Such ligand-binding scaffolds or frameworks include Affibodies based on S. aureus protein A (Affibody, Bromma, Sweden), Trinectins based on fibronectins (Phylos, Lexington, Mass.) and Anticalins based on the lipocalin structure (Pieris Proteolab, Freising-Weihenstephan, Germany). These are used on capture arrays in a similar fashion to antibodies and have advantages of robustness and ease of production.

Nonprotein capture molecules, notably the single-stranded nucleic acid aptamers which bind protein ligands with high specificity and affinity, are also used in arrays (SomaLogic, Boulder, Colo.). Aptamers are selected from libraries of oligonucleotides by the Selex.TM. procedure (SomaLogic, Boulder, Colo.) and their interaction with protein is enhanced by covalent attachment, through incorporation of brominated deoxyuridine and UV-activated crosslinking (photoaptamers). Photocrosslinking to ligand reduces the crossreactivity of aptamers due to the specific steric requirements. Aptamers have the advantages of ease of production by automated oligonucleotide synthesis and the stability and robustness of DNA; on photoaptamer arrays, universal fluorescent protein stains are used to detect binding.

Protein analytes binding to antibody arrays are detected directly or indirectly, for example, via a secondary antibody. Direct labeling is used for comparison of different samples with different colors. Where pairs of antibodies directed at the same protein ligand are available, sandwich immunoassays provide high specificity and sensitivity and are therefore the method of choice for low abundance proteins such as cytokines; they also give the possibility of detection of protein modifications. Label-free detection methods, including mass spectrometry, surface plasmon resonance and atomic force microscopy, avoid alteration of ligand. What is required from any method is optimal sensitivity and specificity, with low background to give high signal to noise. Since analyte concentrations cover a wide range, sensitivity has to be tailored appropriately. Serial dilution of the sample or use of antibodies of different affinities are solutions to this problem. Proteins of interest are frequently those in low concentration in body fluids and extracts, requiring detection in the pictogram (pg) range or lower, such as cytokines or the low expression products in cells.

An alternative to an array of capture molecules is one made through molecular imprinting technology, in which peptides (e.g., from the C-terminal regions of proteins) are used as templates to generate structurally complementary, sequence-specific cavities in a polymerizable matrix; the cavities can then specifically capture (denatured) proteins that have the appropriate primary amino acid sequence (ProteinPrint.TM., Aspira Biosystems, Burlingame, Calif.).

Another methodology which is useful diagnostically and in expression profiling is the ProteinChip.RTM. array (Ciphergen, Fremont, Calif.), in which solid phase chromatographic surfaces bind proteins with similar characteristics of charge or hydrophobicity from mixtures such as plasma or tumor extracts, and SELDI-TOF mass spectrometry is used to detection the retained proteins.

Large-scale functional chips have been constructed by immobilizing large numbers of purified proteins and are used to assay a wide range of biochemical functions, such as protein interactions with other proteins, drug-target interactions, enzyme-substrates, etc. Generally they require an expression library, cloned into *E. coli,* yeast or similar from which the expressed proteins are then purified, e.g., via a His tag and immobilized. Cell free protein transcription/translation is a viable alternative for synthesis of proteins which do not express well in bacterial or other *in vivo* systems.

For detecting protein-protein interactions, protein arrays are in vitro alternatives to the cell-based yeast two-hybrid system and are useful where the latter is deficient, such as interactions involving secreted proteins or proteins with disulphide bridges. High-throughput analysis of biochemical activities on arrays has been described for yeast protein kinases and for various functions (protein-protein and protein-lipid interactions) of the yeast proteome, where a large proportion of all yeast open-reading frames was expressed and immobilized on a microarray. Large-scale proteome chips are also useful in identification of functional interactions, drug screening, etc. (Proteometrix, Branford, Conn.).

As a two-dimensional display of individual elements, a protein array is used to screen phage or ribosome display libraries, in order to select specific binding partners, including antibodies, synthetic scaffolds, peptides and aptamers. In this way, library against library screening is carried out. Screening of drug candidates in combinatorial chemical libraries against an array of protein targets identified from genome projects is another application of the approach.

Multiplexed bead assays use a series of spectrally discrete particles that are used to capture and quantitate soluble analytes. The analyte is then measured by detection of a fluorescence-based emission and flow cytometric analysis. Multiplexed bead assays generate data that is comparable to ELISA based assays, but in a multiplexed or simultaneous fashion. Concentration of unknowns is calculated for the cytometric bead array as with any sandwich format assay, i.e., through the use of known standards and by plotting unknowns against a standard curve. Further, multiplexed bead assays allow quantification of soluble analytes in samples never previously considered due to sample volume limitations. In addition to the quantitative data, powerful visual images are generated revealing unique profiles or signatures that provide the user with additional information at a glance.

In some examples of the disclosed methods, when the level of expression of a biomarker(s) is assessed, the level is compared with the level of expression of the biomarker(s) in a reference standard. By reference standard is meant the level of expression of a particular biomarker(s) from a sample or subject lacking a cancer, at a selected stage of cancer, or in the absence of a particular variable such as a therapeutic agent. Alternatively, the reference standard comprises a known amount of biomarker. Such a known amount correlates with an average level of subjects lacking a cancer, at a selected stage of cancer, or in the absence of a particular variable such as a therapeutic agent. A reference standard also includes the expression level of one or more biomarkers from one or more selected samples or subjects as described herein. For example, a reference standard includes an assessment of the expression level of one or more biomarkers in a sample from a subject that does not have a cancer, is at a selected stage of progression of a cancer, or has not received treatment for a cancer. Another exemplary reference standard includes an assessment of the expression level of one or more biomarkers in samples taken from multiple subjects that do not have a cancer, are at a selected stage of progression of a cancer, or have not received treatment for a cancer.

When the reference standard includes the level of expression of one or more biomarkers in a sample or subject in the absence of a therapeutic agent, the control sample or subject is optionally the same sample or subject to be tested before or after treatment with a therapeutic agent or is a selected sample or subject in the absence of the therapeutic agent. Alternatively, a reference standard is an average expression level calculated from a number of subjects without a particular cancer. A reference standard also includes a known control level or value known in the art. In one aspect of the methods disclosed herein, it is desirable to age-match a reference standard with the subject diagnosed with a cancer.

In one technique to compare protein levels of expression from two different samples (e.g., a sample from a subject diagnosed with a cancer and a reference standard), each sample is separately subjected to 2D gel electrophoresis. Alternatively, each sample is differently labeled and both samples are loaded onto the same 2D gel. See, e.g., Unlu et al. Electrophoresis, 1997; 18:2071-2077, which is cited herein for at least its teachings of methods to assess and compare levels of protein expression. The same protein or group of proteins in each sample is identified by the relative position within the pattern of proteins resolved by 2D electrophoresis. The expression levels of one or more proteins in a first sample is then compared to the expression level of the same protein(s) in the second sample, thereby allowing the identification of a protein or group of proteins that is expressed differently between the two samples (e.g., a biomarker). This comparison is made for subjects before and after they are suspected of having a cancer, before and after they begin a therapeutic regimen, and over the course of that regimen.

In another technique, the expression level of one or more proteins is in a single sample as a percentage of total expressed proteins. This assessed level of expression is compared to a preexisting reference standard, thereby allowing for the identification of proteins that are differentially expressed in the sample relative to the reference standard.

There are a variety of sequences related to biomarkers as well as any other protein disclosed herein that are disclosed on GenBank, and these sequences and others are herein cited in their entireties as well as for individual subsequences contained therein. Thus, a variety of sequences are provided herein and these and others are found in GenBank at http://www.ncbi.nih.gov/entrez/query.fcgi. Those of skill in the art understand how to resolve sequence discrepancies and differences and to adjust the compositions and methods relating to a particular sequence to other related sequences. Primers and/or probes are designed for any sequence given the information disclosed herein and known in the art.

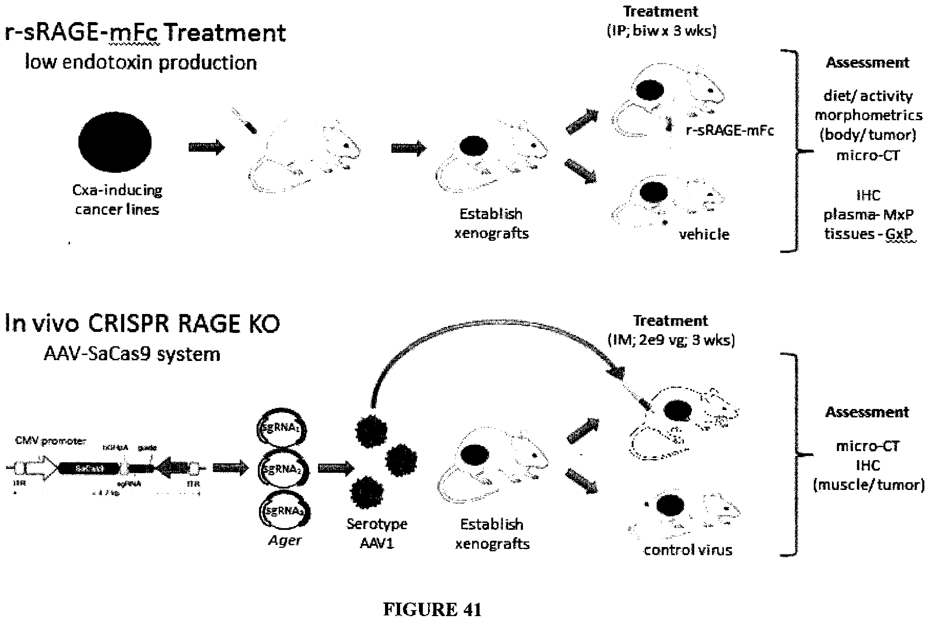

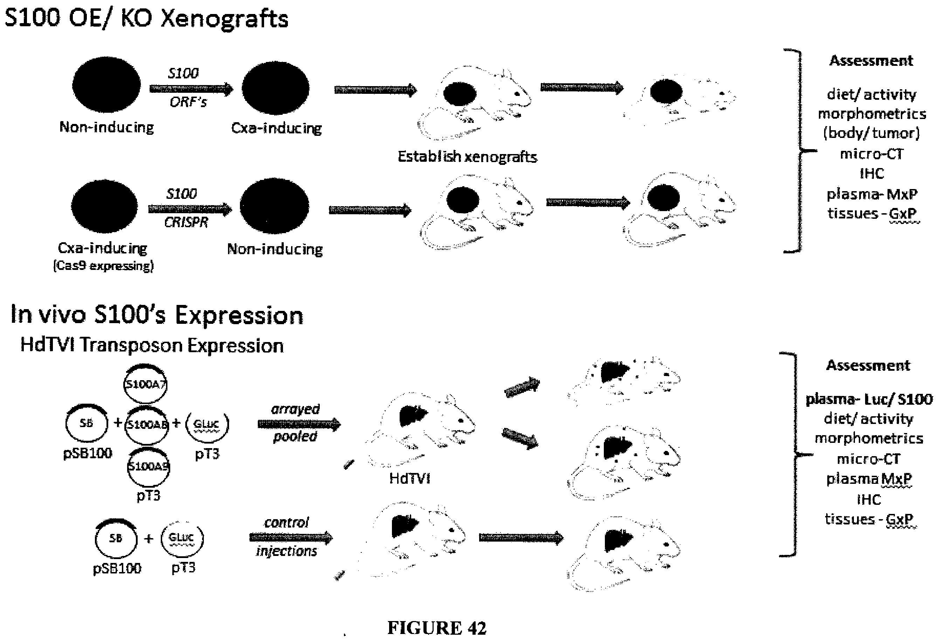

Screens for *in vivo* validation of the RAGE pathway in cachexia, eg. cancer cachexia may be conducted by either targeting RAGE receptors or RAGE ligands, as illustrated in Figures 41 and 42. In particular the aspects of the CRISPR system may be used to knock out the RAGE receptor using AAV-Cas9 system (e.g. AAV-SaCas9) or knock out a RAGE ligand by expressing a CRISPR-Cas system with a guide RNA targeting a genomic locus comprising the coding sequence of a particular RAGE ligand. With respect to general information on CRISPR-Cas Systems, components thereof, and delivery of such components, including methods, materials, delivery vehicles, vectors, particles, AAV, and making and using thereof, including as to amounts and formulations, all useful in the practice of the instant invention, reference is made to general information on CRISPR-Cas Systems, mention is made of the following:
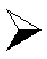 Multiplex genome engineering using CRISPR/Cas systems. Cong, L., Ran, F.A., Cox, D., Lin, S., Barretto, R., Habib, N., Hsu, P.D., Wu, X., Jiang, W., Marraffini, L.A., & Zhang, F. Science Feb 15;339(6121):819-23 (2013);
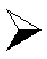 RNA-guided editing of bacterial genomes using CRISPR-Cas systems. Jiang W., Bikard D., Cox D., Zhang F, Marraffini LA. Nat Biotechnol Mar;31(3):233-9 (2013);
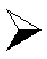 One-Step Generation of Mice Carrying Mutations in Multiple Genes by CRISPR/Cas-Mediated Genome Engineering. Wang H., Yang H., Shivalila CS., Dawlaty MM., Cheng AW., Zhang F., Jaenisch R. Cell May 9;153(4):910-8 (2013);
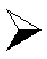 Optical control of mammalian endogenous transcription and epigenetic states. Konermann S, Brigham MD, Trevino AE, Hsu PD, Heidenreich M, Cong L, Platt RJ, Scott DA, Church GM, Zhang F. Nature. 2013 Aug 22;500(7463):472-6. doi: 10.1038/Nature12466. Epub 2013 Aug 23;
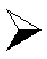 Double Nicking by RNA-Guided CRISPR Cas9 for Enhanced Genome Editing Specificity. Ran, FA., Hsu, PD., Lin, CY., Gootenberg, JS., Konermann, S., Trevino, AE., Scott, DA., Inoue, A., Matoba, S., Zhang, Y., & Zhang, F. Cell Aug 28. pii: S0092-8674(13)01015-5. (2013);
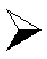 DNA targeting specificity of RNA-guided Cas9 nucleases. Hsu, P., Scott, D., Weinstein, J., Ran, FA., Konermann, S., Agarwala, V., Li, Y., Fine, E., Wu, X., Shalem, O., Cradick, TJ., Marraffini, LA., Bao, G., & Zhang, F. Nat Biotechnol doi:10.1038/nbt.2647 (2013);
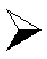 Genome engineering using the CRISPR-Cas9 system. Ran, FA., Hsu, PD., Wright, J., Agarwala, V., Scott, DA., Zhang, F. Nature Protocols Nov;8(11):2281-308. (2013);
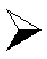 Genome-Scale CRISPR-Cas9 Knockout Screening in Human Cells. Shalem, O., Sanjana, NE., Hartenian, E., Shi, X., Scott, DA., Mikkelson, T., Heckl, D., Ebert, BL., Root, DE., Doench, JG., Zhang, F. Science Dec 12. (2013). [Epub ahead of print];
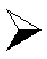 Crystal structure of cas9 in complex with guide RNA and target DNA. Nishimasu, H., Ran, FA., Hsu, PD., Konermann, S., Shehata, SI., Dohmae, N., Ishitani, R., Zhang, F., Nureki, O. Cell Feb 27. (2014). 156(5):935-49;
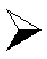 Genome-wide binding of the CRISPR endonuclease Cas9 in mammalian cells. Wu X., Scott DA., Kriz AJ., Chiu AC., Hsu PD., Dadon DB., Cheng AW., Trevino AE., Konermann S., Chen S., Jaenisch R., Zhang F., Sharp PA. Nat Biotechnol. (2014) Apr 20. doi: 10.1038/nbt.2889,
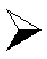 CRISPR-Cas9 Knockin Mice for Genome Editing and Cancer Modeling, Platt et al., Cell 159(2): 440-455 (2014) DOI: 10.1016/j.cell.2014.09.014,
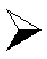 Development and Applications of CRISPR-Cas9 for Genome Engineering, Hsu et al, Cell 157, 1262-1278 (June 5, 2014) (Hsu 2014),
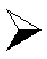 Genetic screens in human cells using the CRISPR/Cas9 system, Wang et al., Science. 2014 January 3; 343(6166): 80-84. doi:10.1126/science.1246981,
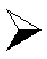 Rational design of highly active sgRNAs for CRISPR-Cas9-mediated gene inactivation, Doench et al., Nature Biotechnology published online 3 September 2014; doi:10.1038/nbt.3026, and
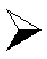 In vivo interrogation of gene function in the mammalian brain using CRISPR-Cas9, Swiech et al, Nature Biotechnology published online 19 October 2014; doi: 10.1038/nbt.3055,
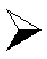 Genome-scale transcriptional activation by an engineered CRISPR-Cas9 complex. Konermann, et al, Nature published online 10 December 2014, doi: 10.1038/nature14136,
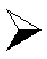 Genome-wide CRISPR Screen in a Mouse Model of tumor Growth and Metastasis. Chen, et al, Cell published 12 March 2015, doi: 10.1016/j.cell.2015.02.038, and
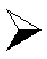 In vivo genome editing using Staphyloccocus aureus Cas9. Ran et al, Nature published online 9 April 2015, doi: 10.1038/nature14299 .

In aspects of the invention, screening may be via High-throughput means or *in vivo* animal model systems in which expression, epigentic state and loss of function metrics are utilized. In a preferred embodiment, a GeCKO library may be utilized in the screen.

### Small Molecules for Identifying a Patient as Having Pre-Cachexia or Cachexia

The disclosure provides a small molecule which associates with the RAGE receptor as a means of identifying and treating pre-cachexia, cardiovascular cachexia, insulin resistance, and skeletal muscle wasting. Previous reports have disclosed molecules which treat mild Alzheimer's disease (AD). Herein is provided a summary of molecules which have been identified for uses in treating Alzheimer's disease.

Alzheimer's Disease (AD) is a neurodegenerative disorder with aspects of inflammatory, metabolic and vascular pathology. An overproduction of amyloid beta (Aβ) has been implicated as the leading mechanistic factor in AD pathology. It has been found that amyloid beta binds to a receptor known as Receptor for Advanced Glycation Endproducts (RAGE) which is an immunoglobulin supergene family member expressed on multiple cell types in the brain and the periphery. Previous studies support that RAGE is strongly associated with the pathogenesis of Alzheimer's Disease. The authors investigated an orally active, centrally acting antagonist of RAGE-RAGE ligand interaction compound TTP448, developed by TransTech Pharma for the treatment of mild AD. Investigators discovered that dosing regimens favored 5 mg/day of TTP448 with a slight significance in efficacy after 18 months (Burstein et al. BMC Neurology 2014, 14:12).

Deane et al. recently identified a specific high-affinity RAGE-specific inhibitor (FPS-ZM1) that blocked Aβ binding to the V domain of RAGE and inhibited Aβ40- and Aβ42-induced cellular stress in RAGE-expressing cells in vitro and in the mouse brain in vivo. FPS-ZMI is a tertiary amide with a hydrophobic cyclohexane, an electron-rich aromatic group, and a deactivated aromatic group (Deane, R. et al., J Clin Investigation 2012, Vol. 122, No. 4, pp. 1378-1392).

Previous to research conducted by Han and co-workers, most studies of RAGE inhibition have focused on protein inhibitors such as sRAGE and RAGE antibodies. Han et al herein, report the ligand-based development of a novel series of 2-aminopyrimidines that are RAGE inhibitors that may serve as potential AD therapeutics. The authors modified the the 2-aminopentanoic acid moiety of the argpyrimidine. Compounds 4-(4-(2-cyclohexylethoxy)phenyl)-N-(3-(2-(diethylamino)ethoxy)-phenyl)-6-methylpyrimidin-2-amine (53), 4,6-bis(4-chlorophenyl)-N-(3-(2-(diethylamino)ethoxy)phenyl)-pyrimidin-2-amine (59), and 4,6-bis(4-chlorophenyl)-N-(2-(2-(diethylamino)ethoxy)phenyl)-pyrimidin-2-amine (60) showed highest affinity to ligand. Compound 59 showed most potent A-beta brain entry inhibition. Compound 60 exhibited the best in vitro activity. Compound 59 had the most significant inhibition.

In a study by Lee et al., a series of thiazole derivatives were designed, and prepared to develop RAGE antagonist for the treatment of Alzheimer's disease (AD). SAR studies were performed to optimize inhibitory activity on Ab-RAGE binding. Specifically, SAR data confirmed that the alkylamino group of part A was essential for inhibitory activity of Aβ-RAGE binding, and compounds that can bind to RAGE inhibited transport of Aβ. (Lee et al Bioorganic & Medicinal Chemistry Letters 22 (2012) 7555-7561.)

In an investigation by Yang et al., the authors studied whether RAGE plays a role in early brain injury from intracerebral hemorrhage (ICH). RAGE expression and activation after injury were examined in a rat model of ICH with or without administration of a recently identified small molecule FPS-ZM1 which showed promising activity as a RAGE-specific antagonist. It was found that RAGE expression and its ligand high-mobility group protein b1 were increased 12 hours after ICH, along with blood-brain barrier permeability and perihematomal nerve fiber injury. Results showed that FPS-ZM1 administration resulted in, inter alia, significant improvements of BBB damage, brain edema, motor dysfunction, and nerve fiber injury. FPS-ZM1 also reversed FeCl2-induced RAGE and nuclear factor κB p65 upregulation as shown in the publication. (Yang et al., Stroke. 2015;46:1328-1336.)

RAGE comprises a heterogeneous group of compounds formed by the Maillard chemical process, which refers to a non-enzymatic glycation of free amino groups of proteins, lipids, and nucleic acids, mostly, by reducing sugars and reactive aldehydes. In relation to cardiovascular diseases, elevated serum AGEs are observed in patients with coronary artery disease and thereby associated with aortic stiffness. AGEs may also induce local inflammation and intracellular ROS which all together lead to vascular alterations and the expression of a variety of atherosclerosis-related genes, including VEGF and RAGE. AGE modification of low-density lipoprotein (LDL) results in reduced LDL plasma clearance contributing significantly to increased LDL in vivo, and potentially to atherosclerosis. In aspect of the invention, compounds, and molecules which are used as a therapeuctic in cardiovascular diseases which bind to the RAGE receptor are envisioned as identifying and treating pre-cachexia (Nedic et al Free Radical Research, August 2013; 47(Suppl. 1): 28-38.)

A series of compounds were designed based on a large screening library capable of binding to RAGE and inhibiting the RAGE-Aβ interaction in a study by Ross et al. Thirteen compounds were identified based on the highest positive binding constant which inhibit the RAGE-Aβ interaction. Compounds were investigated to further identify moieties with high binding affinities to the RAGE receptor. Results showed that NTR-N1A2B1 exhibited the most promising properties and are currently being further investigated in both *in vitro* and *in vivo* studies towards the treatment Alzheimer's Disease. (Ross, N. et al. Tetrahedron 2013, 69, 7653-7658.)

S100 proteins are calcium-binding proteins that represent a subfamily of EF-hand proteins. Penumutchu and co-workers found additional significance of the V domain in RAGE homodimerization and signal transduction. They were able to characterize the molecular interactions between S100P and the V domain of RAGE and further identify the role of crucial residues at the interface of both proteins. (Penumutchu SR, Chou R-H, Yu C (2014) Structural Insights into Calcium-Bound S100P and the V Domain of the RAGE Complex. PLoS ONE 9(8): e103947.doi:10.1371/journal.pone.0103947)

RAGE antagonists that may be used in the methods of the present invention include azole compounds such as those provided for in US 2009/0035302 A1 to Mjalli et al.,. These azole compounds include compounds of the formula: 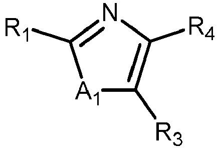 wherein for the compounds of Formula (1):
R₁ comprises -hydrogen, -aryl, -heteroaryl, -cycloalkyl, -heterocyclyl, -alkyl, - alkenyl, alkynyl. -alkylene-aryl, - alkylene heteroaryl, - alkylene -heterocyclyl, -alkylene-cycloalkyl, -fused cycloalkylaryl, -fused cycloalkylheteroaryl, -fused heterocyclylaryl, -fused heterocyclylheteroaryl, -alkylene fused cycloalkylaryl, -alkylene -fused cyclo alkyleneheteroaryl, - alkylene -fused heterocyclylaryl, - alkylene -fused heterocyclylheteroaryl, or -G₁-G₂-G₃-R₅
wherein
G₁ and G₃ independently comprise alkylene, alkenylene, alkynylene, cyclo alkylene, heterocyclylene, arylene, heteroarylene, arylalkylene, heteroarylalkylene, arylalkenylen, heteroarylalkenylene, or a direct bond;
G₂ comprises -O-, -S-, -S(O)- N(R₆)-, S(O)₂-, -C(O)-, -O-C(O)-, -C(O)-O-, - C(O))N(R6)-, N(R6)C(O)- S(O2)N(R6)- , -N(R6)S(O2)-, -O-alkylene-C(O)-, -(O)C-alkylene-O-, -O-alkylene-, alkylene-O-, alkylne, alkenylene, alkynylene, cycloalkylene, heterocyclylene, arylene, heteroarylene, fused cycloalkylarylene, fused cycloalkylheteroaarylene, fused heterocycclylarylene, fused heterocyclelhteroarylen, or a direct bond, wherein R6 comprises hydrogen, aryl, alkyl, -alkylene-aryl, alkoxy, or -alkylene-O-aryl; and
R₅ comprises hydrogen, aryl, heteroaryl, cycloalkyl, heterocyclyl, alkyl, alkenyl, alkynyl, -alkylene-aryl, -alkylene-heteroaryl, -alkylene-heterocyclyl, -alkylene-cycloalkyl, fused cycloalkylaryl, fused cycloalkylheteroary, fused heterocyclylaryl, fused hterocyclylheteroaryl, - alkylene-fused cyclcoalkylaryl, -alkylene-fused cycloalkylheteroaryl, -alkylene-fused heterocyclylaryl, or-alkylene-fused heterocyclylheteroaryl;
A1 comprises O,S, or -N(R2)-;
wherein
R2 comprises
   a) -H;
   b) -aryl;
   c) -heteroaryl;
   d0 -cycloalkyl
   e) heterocyclyl;
   f) -alkyl;
   g) -alkenyl;
   h) -alkynyl;
   i) alkylene-aryl,
   j) -alkylene-heteroaryl,
   k) -alkylene-heterocyclyl,
   l) -alkylene-cycloalkyl;
   m) -fused cycloalkylaryl,
   n) -fused cycloalkylheteroaryl,
   ο) -fused heterocyclylaryl,
   p) -fused heterocyclylheteroaryl;
   q) -alkylene-fused cycloalkylaryl,
   r) -alkylene-fused cycloalkylheteroaryl,
   s) -alkylene-fused heterocyclylarly,
   t) -alkylene-fused heterocyclylheteroaryl; or
   u) a group of the forumula 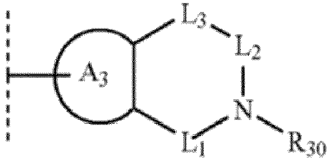 wherein
      A₃ comprises an aryl or heteroaryl group;
      L₁ and L₂ independently comprise alkylene or alkenylene; and
      L3 comprises a direct bond, alkylene, -O-, -S-, -S(O2)-, -C(O)-, -CON(H)-, - NHC(O)-, -NHCON(H)-, -NHSO2-, -SO2N(H)-, -C(O)-O-, -O-C(O)-, -NHSO2NH-, 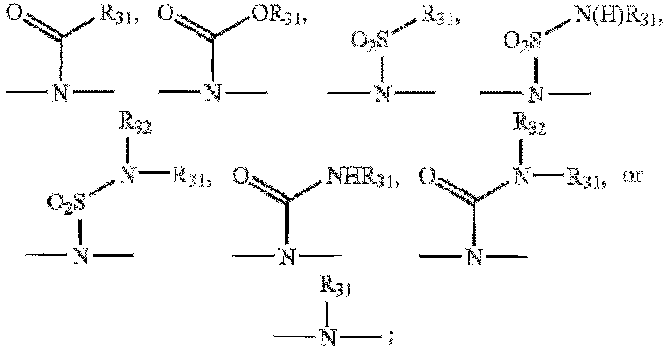
   wherein R₃₀, R₃₁, and R₃₂ independently comprise hydrogen, aryl, heteroaryl, alkyl, alky lene-aryl, or -alkylene-heteroaryl;
R₃ and R₄ independently comprise
   a) -hydrogen,
   b) -halogen,
   c) -hydroxyl,
   d) -cyano,
   e) -carbamoyl,
   f) -carboxyl,
   g) -aryl,
   h) -heteroaryl,
   i) -cycloalkyl,
   j) -heterocyclyl,
   k) -alkyl,
   1) -alkenyl,
   m) -alkynyl,
   n) -alkylene-aryl,
   ο) -alkylene-heteroaryl,
   p) -alkylene-heterocyclyl,
   q) -alkylene-cycloalkyl,
   r) -fused cycloalkylaryl,
   s) -fused cycloalkylheteroaryl,
   t) -fused heterocyclylaryl,
   u) -fused heterocyclylheteroaryl,
   v) -alkylene-fused cycloalkylaryl,
   w) -alkylene-fused cycloalkylheteroaryl,
   x) -alkylene-fused heterocyclylaryl,
   y) -alkylene-fused heterocyclylheteroaryl;
   z) -C(O)-O-alkyl;
   aa) -C(O)-O-alkylene-aryl;
   bb) -C(O)-NH-alkyl;
   cc) -C(O)-NH-alkylene-aryl;
   dd) -SO₂-alkyl;
   ee) -SO₂-alkylene-aryl;
   ff) -SO₂-aryl;
   gg) -SO₂-NH-alkyl;
   hh) -SO₂-NH-alkylene-aryl;
   ii) -C(O)-alkyl;
   jj) -C(O)-alkylene-aryl;
   kk) -G₄-G5-G₆-R₇;
   11) -Y₁-alkyl;
   mm) -Y₁-aryl;
   nn) -Y₁-heteroaryl;
   oo) -Y₁-alkylene-aryl;
   pp) -Y₁-alkylene-heteroaryl;
   qq) -Y₁-alkylene-NR₉R₁₀; or
   rr) -Y₁-alkylene-W₁-R₁ 1;
wherein
   G₄ and G₆ independently comprise alkylene, alkenylene, alkynylene, cycloalkylene, heterocyclylene, arylene, heteroarylene, (aryl)alkylene, (heteroaryl)alkylene, (aryl)alkenylene, (heteroaryl)alkenylene, or a direct bond;
   G₅ comprises -O-, -S-, -N(R₈)-, -S(O)-, -S(O)₂-, -C(O)-, -O-C(O)-, -C(O)-O-, -C(O)N(R₈)-, N(R₈)C(O)-, -S(O₂)N(R₈)-, N(R₈)S(O₂)-, -O-alkylene-C (O), -(O)C-alkylene-O-, -O-alkylene-, -alkylene-O-, alkylene, alkenylene, alkynylene, cycloalkylene, heterocyclylene, arylene, heteroarylene, fused cycloalkylarylene, fused cycloalky-lheteroarylene, fused heterocyclylarylene, fused het-erocyclylheteroarylene, or a direct bond, wherein R₈ comprises -hydrogen, -aryl, -alkyl, -alkylene-aryl, or -alkylene-O-aryl;
   R₇ comprises hydrogen, aryl, heteroaryl, cycloalkyl, heterocyclyl, alkyl, alkenyl, alkynyl, alkylene-aryl, -alkylene-heteroaryl, -alkylene-heterocyclyl, -alkylene-cycloalkyl, fused cycloalkylaryl, fused cycloalkylheteroaryl, fused heterocyclylaryl, fused heterocyclylheteroaryl, alkylene-fused cycloalkylaryl, -alkylene-fused cycloalkylheteroaryl, -alkylene-fused heterocyclylaryl, or -alkylene-fused heterocyclylheteroaryl;
   Y₁ and W₁ independently comprise -CH₂-, -O-, -N(H), -S-, SO₂-, -CON(H)-, -NHC(O)-, -NHCON(H)-, -NHSO₂-, -SO₂N(H)-, -C-(O)-O-, -NHSO₂NH-, -O-CO-, 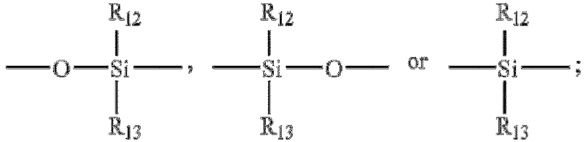 wherein R₁₂ and R₁₃ independently comprise aryl, alkyl, -alkylene-aryl, alkoxy, or -alkylene-O-aryl; and
   R₉, R₁₀, and R₁ independently comprise aryl, heteroaryl, alkyl, -alkylene-heteroaryl, or -alkylene-aryl; and R₉ and R₁₀ may be taken together to form a ring having the formula -(CH₂)*ₒ*-X₁-(CH₂)*ₚ*-bonded to the nitrogen atom to which R₉ and R₁₀ are attached,
      wherein
      o and p are, independently, 1, 2, 3, or 4; and
      X₁ comprises a direct bond, -CH₂-, -O-, -S-, -S(O₂)-, -C(O)-, -CON (H)-, -NHC(O)-, -NHCON(H)-, -NHSO₂-, -SO₂N(H)-, -C(O)-O-, -O-C(O)-, -NHSO₂NH-, 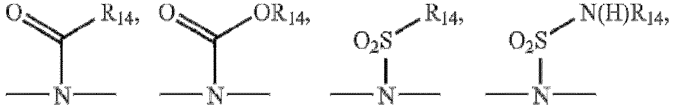 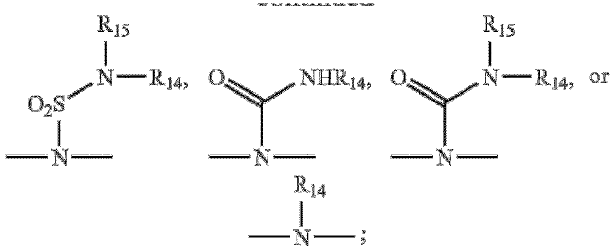 wherein R₁₄ and R₁₅ independently hydrogen, aryl, heteroaryl, alkyl, -alkylene-aryl, or -alkylene-heteroaryl;
         wherein
the aryl and/or alkyl group(s) in R₁, R₂, R₃, R₄, R₅, R₆, R₇, R₈, R₉, R₁₀, R₁₁, R₁₂, R₁₃, R₁₄, and R₁₅ may be optionally substituted 1-4 times with a substituent group, wherein said substituent group(s) or the term substituted refers to a group comprising:
   a) -H,
   b) -halogen,
   c) -hydroxyl,
   d) -cyano,
   e) -carbamoyl,
   f) -carboxyl,
   g) -Y₂-alkyl;
   h) -Y₂-aryl;
   i) -Y₂-heteroaryl;
   j) -Y₂-alkylene-heteroarylaryl;
   k) -Y₂-alkylene-aryl;
   l) -Y₂-alkylene-W₂-R₁₈;
   m) -Y₃-Y₄-NR₂₃R₂₄,
   n) -Y₃-Y₄-NH-C(=NR₂₅)NR₂₃R₂₄,
   o) -Y₃-Y⁴-C(=NR₂₅)NR₂₃R₂₄, or
   p) -Y₃-Y₄-Y₅-A₂,
      wherein
      Y₂ and W₂ independently comprise -CH₂-, -O-, -N(H), -S-, SO₂-, -CON (H)-, -NHC(O)-, -NHCON(H)-, -NHSO₂-, -SO₂N(H)-, -C(O)-O-, -NHSO₂NH-, -O-S(O)₂-, -O-CO-, 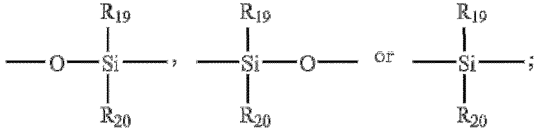 wherein;
         R₁₉ and R₂₀ independently comprise hydrogen, aryl, alkyl, -alkylene-aryl, alkoxy, or -alkylene-O-aryl; and
      R₁₈ comprises aryl, alkyl, -alkylene-aryl, -alkylene-heteroaryl, and -alkylene-O-aryl;
      Y₃ and Y₅ independently comprise a direct bond, -CH₂-, -O-, -N(H), -S-, SO₂-, -C(O)-, -CON(H)-, -NHC(O)-, -NH-CON(H)-, -NHSO₂-, -SO₂N(H)-, -C(O)-O-, NHSO₂NH-, -OCO-, 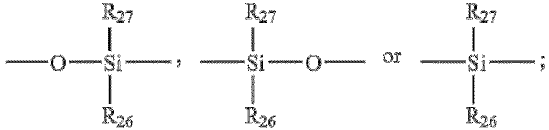 wherein R₂₇ and R₂₆ independently comprise aryl, alkyl, -alkylene-aryl, alkoxy, or -alkyl-O-aryl;
Y₄ comprises
   a) -alkylene;
   b) -alkenylene;
   c) -alkynylene;
   d) -arylene;
   e) -heteroarylene;
   f) -cycloalkylene;
   g) -heterocyclylene;
   h) -alkylene-arylene;
   i) -alkylene-heteroarylene;
   j) -alkylene-cycloalkylene;
   k) -alkylene-heterocyclylene;
   l) -arylene-alkylene;
   m) -heteroarylene-alkylene;
   n) -cycloalkylene-alkylene;
   o) -heterocyclylene-alkylene;
   p) -O-;
   q) -S-;
   r) -S(O₂)-; or
   s) -S(O)-;
   wherein said alkylene groups may optionally contain one or more O, S, S(O), or SO₂ atoms;
A₂ comprises
   a) heterocyclyl, fused arylheterocyclyl, or fused heteroarylheterocyclyl, containing at least one basic nitrogen atom,
   b) -imidazolyl, or
   c) -pyridyl; and
R₂₃, R₂₄, and R₂₅ independently comprise hydrogen, aryl, heteroaryl, -alkylene-heteroaryl, alkyl, -alkylene-aryl, -alkylene-O-aryl, or -alkylene-O-heteroaryl; and R₂₃ and R₂₄ may be taken together to form a ring having the formula -(CH₂) *ₛ*-X₃-(CH₂)*ₜ*- bonded to the nitrogen atom to which R₂₃ and R₂₄ are attached
   wherein
   s and t are, independently, 1, 2, 3, or 4;
   X₃ comprises a direct bond, -CH₂-, -O-, -S-, -S(O₂)-, -C(O)-, -CON (H)-, -NHC(O)-, -NHCON(H)-, -NHSO₂-, -SO₂N(H)-, -C(O)-O-, -O-C(O)-, -NHSO₂NH-, 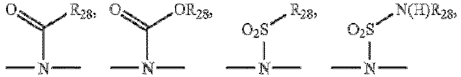 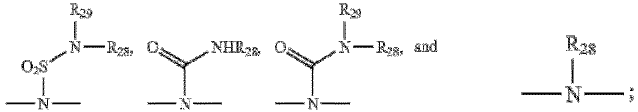 wherein R₂₈ and R₂₉ independently comprise hydrogen, aryl, heteroaryl, alkyl, -alkylene-aryl, or -alkylene-heteroaryl; wherein
   either
      at least one of the groups R₁, R₂, R₃ and R₄ are substituted with at least one group of the formula -Y₃-Y₄-NR₂₃R₂₄, -Y₃-Y₄-NH-C (=NR₂₅)NR₂₃R₂₄, -Y₃-Y₄-C(=NR₂₅) NR₂₃R₂₄, or -Y₃-Y₄-Y₅-A₂, with the proviso that no more than one of R₂₃, R₂₄, and R₂₅ may comprise aryl or heteroaryl; or
      R₂ is a group of the formula 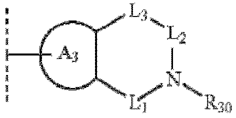 and
         wherein
one of R₃ and R₄, R₃ and R₂, or R₁ and R₂ may be taken together to constitute, together with the atoms to which they are bonded, an aryl, heteroaryl, fused arylcycloalkyl, fused arylheterocyclyl, fused heteroarylcycloalkyl, or fused heteroarylheterocyclyl ring system,
wherein
   said ring system or R₁, R₂, R₃, or R₄ is substituted with at least one group of the formula
   a) -Y₅-Y₆-NR₃₃R₃₄;
   b) -Y₅-Y₆-NH-C(=NR₃₅)NR₃₃R₃₄;
   c) -Y₅-Y₆-C(=NR₃₅)NR₃₃R₃₄; or
   d) -Y₅-Y₆-Y₇-A₄;
      wherein
      Y₅ and Y₇ independently comprise a direct bond, -CH₂-, -O-, -N(H), -S-, SO₂-, -CON(H)-, -NHC(O)-, -NHCON(H)-, -NHSO₂-, -SO₂N(H)-, C(O)-O-, -NHSO₂NH-, -O-CO-, 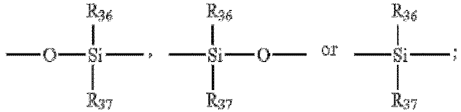 wherein R₃₆ and R₃₇ independently comprise aryl, alkyl, -alkylene-aryl, alkoxy, or -alkyl-O-aryl;
Y₆ comprises
   a) alkylene;
   b) alkenylene;
   c) alkynylene;
   d) arylene;
   e) heteroarylene;
   f) cycloalkylene;
   g) heterocyclylene;
   h) alkylene-arylene;
   i) alkylene-heteroarylene;
   j) alkylene-cycloalkylene;
   k) alkylene-heterocyclylene;
   l) arylene-alkylene;
   m) heteroarylene-alkylene;
   n) cycloalkylene-alkylene;
   o) heterocyclylene-alkylene;
   p) -O-;
   q) -S-;
   r) -S(O₂)-; or
   s) -S(O)-;
   wherein said alkylene groups may optionally contain one or more O, S, S(O), or SO₂ atoms;
A₄ comprises
   a) heterocyclyl, fused arylheterocyclyl, or fused heteroarylheterocyclyl, containing at least one basic nitrogen atom,
   b) -imidazolyl, or
   c) -pyridyl; and
R₃₃, R₃₄ and R₃₅ independently comprise hydrogen, aryl, heteroaryl, alkyl, -alkylene-aryl, or -alkylene-O-aryl; with the proviso that no two of R₃₃, R₃₄ and R₃₅ are aryl and/or heteroaryl; and R₃₃ and R₃₄ may be taken together to form a ring having the formula -(CH₂)*ₙ*-X₄-(CH₂)*ᵥ*- bonded to the nitrogen atom to which R₃₃ and R₃₄ are attached, wherein
   u and v are, independently, 1, 2, 3, or 4;
   X₄ comprises a direct bond, -CH₂-, -O-, -S-, -S(O₂)-, -C(O)-, -CON (H)-, -NHC(O)-, -NHCON(H)-, -NHSO₂-, -SO₂N(H)-, C(O)-O-, -O-C(O)-, -NHSO₂NH-, 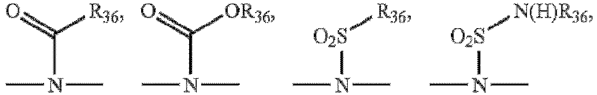 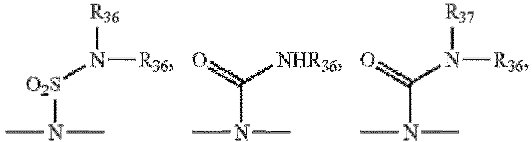 or 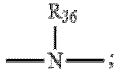
wherein R₃₆ and R₃₇ independently comprise hydrogen, aryl, heteroaryl, alkyl, -alkylene-aryl, or -alkylene-heteroaryl; and
wherein said ring system is optionally substituted with substituents comprising
   a) -H;
   b) -halogen;
   c) -hydroxyl;
   d) -cyano;
   c) -carbamoyl;
   f) -carboxyl;
   g) -Y₈-alkyl;
   h) -Y₈-aryl;
   i) -Y₈-heteroaryl;
   j) -Y₈-alkylene-aryl;
   k) -Y₈-alkylene-heteroaryl;
   l) -Y₈-alkylene-NR₃₈R₃₉; or
   m) -Y₈-alkylene-W₃-R₄₀;
      wherein
         Y₈ and W₃ independently comprise -CH₂-, -O-, -N(H), -S-, SO₂-, -CON(H)-, -NHC(O)-, -NHCON(H)-, -NHSO₂-, -SO₂N(H)-, -C(O)-O-, -NHSO₂NH-, -O-CO-, 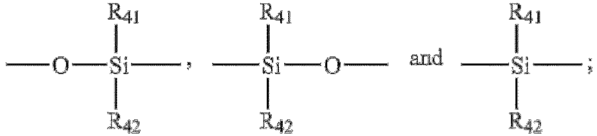
      wherein R₄₁ and R₄₂ independently comprise aryl, alkyl, -alkylene-aryl, alkoxy, or -alkyl-O-aryl; and 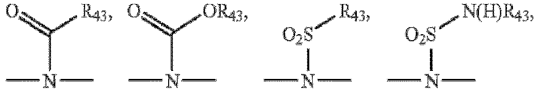 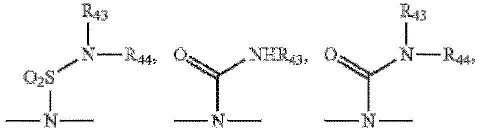 or 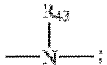
wherein R₄₃ and R₄₄ independently comprise hydrogen, aryl, heteroaryl, alkyl, -alkylene-aryl, or -alkylene-heteroaryl;
or a pharmaceutically acceptable salt thereof.

In another embodiment, the small molecule RAGE antagonists comprise benzimidazole compounds of Formula II. 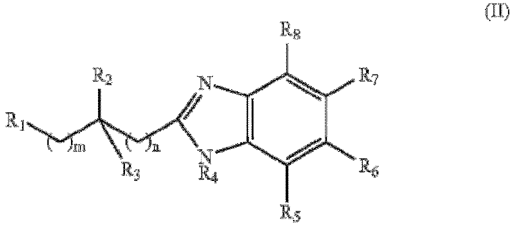 wherein for compounds of Formula (II)
m is an integer of from 0 to 3;
n is an integer of from 0 to 3;
R₁ comprises aryl;
R₂ comprises
   a) a group of the formula -N(R₉R₁₀), -NHC (O)R₉, or -NHC(O)OR₉;
   b) a group of the formula -OR₉;
   c) a group of the formula -SR₉, -SOR₉, -SO₂R₉, -SO₂NHR₉, or -SO₂N(R₉R₁₀);
   wherein R₉ and R₁₀ independently comprise
   1) -H;
   2) -Aryl;
   3) a group comprising
      a) -C₁₋₆ alkyl;
      b) -C₁₋₆ alkylaryl;
      c) 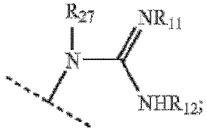
      d) -aryl;
      e) -C₁₋₆ alkyl; or
      f) -C₁₋₆ alkylaryl;
R₃ and R₄ independently comprise
   a) H;
   b) -aryl;
   c) C₁₋₆ alkyl;
   d) -C₁₋₆ alkylaryl; or
   e) -C₁₋₆ alkoxyaryl;
R₅, R₆, R₇, and R₈ independently comprise
   a) -H;
   b) -C₁₋₆ alkyl;
   c) -aryl;
   d) -C₁₋₆ alkylaryl;
   e) -C(O)-O-C₁₋₆ alkyl;
   f) -C(O)-O-C₁₋₆ alkylaryl;
   g) -C(O)-NH-C₁₋₆ alkyl;
   h) -C(O)-NH-C₁₋₆ alkylaryl;
   i) -SO₂-C₁₋₆ alkyl ;
   j) -SO₂-C₁₋₆ alkylaryl;
   k) -SO₂-aryl;
   l) -SO₂-NH-C₁₋₆ alkyl;
   m) -SO₂-NH-C₁₋₆ alkylaryl;
   n) -C(O)-C₁₋₆ alkyl;
   o) -C(O)-C₁₋₆ alkylaryl;
   p) -Y-C₁₋₆ alkyl;
   q) -Y-aryl;
   r) -Y-C₁₋₆ alkylaryl;
   s) -Y-C₁₋₆ alkylene-NR₁₃R₁₄; or
   t) -Y-C₁₋₆alkylene-W-R₁₅;
   wherein Y and W independently comprise -CH₂-, -O, -N(H)-, -S-, SO₂-, -CON (H)-, -NHC(O)-, -NHCON(H)-, -NHSO₂NH-, -SO₂N(H₎-, -C(O)-O-, 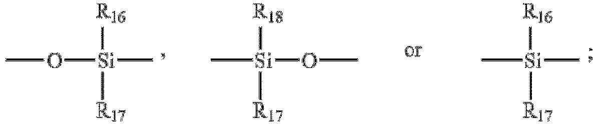
   R₁₆ and R₁₇ independently comprise aryl, C₁-C₆ alkyl, C₁-C₆ alkylaryl, C₁-C₆ alkoxy, or C₁-C₆ alkoxyaryl;
   R₁₅ independently comprise aryl, C₁-C₆ alkyl, or C₁-C₆ alkylaryl; or
   u) halogen, hydroxyl, cyano, carbamoyl, or carboxyl;
R₁₁, R₁₂, R₁₃, and R₁₄ independently comprise hydrogen, aryl, C₁-C₆, alkyl, C₁-C₆ alkylaryl, C₁-C₆ alkoxy, or C₁-C₆ alkoxyaryl;
R₁₃ and R₁₄ may be taken together to form a ring having the formula -(CH₂)*ₒ*-X-(CH₂)*ₚ*- bonded to the nitrogen atom to which R₁₃ and R₁₄ are attached, and/or R₁₁ and R₁₂ may, independently, be taken together to form a ring having the formula -(CH₂)*ₒ*-X-(CH₂)*ₚ*- bonded to the atoms to which R₁₁ and R₁₂ are connected, wherein o and p are, independently, 1, 2, 3, or 4; X comprises a direct bond, -CH₂-, -O-, -S-, -S(O₂)-, -C(O)-, -CON(H)-, -NHC(O)-, -NHCON(H)-, -NHSO₂-, -SO₂N (H) -, C(O)-O-, -O-C(O)-, -NHSO₂NH-, 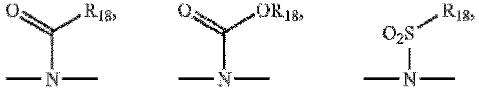 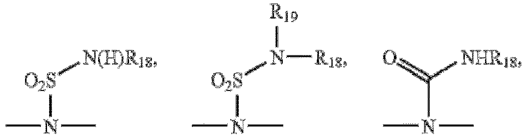 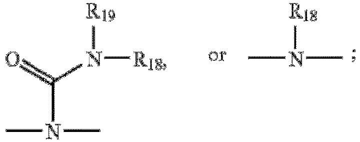
wherein the aryl and/or alkyl group(s) in R₁, R₂, R₃, R₅, R₆, R₇, R₈, R₉, R₁₀, R₁₁, R₁₂, R₁₃, R₁₄ R₁₅, R₁₆, R₁₇, R₁₈, and R₁₉ may be optionally substituted 1-4 times with a substituent group, wherein said substituent group(s) or the term substituted refers to groups comprising:
   a) -H;
   b) -Z-C₁₋₆ alkyl;
      -Z-aryl;
      -Z-C-₁₋₆ alkylaryl;
      -Z-C₁₋₆-alkyl-NR₂OR₂₁;
      -Z-C₁₋₆-alkyl-W-R₂₂;
      wherein Z and W independently comprise -CH₂-, -O-, -N(H), -S-, SO₂-, -CON (H)-, -NHC(O)-, -NHCON(H)-, -NHSO₂-, -SO₂N(H)-, -C(O)-O-, -NHSO₂NH-, -O-CO-, 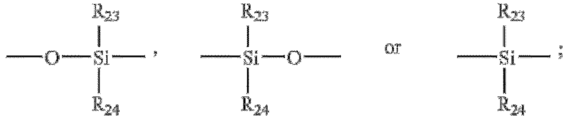 wherein;
      R₂₀ and R₂₁ independently comprise hydrogen, aryl, C₁-C₆ alkyl, C₁-C₆ alkylaryl, C₁-C₆ alkoxy, or C₁-C₆ alkoxyaryl;
      R₂₂, R₂₃, and R₂₄ independently comprise aryl, C₁-C₆ alkyl, C₁-C₆ alkylaryl, C₁-C₆ alkoxy, or C₁-C₆ alkoxyaryl; or 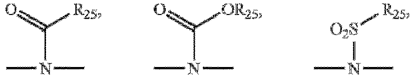 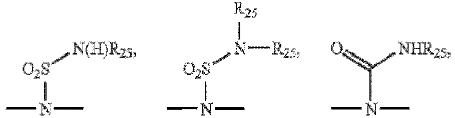 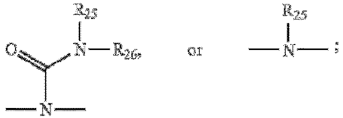
         R₂₅, R₂₆, and R₂₇ independently comprise hydrogen, aryl, C₁-C₆ alkyl, or C₁-C₆ alkylaryl;
         or a pharmaceutically acceptable salt, solvate or prodrug thereof.

In yet another embodiment, the small molecule RAGE antagonist comprises carboxamide compounds of Formula (III): 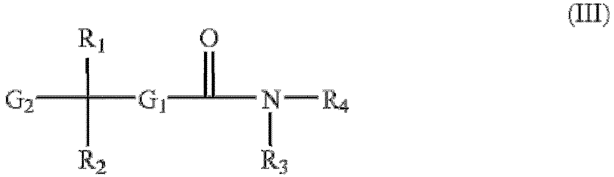 wherein for compound of Formula (III)
G₁ comprises C₁-C₆ alkylene or (CH₂)*ₖ*, where k is 0 to 3;
G₂ comprises
   a) hydrogen
   b) -C₁₋₆ C₁₋₆ alkyl;
   c) -aryl;
   d) -C₁₋₆ alkylaryl
   e) 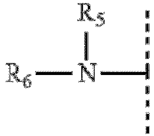 where R₅ and R₆ independently comprise
      i) -H;
      ii) -C₁₋₆ alkyl;
      iii) -aryl;
      iv) -C₁₋₆ alkylaryl;
      v) -C(O)-O-C₁₋₆ alkyl;
      vi) -C(O)-O-C₁₋₆ alkylaryl;
      vii) -C(O)-O-C₁₋₆ alkylcycloalkylaryl;
      viii) -C(O)-NH-C₁₋₆ alkyl;
      ix) -C(O)-NH-C₁₋₆ alkylaryl;
      x) -SO₂-C₁₋₆ alkyl;
      xi) -SO₂-C₁₋₆ alkylaryl;
      xii) -SO₂-aryl;
      xiii) -SO₂-NH-C₁₋₆ alkyl;
      xiv) -SO₂-NH-C₁₋₆ alkylaryl; 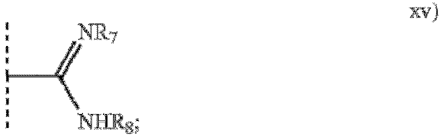
      xvi) -C(O)-C₁₋₆ alkyl; or
      xvii) -C(O)-C₁₋₆ alkylaryl; or
   f) a group of the formula 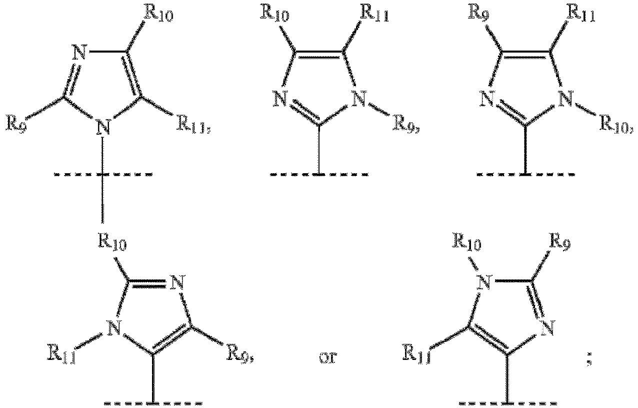 wherein
      R₉, R₁₀ and R₁₁ may comprise hydrogen; or
      R₉, R₁₀, and R₁₁ independently comprise
         i) -C₁₋₆ alkyl;
         ii) -aryl;
         iii) -C₁₋₆ alkylaryl;
         iv) -C(O)-O-C₁₋₆ alkyl;
         v) -C(O)-O-C₁₋₆ alkylaryl;
         vi) -C(O)-NH-C₁₋₆ alkyl;
         vii) C(O)-NH-C₁₋₆ alkylaryl;
         viii) -SO₂-C₁₋₆ alkyl;
         ix) -SO₂-C₁₋₆ alkylaryl;
         x)-SO₂-aryl;
         xi) -SO₂-NH-C₁₋₆ alkyl;
         xii) -SO₂-NH-C₁₋₆ alkylaryl;
         xiii) -(C(O)-C₁₋₆ alkyl; or
         xiv) -C(O)-C₁₋₆ alkylaryl;
      or R₁₀ and R₁₁ may be taken together to constitute a fused cycloalkyl, fused heterocyclyl, or fused aryl ring containing the atoms to which R₁₀ and R₁₁ are bonded;
R₁ comprises
   a) hydrogen;
   b) -C₁₋₆ alkyl;
   c) -aryl; or
   d) -C₁₋₆ alkylaryl;
R₂ comprises
   a) -C₁₋₆ alkyl;
   b) -aryl;
   c) -C₁₋₆ alkylaryl; or
   d) a group of the formula 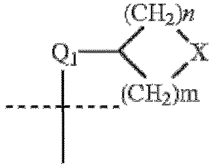 wherein m and n are independently selected from 1, 2, 3, or 4; X comprises a direct bond, CH₂₋, -O-, -S-, -S(O₂)-, -C(O)-, -CON(H)-, -NHC(O)-, -NH-CON(H)-, NHSO₂-, SO₂N(H)-, C(O)O-, -O-C(O)-, -NHSO₂NH-, 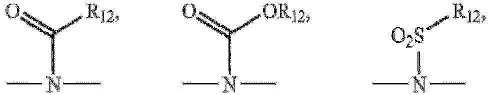 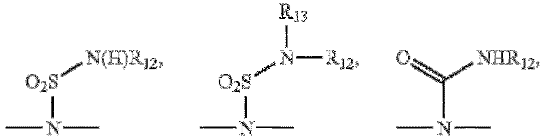 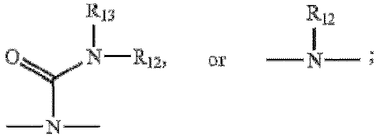
-Q1 - comprises C₁₋₆ alkylene, C₂₋₆ alkenylene, or C₂₋₆ alkynylene;
R₃ comprises
   a) hydrogen;
   b) - C₁₋₆ alkyl;
   c) - C₁₋₆ alkylaryl; or
   d) - C₁₋₆
R₄ comprises
   a) - C₁₋₆ alkylaryl;
   b) - C₁₋₆ alkoxyaryl; or
   c) -aryl;
R₇, R₈, R₁₂ and R₁₃ independently comprise hydrogen, C₁ - C₆ alkyl, C₁ - C₆ alkylaryl, or aryl; and wherein
   the aryl and/or alkyl group(s) in R₁, R₂, R₃, R₄, R₅, R₆, R₇, R₈, and R₉, R₁₀, R₁₁, and R₁₂, and R₁₃, may be optionally substituted 1-4 times with a substituent group, wherein said substituent group(s) or the term substituted refers to groups comprising:
   a) -H;
   b) -Y- C₁₋₆ alkyl;
      -Y-aryl;
      -Y- C₁₋₆ alkylaryl;
      -Y- C₁₋₆ alkyl-NR₁₄R₁₅;
      -Y- C₁₋₆ alkyl-W-R₁₆;
      wherein Y and W independently comprise -CH₂-, -O-, -N(H)-, -S-, - SO₂-, -CON(H)-, -NHC(O)-, -NHCON(H)-, -NHSO₂-, -SO₂N(H)-, - C(O)-O-, -NHSO₂NH-, -O-CO-, 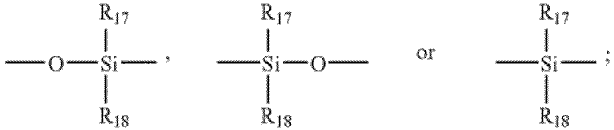 R₁₆, and R₁₇, and R₁₈ comprise hydrogen, aryl, C₁ - C₆ alkyl, C₁ - C₆ alkylaryl, C₁ - C₆ alkoxy, or C₁ - C₆ alkoxyaryle; or
   c) halogen, hydroxyl, cyano, carbamoyl, or carboxyl; and
R₁₄ and R₁₅ independently comprise hydrogen, aryl, C₁ - C₆ alkyl, or C₁ - C₆ alkylaryl; and wherein
   R₁₄, and R₁₅ may be taken together to form a ring having the formula -(CH₂)*ₒ*-Z-(CH₂)*ₚ* - bonded to the nitrogen atom to which R₁₄ and R₁₅ are attached, and/or R₇ and R₈ may, independently, be taken together to form a ring having the formula -(CH₂)*ₒ*-Z-(CH₂)*ₚ* - bonded to the atoms to which R₇ and R₈ are attached, wherein o and p are, independently, 1, 2, 3, or 4; Z comprises a direct bond, -CH₂-, -O-,-S-, -S(O₂)-, -C(O)-, -CON(H)-, -NHC(O)-, - NHCON(H)-, -NHSO₂-, -SO₂N(H)-, -C(O)-O-, -O-CO-, - NHSO₂NH-, 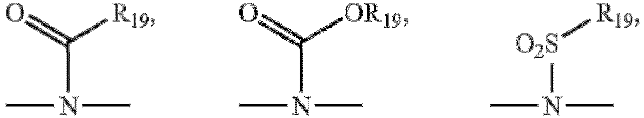 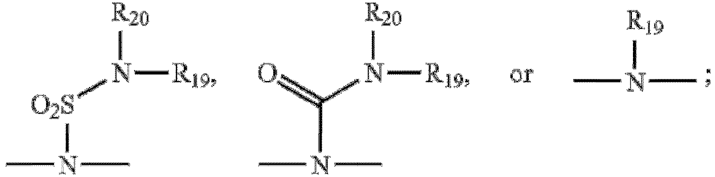
R₁₉ and R₂₀ independently comprise hydrogen, aryl, C₁ - C₆ alkyl, or C₁ - C₆ alkylaryl.

The present disclosure also comprises compounds of formula (IV) as a small molecule RAGE antagonists capable of reversing Alzheimer's Disease: 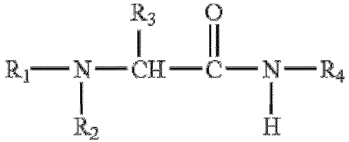 wherein for compounds of Formula (IV):
R₁ and R₂ are independently selected from
   a) -H;
   b) -C₁₋₆ alkyl;
   c) -aryl;
   d) -C₁₋₆ alkylaryl; or
   e) -C(O)-O-C₁₋₆ alkyl;
   f) -C(O)-O-C₁₋₆ alkylaryl;
   g) -C(O)-NH-C₁₋₆ alkyl;
   h) -C(O)-NH-C₁₋₆ alkylaryl;
   i) -SO₂-C₁₋₆ alkyl;
   j) -SO₂-C₁₋₆ alkylaryl;
   k) -SO₂-aryl;
   l) -SO₂-NH-C₁₋₆ alkyl;
   m) -SO₂-NH-C₁₋₆ alkylaryl;
   n) 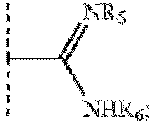
   o) -C(O)-C₁₋₆ alkyl; and
   p) -SO₂-C₁₋₆ alkylaryl;
R₃ is selected from
   a) - C₁₋₆ alkyl;
   b) -aryl; and
   c) - C₁₋₆ alkylaryl;
R₄ is selected from
   a) - C₁₋₆ alkylaryl;
   b) - C₁₋₆ alkoxylaryl; and
   c) -aryl;
R₅ and R₆ are independently selected from the group consisting of hydrogen, C₁-C₆ alkyl, C₁-C₆ alkylaryl, and aryl; and wherein
   the aryl and/or alkyl group(s) in R₁, R₂, R₃, R₄, R₅, R₆, R₇, R₈, R₉, R₁₀, R₁₈, R₁₉, and R₂₀ may be optionally substituted 1-4 times with a substituent group, wherein said substituent group(s) or the term substituted refers to groups selected from the group consisting of:
   a) -H;
   b) - Y-C₁₋₆ alkyl;
      -Y-aryl;
      -Y-C₁₋₆ alkylaryl;
      -Y-C₁₋₆ alkyl-NR₇R₈; and
      -Y-C₁₋₆ alkyl-W-R₂₀;
      wherein Y and W are, independently selected from the group consisting of -CH₂-, -O-, -N(H)-, -S-, -SO₂-, -CON(H)-, - NHC(O)-, -NHCON(H)-, - NHSO₂-, -SO₂N(H)-, -C(O)-O-, - NHSO₂NH-, -O-CO-, 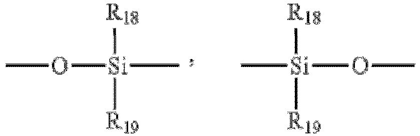 and 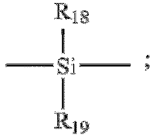 and
   c) halogen, hydroxyl, cyano, carbamoyl, or carboxyl; and
R₁₈ and R₁₉ are independently selected from the group consisting of aryl, C₁-C₆ alkyl, C₁-C₆ alkylaryl, C₁-C₆ alkoxy, and C₁-C₆ alkoxylaryl;
R₂₀ is independently selected from the group consisting of aryl, C₁-C₆ alkyl, and C₁-C₆ alkylaryl; R₇, R₈ R₉, and R₁₀ are independently selected from the group consisting of hydrogen, aryl, C₁-C₆ alkyl, and C₁-C₆ alkylaryl; and wherein
R₇ and R₈ may be taken together to form a ring having the formula -(CH₂)*ₘ*-X-(CH₂)*ₙ*-bonded to the nitrogen atom to which R₇ and R₈ are attached, and/or R₅ and R₆ may, independently, be taken together to form a ring having the formula -(CH₂)*ₘ*-X-(CH₂)*ₙ*-bonded to the nitrogen atoms to which R₅ and R₆ are attached, wherein m and n are, independently, 1, 2, 3, or 4; X is selected from the group consisting of -CH₂-, -O-, -S-, -S(O₂)-, -C(O)-, - CON(H)-, -NHC(O)-, -NHCON(H)-, -NHSO₂-, - SO₂N(H)-, -C(O)-O-, - O-CO-, -NHSO₂NH-, 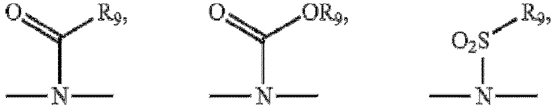 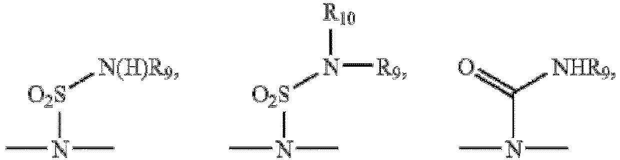 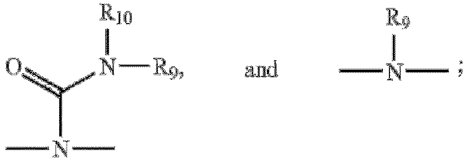 or a pharmaceutically acceptable salt, solvate or prodrug thereof.

Embodiments of the compounds above or their pharmaceutically acceptable salts include:

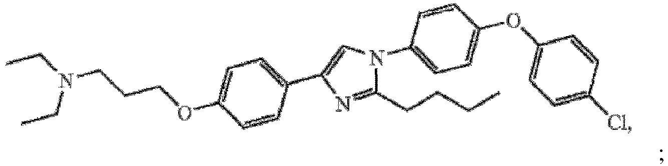

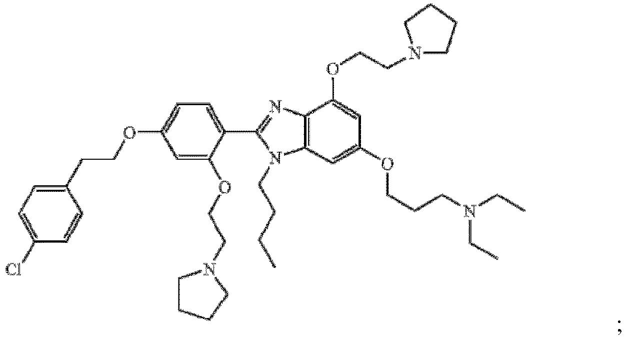

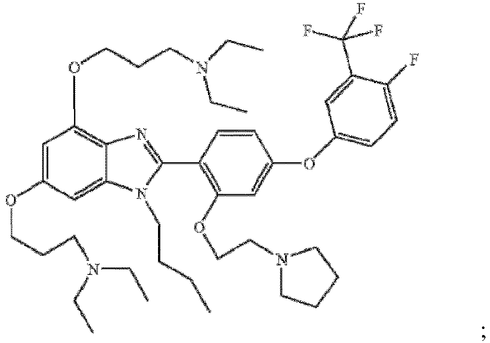

Other disclosures which provide aspects of the disclosure related to small molecules include US 7,737,285; US 7,776,919; US 7,714,013; US 7,329,684; US 7,067,554; WO 2007/089616.

Additional compounds related to RAGE antagonists which may be used in the methods of the present disclosure include phenylheteroaryl compounds such as those provided for in US 9045461 B2 to Gohimukkla et al.*,.* These phenylheteroaryl compounds include compounds of the formula: 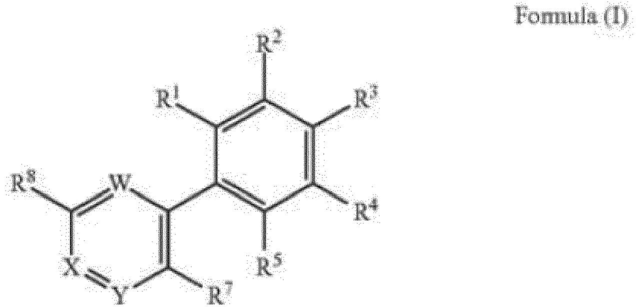 wherein
W is CR⁶, and X and Y are N, and R⁶ is - H, R¹, R², R⁴, and R⁵ are - H, R³ is the group - X¹-L¹-R¹³ wherein
X¹ is selected from the group consisting of a direct bond and -O- ,
L¹ is selected from the group consisting of a direct bond, -CH₂- , and - CH₂CH₂- , and
R¹³ is selected from the group consisting of -phenyl and -cyclohexyl, wherein the cyclohexyl and phenyl groups of R¹³ are optionally substituted one or more times with R¹⁴, wherein each R¹⁴ is independently selected from the group consisting or -halogen, -(C₁-C₆)alkyl, and -(C₁-C₆)haloalkyl,
R₇ is the group -L²-X² - R¹⁵ wherein
   L² is -(C₁-C₄)alkylene-,
   X² is selected from the group consisting of a direct bond and -O-, and
   R¹⁵ is selected from the group consisting of - (C₁-C₄)alklyl, optionally substituted one or more times with R¹⁶, wherein each R¹⁶ is independently selected from the group consisting of -halogen,
R⁸ is the group - X³ -L³ -R¹⁷, wherein
   X³ is selected from the group consisting of direct bond, -O-, and - C(O)NH-,
   L³ is selected from the group consisting of a direct bond and -CH₂- ,
   R¹⁷ is 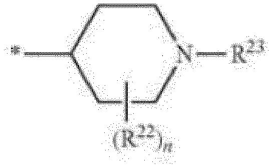 wherein
      each R²² may be attached to any of the ring carbon atoms of R¹⁷, and
      wherein
         each R²² is independently selected from the group
         consisting of -halogen, X⁴- R²⁴, -(C₁-C₆)alkylene-R²⁴, -(C₁-C₆)alkylene-X⁵- R²⁴. and
         - X⁴-(C₁-C₆)alkylene-NR²⁵R²⁶, wherein
      X⁴ and X⁵ are independently selected from the group consisting of: direct bond, -O-, and -N(R²⁷)- , wherein R²⁷ is selected from the group consisting of -H and -(C₁-C₆)alkyl,
      R²⁴ is selected from the group consisting of - H, and -(C₁-C₆)alkyl, wherein the alkyl groups of R²⁴ is optionally substituted one or more times with R²⁸, wherein each R²⁸ is independently selected from the group consisting of halogen,
      R²⁵ and R²⁶ are independently selected from the group consisting of -H, and -(C₁-C₆)alkyl,
      R²³ is selected from the group consisting of -H, and -(C₁-C₆)alkyl, and
      n is 0, 1, 2, or 3.

Other compounds related to RAGE antagonists which may be used in the methods of the present disclosure include phenylheteroaryl compounds such as those provided for in US 9,045,461 B2 to Gohimukkla et al.*,.* These phenylheteroaryl compounds include compounds of the formula:

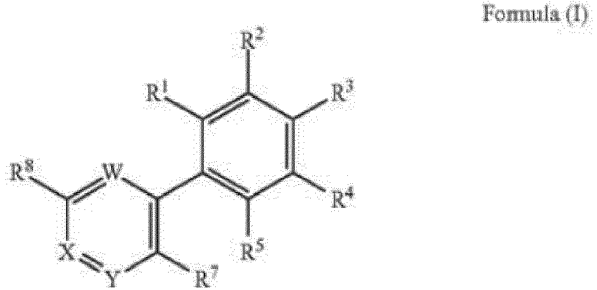

wherein
W, X, and Y are independently selected from the group consisting of CR⁶, N, and N(O), wherein at least one of W, X, and Y is N or N(O), and R⁶ is selected fro the group consisting of - H, -halogen, -(C₁-C₆)alkyl, and -(C₁-C₆)haloalkyl;
R¹, R², R⁴, and R⁵ are independently selected from the group consisting of -H, - halogen, -cyano, -NO₂, -OR⁹, -SR⁹, -S(O)₃R⁹, -S(O)₂OR⁹, -S(O)₃NR⁹R¹⁰, - NR⁹S(O)₂R¹⁰, -NHC(O)NHR⁹, -NR⁹C(O)R¹⁰, -(CR¹¹R¹²)ₜNR⁹R¹⁰, -C(O)R⁹, - C(O)OR⁹, -C(O)O(CR¹¹R¹²)ₜCONR⁹R¹⁰, -C(O)NR⁹R¹⁰, -(C₁-C₁₀)alkyl, -(C₂-C₁₀)alkenyl, -(C₂-C₁₀)alkynyl, -phenyl, -(C₃-C₁₀)cycloalkyl, -pyrazolyl, -isoxazolyl, - tetrazolyl, -oxazolyl, - dihydro-oxazolyl, wherein the alkyl, alkenyl, phenyl, cycloalkyl, pyrazolyl, isoxazolyl, tetrazolyl, oxazolyl, and dihydro-oxazolyl groups are optionally substituted one or more times with substituents independently from R³⁹, wherein
R⁹ and R¹⁰ are independently selected from the group consisting of -H, - (C₁-C₆)alkyl, -(C₃-C₁₀)cycloalkyl, -phenyl, -(C₁-C₆)alkylene-(C₃-C₁₀)cycloalkyl, and - (C₁-C₆)alkylene-phenyl, wherein the alkyl, alkylene, cycloalkyl, phenyl groups of R⁹ and R¹⁰ are optionally substituted one or more times with substituents independently selected from R³⁹, or R⁹ and R¹⁰ together with the atoms to which they are attached form a heterocyclic ring of 5 to 7 members containing 0 to 2 additional heteroatoms independently selected from oxygen, sulfur and nitrogen and the ring is optionally substituted one or more times with substituents independently selected from R³⁹, and
   R¹¹ and R¹² are independently selected from the group consisting of -H, -(C₁-C₆)alkyl, -(C₃-C₁₀)cycloalkyl, -phenyl, -(C₁-C₆)alkylene-(C₃-C₁₀)cycloalkyl, and -(C₁-C₆)alkylene-phenyl, wherein the alkyl, alkylene, cycloalkyl, phenyl groups of R¹¹ and R¹² are optionally substituted one or more times with substituents independently selected from R³⁹, or R¹¹ and R¹² together with the atoms to which they are attached form a heterocyclic ring of 5 to 7 members containing 0 to 2 heteroatoms independently selected from oxygen, sulfur and nitrogen and the ring is optionally substituted one or more times with substituents independently selected from R³⁹:
   R³ is selected from the group consisting of -X¹-L¹-R¹³ and -L¹-X¹-R¹³ wherein X¹ is selected from the group consisting of a direct bond, -O-, -C(O)-, -C(O)O-, -OC(O)-, -S(O)₂-, -S(O)₂NH-, -NHS(O)₂-, -C(O)NH-, and -NHC(O)-,
      L¹ is selected from the group consisting of a direct bond and -(C₁-C₆)alkylene-, and
      R¹³ is selected from the group consisting of a direct bond and -(C₁-C₆)alkyl, - (C₃-C₁₀)cycloalkyl, -(C₅-C₁₀)cycloalkyl, -phenyl, -pyridyl, -pyridazinyl, - piperidinyl, and -tetrahydropyranyl, wherein the alkyl, cycloalkyl, cycloalkenyl, phenyl, pyridyl, pyridazinyl, piperidinyl, tetrahydropyranyl groups of R¹³ is optionally substituted one or more times with R¹⁴ , wherein each R¹⁴ is independently selected from the group R³⁹ ;
   R⁷ is the group -L²-X²-R¹⁵ wherein
      L² is selected from the group consisting of a direct bond and -(C₁-C₆)alkylene-,
      X² is selected from the group consisting of a direct bond and -O-, and
      R¹⁵ is selected from the group consisting of -(C₁-C₁₀)alkyl, -(C₃-C₁₀)cycloalkyl, - (C₁-C₆)alkylene-(C₃-C₁₀)cycloalkyl, -phenyl, and -(C₁-C₆)alkylene-phenyl, wherein the alkyl, alkylene, cycloalkyl, and pheny groups of R¹⁵ is optionally substituted one or more times with R¹⁶, wherein each R¹⁶ is independently selected from the group R³⁹;
   R⁸ is selected from the group consisting of - X³-L³R¹⁷ and -L³-X³-R¹⁷ and wherein
      X³ is selected from the group consisting of a direct bond, -O-, -C(O)-, -C(O)O-, -C(O)NH-, -C(O)N(R¹⁸ )-, -NHC(O)-, and -N(R¹⁸)C(O)- wherein R¹⁸ is selected from the group consisting of -(C₁-C₆)alkyl, -(C₃-C₆)cycloalkyl, - (C₁-C₆)alkylene-O-(C₁-C₆)alkyl, -(C₁-C₆)alkylene-(C₃-C₆)cycloalkyl,
      L³ is selected from the group consisting of a direct bond and -(C₁-C₆)alkylene- wherein the aklylene group is optionally substituted one or more times with R¹⁹, wherein each R¹⁹ is independently selected from the group consisting of -OH, -NH₂, - O-(C₁-C₆)alkyl, -(C₁-C₆)alkylene-NH₂, -(C₁-C₆)alkylene-NH((C₁-C₆)alkyl), and -(C₁-C₆)alkylene-N((C₁-C₆)alkyl)₂, wherein the alkyl and alkylene groups of R¹⁹ are optionall substituted one or more times with -halogen,
   R¹⁷ is selected from the group consisting of 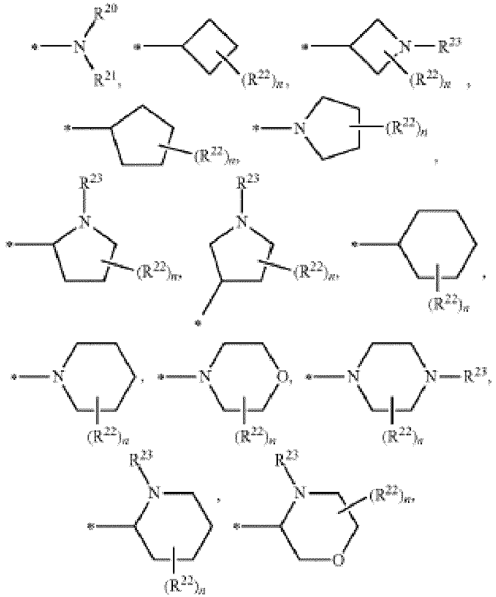 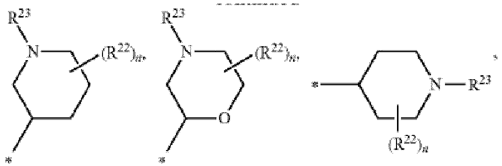 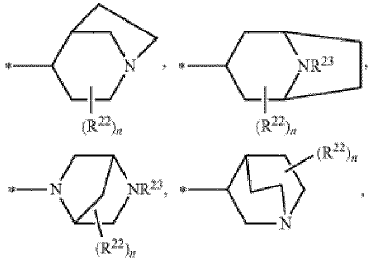 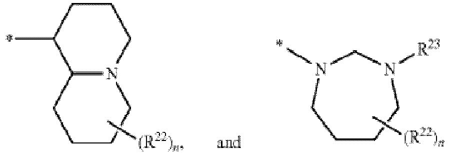 wherein
      each R²² may be attached to any of the ring carbon atoms of R¹⁷, and wherein
      R²⁰ and R²¹ are independently selected from the group consisting of -H, -(C₁-C₆)alkyl, and -(C₁-C₆)haloalkyl,
      each R²² is independently selected from the group consisting of -cyano, halogen, - X⁴-R²⁴, -(C₁-C₆)alkylene-R²⁴, -X⁴-(C₁-C₆)alkylene-R²⁴(C₁-C₆)alkylene-X⁵R²⁴ - X⁴-(C₁-C₆) alkylene-X⁵-R²⁴ and -X⁴-(C₁-C₆)alkylene-NR²⁵R²⁶, wherein
      X⁴ and X⁵ are independently selected from the group consisting of: direct bond, - O-, -N(R²⁷)-, -C(O)-, -C(O)O-, -OC(O)-, -S(O)₂-, -C(O)N(R²⁷)-, - N(R²⁷)C(O)-, -S(O)₂N(R²⁷)-, -N(R²⁷)S(O)₂-, and -C(O)N(R²⁷)-S(O)₂-,
      wherein R²⁷ is selected from the group consisting of -H and -(C₁-C₆)alkyl,
      R₂₄ is selected from the group consisting of -H, and -(C₁-C₆)alkyl, -phenyl, and - (C₃-C₁₀)cycloalkyl, wherein the alkyl, phenyl, and cycloalkyl groups of R₂₄ are optionally substituted one or more times with R²⁸ , wherein each R²⁸ is independently selected from R³⁹,
      R²⁵ and R²⁶ are independently selected from the group consisting of -H, -(C₁-C₆)alkyl, and -(C₁-C₆)haloalkyl,
      wherein the alkylene groups of R²² are optionally substituted one or more times with R²⁹, wherein each R²⁹ is independently selected from the group consisting of halogen, -OH, - NH₂, -O-(C₁-C₆)alkyl, -(C₁-C₆)alkylene-NH₂, -(C₁-C₆)alkylene-NH((C₁-C₆)alkyl), and - (C₁-C₆)alkylene-N((C₁-C₆)alkyl)₂, wherein the alkyl and alkylene groups of R²⁹ are optionally substituted one or more times with halogen;
      R²³ is selected from the group consisting of -H, -R³⁰, -(C₁-C₆)alkylene-R³¹, - (C₁-C₆)alkylene-NR³²R³³, -X⁶-(C₂-C₆)alkylene-NR³²R³³, and -(C₁-C₆)alkylene-X⁷-R³¹, wherein
      X⁶ is selected from the group consisting of -C(O)-, -C(O)O-, -S(O)₂-,-C(O)N(R³⁴)-, and -S(O)₂N(R³⁴)-, wherein R³⁴ is selected from the group consisting of - H, -(C₁-C₆)alkyl, and -(C₁-C₆)haloalkyl,
      X⁷ is selected from the group consisting of -O-, -N(R³⁵)-, -C(O)-, - C(O)O-, -O-C(O)-, - C(O)N(R³⁵)-, -N(R³⁵)C(O)-, -S(O)₂N(R³⁵)-, and - N(R³⁵)S(O)₂-,
      wherein R³⁵ is selected from the group consisting of -H, -(C₁-C₆)alkyl, and -(C₁-C₆)haloalkyl,
      R³⁰ is selected from the group consisting of -H, and -(C₁-C₆)alkyl, -phenyl, and - (C₃-C₁₀)cycloalkyl, wherein the alkyl, phenyl, and cycloalkyl groups of R³⁰ are optionally substituted one or more times with R³⁶, wherein each R³⁶ is independently selected from R³⁹,
      R³¹ is selected from the group consisting of -H, and -(C₁-C₆)alkyl, -phenyl, -(C₃-C₁₀)cycloalkyl, -tetrazolyl, -1,3-dioxanyl, 1,3,4-oxadiazolyl, and piperidinyl, wherein the alkyl, phenyl, cycloalkyl, tetrazolyl, 1,3-dioxanyl, 1,3,4-oxadiazolyl and piperidinyl groups of R³¹ are optionally substituted one or more times with R³⁷, wherein each R³⁷ is independently selected from R³⁹,
      R³² and R³³ are independently selected from the group consisting of -H,-(C₁-C₆)alkyl, and -(C₁-C₆)haloalkyl,
      wherein the alklylene groups of R²³ are optionally substituted one or more times with R³⁸, wherein each R³⁸ is independently selected from the group consisting of halogen, -OH, - NH₂, -O-(C₁-C₆)alkyl, -(C₁-C₆)alkylene-NH₂, -(C₁-C₆)alkylene-NH((C₁-C₆)alkyl), and - (C₁-C₆)alkylene-N((C₁-C₆)alkyl)₂, wherein the alkyl and alkylene groups of R³⁸ are optionally substituted one or more times with halogen;
      each R³⁹ is independently selected from the group consisting of -halogen, -(C₁-C₆)haloalkyl, -C(O)OH-, -(C₁-C₆)alkyl, -O-(C₁-C₆)alkyl, -O-(C₁-C₆)haloalkyl, - C(O)-(C₁-C₆)alkyl, -(C₃-C₁₀)cycloalkyl, -O-(C₃-C₁₀)cycloalkyl, -OH-, -NH₂-, -(C₁-C₆)alkyleneOH, -(C₁-C₆)alkylene-NH₂, -NH((C₁-C₆)alkyl), -N((C₁-C₆)alkyl)₂, -(C₁-C₆)alkylene-NH((C₁-C₆)alkyl), -(C₁-C₆)alkylene-N((C₁-C₆)alkyl)₂, -phenyl; -O-phenyl; - (C₁-C₆)alkylene-phenyl, -O-(C₁-C₆)alkylene-phenyl, -C(O)NH₂-, -C(O)NH-(C₁-C₆)alkyl, -C(O)N((C₁-C₆)alkyl)₂, -S(O)₂-(C₁-C₆)alkyl, -C(O)O-(C₁-C₆)alkyl, and - C(O)O-(C₁-C₆)alkylene-phenyl,
      n is an integer from 0 to 5,
      s is an integer from 1 to 2, and
      t is an integer from 1 to 10.

In another other aspect of the present disclosure, compounds related to RAGE antagonists which may be used in the methods of the present disclosure include substituted imidazole derivative compounds such as those provided for in US 8,580,833 B2 to Jones et al.

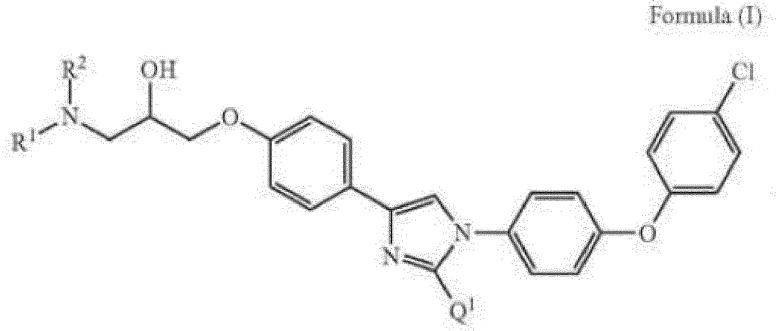

or a pharmaceutically acceptable salt thereof, wherein
R¹ and R² are independently selected from the group consisting of
-CH₃, -CH₂CH₃, -CH(CH₃)₃, and -CH₂CH₂CH₃; and
Q¹ is selected from the group consisting of -CH₂CH₂CH₃ and -CH₂CH₂CH₂CH₃.

In yet another aspect of the disclosure, additional compounds related to RAGE antagonists which may used in the methods of the present invention include, but are not limited to the following compounds and their derivatives:

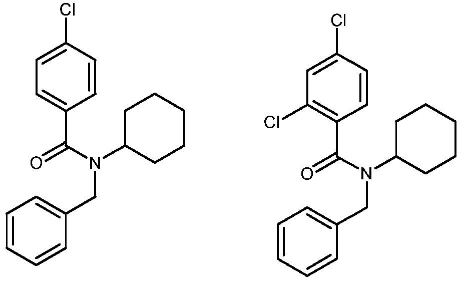

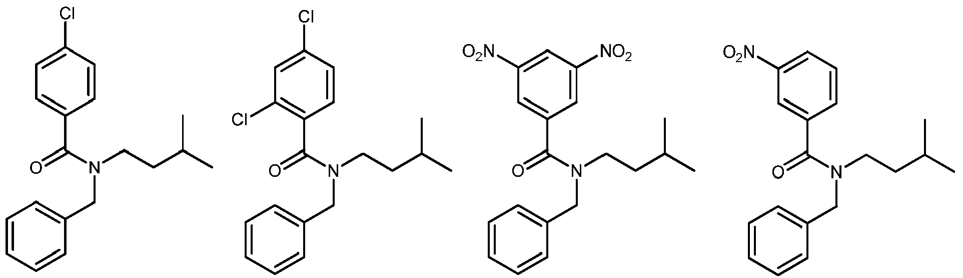

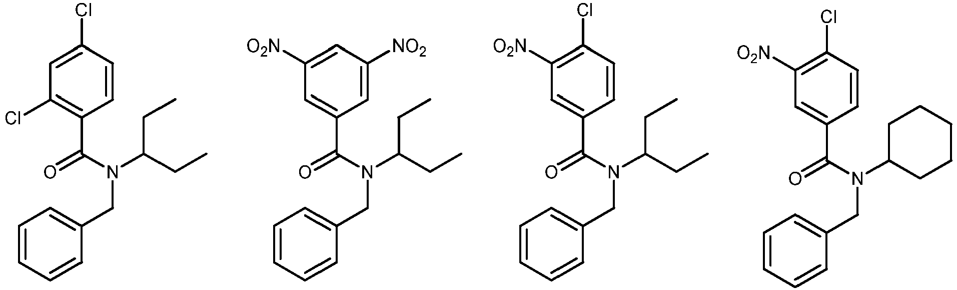

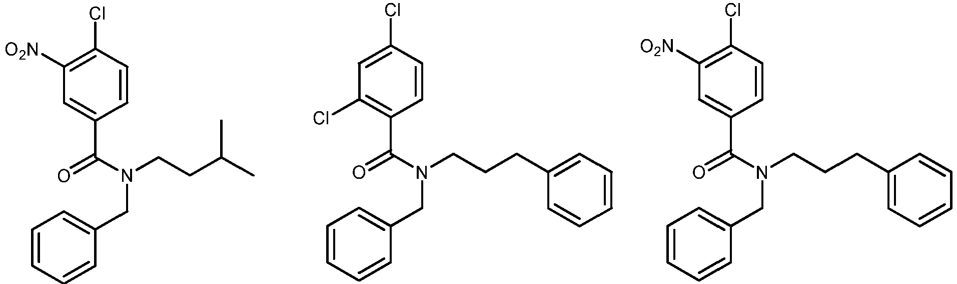

### Methods of Treating and/or Preventing Pre-cachexia and/or Cachexia

In one aspect, the present invention provides a method of treating and/or preventing cachexia or pre-cachexia in a patient, comprising administering to a patient in need thereof an effective dose of a compound that inhibits the RAGE signaling pathway, or a pharmaceutically acceptable salt or prodrug thereof. As described herein, activation of the Receptor for Advanced Glycation End-products was associated with the cachectic phenotype induced by multiple cancer types.

In certain embodiments, the compound inhibits the Receptor for Advanced Glycation Endproducts (RAGE). RAGE is a member of the immunoglobulin supergene family of molecules and was identified herein to be transcriptionally upregulated in muscle cells exposed to a cachexia-inducing factor(s). The extracellular (N-terminal) domain of RAGE includes three immunoglobulin-type regions: one V (variable) type domain followed by two C-type (constant) domains (Neeper et al., J Biol Chem 267:14998-15004 (1992); and Schmidt et al., Circ (Suppl) 96#194 (1997)). A single transmembrane-spanning domain and a short, highly charged cytosolic tail follow the extracellular domain. The N-terminal, extracellular domain can be isolated by proteolysis of RAGE or by molecular biological approaches to generate soluble RAGE (sRAGE) comprising the V and C domains. Soluble RAGE (a negative regulator of RAGE signaling that acts as a sink for RAGE ligands) was also identified herein to be elevated in non-cachexia-inducing conditioned media in comparison to cachexia-inducing conditioned media. Additionally, treatment with a blocking peptide or antibody to RAGE has been shown to inhibit and reverse the cachectic induced loss of myosin heavy chain protein in human muscle cells *in vitro.* RAGE blocking peptides are known in the art (see, for example, Arumugam et al., Clin Cancer Res. 2012; 18(16): 10.1158/1078-0432.CCR-12-0221), and are commercially available (e.g., RAP; a 10 amino acid sequence from S100P (R&D Systems)). Importantly, in contrast to the prior art, in the present invention antibody(ies) or blocking peptide(s) specific for RAGE compete(s) for binding to RAGE with RAGE ligand(s) S100A7, S100A8, S100A9 and/or Calprotectin, and preferably may not compete for binding to RAGE with RAGE ligand HMGB1.

RAGE binds to multiple functionally and structurally diverse ligands, such as proteins having β-sheet fibrils characteristic of amyloid deposits and pro-inflammatory mediators, and includes amyloid beta (Aβ), serum amyloid A (SAA), Advanced Glycation End products (AGEs), S100 (a proinflammatory member of the Calgranulin 65 family), carboxymethyl lysine (CML), Mac-1, β2-integrin, CD11b/CD18 and high-mobility group protein 1 (HMG1), which is also known as high-mobility group protein box-1 (HMGB1) or amphoterin (Bucciarelli et al., Cell Mol Life Sci 59:1117-1128 (2002); Chavakis et al., Microbes Infect 6:1219-1225 (2004); Kokkola et al., Scand J Immunol 61:1-9 (2005); Schmidt et al., J Clin Invest 108:949-955 (2001); Rocken et al., Am J Pathol 162:1213-1220 (2003); Donato et al., Curr Mol Med 13(1): 24-57 (2013)).

The invention also provides methods for inhibiting RAGE by alternative means, such as with the use antibodies. The use of of small organic molecules, soluble receptor fragments, fusion proteins, antibodies or peptides is also described. Mention is made of WO2011/042548, WO2007/109747, WO2009/136382, WO2011053707, WO2007/109749, WO2008/137552, WO2004/016229, US2010/0226915A1 and U.S. Patent Nos. 7,981,424, 7,485,697 and 8,420,083, which are cited for the compounds and methods for inhibiting RAGE disclosed therein. Importantly, in contrast to the prior art, in the present invention antibody(ies) specific for RAGE compete(s) for binding to RAGE with RAGE ligand(s) S100A7, S100A8, S100A9 and/or Calprotectin, and preferably may not compete for binding to RAGE with RAGE ligand HMGB1. Also in contrast to the prior art, in the present disclosure small organic molecule(s), soluble receptor fragment(s), fusion protein(s), or peptide(s) specific for RAGE compete(s) for binding to RAGE with RAGE ligand(s) S100A7, S100A8, S100A9 and/or Calprotectin, and preferably may not compete for binding to RAGE with RAGE ligand HMGB1

The invention provides therapeutic, engineered protein/peptide compositions comprising *e.g.*, anti-RAGE antibodies, to target a RAGE receptor (including soluble forms thereof and endogenous secretory RAGE), directly and/or *via* differential competition with one or more pre-cachexia and/or cachexia-associated RAGE ligands or markers. In one aspect, the invention provides an anti-RAGE antibody or antibodies specific for a RAGE receptor, which antibody competes for binding to the RAGE receptor with a RAGE ligand of the S100 family, *e.g.,* S100A7, S100A8, S100A9 and/or Calprotectin, and preferably may not compete for binding to the RAGE receptor with the RAGE ligand HMGB 1. In another aspect, the invention provides an anti-RAGE antibody mixture/cocktail. comprising one or more antibodies which compete for ligand binding to the RAGE receptor, wherein each antibody in said mixture/cocktail competes for binding of one or more specific S100 family member/ligand (*e.g.*, S100A7, S100A8, S100A9 and/or Calprotectin) to the RAGE receptor, and preferably may not compete for binding to the RAGE receptor with the RAGE ligand HMGB1. In another aspect, the invention provides an antibody specific for a RAGE receptor, which antibody competes for binding to the RAGE receptor with a RAGE ligand of the S100 family, wherein said competition is *via* a moiety/epitope (linear and/or conformational) which is bound by one or more specific S100 family members/ligands that induce pre-cachexia and/or cachexia. In one embodiment, the antibody binds to an epitope on the RAGE receptor selected from an epitope, which is bound by S100A7, S100A8, S100A9 and/or Calprotectin, and preferably may not bind to an epitope which is bound by the RAGE ligand HMGB1.

Suitable antibodies (or small organic molecules, soluble receptor fragments, fusion proteins) may also comprise a label attached thereto, such as a detectable label (e.g., a radioisotope, fluorescent compound, enzyme or enzyme co-factor). Suitable antibodies include whole antibodies and fragments thereof including chimeric antibodies, humanized antibodies, single chain antibodies, tetrameric antibodies, tetravalent antibodies, heteroconjugate antibodies, bispecific antibodies, multispecific antibodies, domain-specific antibodies, domain-deleted antibodies, diabodies, antibody conjugates (e.g., with an Fc domain (e.g., an antigen binding domain fused to an immunoglobulin constant region), PEG, an immunoglobulin domain, etc.), Fab fragments, Fab' fragments, F(ab')₂ fragments, Fv fragments, ScFv fragments, Fd fragments, single domain antibodies, and dAb fragments, and Fc fusion protein.

In certain embodiments, the compound inhibits MEK/ERK signaling either directly or downstream of MEK/ERK.

In certain embodiments, the invention provides methods for the treatment and/or prevention of cachexia or pre-cachexia by administering a low dose(s) of MEK/ERK inhibitors, e.g., as compared to an effective dose of that inhibitor for the treatment of cancer. In certain embodiments, an effective dose of the compound for treating or preventing cachexia or pre-cachexia is at most half the effective therapeutic dose of that compound for the treatment of cancer. In certain embodiments, an effective dose of the compound is half the effective therapeutic dose for the treatment of cancer. In certain embodiments, an effective dose of the compound is less than half the effective therapeutic dose for the treatment of cancer. Accordingly, a dose of a MEK/ERK inhibitor that effectively treats and/or prevents cachexia or pre-cachexia may not be an effective dose for treating cancer. Additionally, because complete inactivation of MEK/ERK in muscle cells can be deleterious, in some embodiments, an effective dose of MEK/ERK inhibitors to treat and/or prevent cachexia results in MEK/ERK pathway activity that is at least 50%, 40%, 30% or 20% of healthy baseline. For example, an effective amount of a MEK/ERK inhibitor to treat and/or prevent cachexia results in MEK/ERK pathway activity that is at least 50%, 40%, 30% or 20% of healthy baseline.

In certain embodiments, MEK/ERK and/or RAGE signaling is inhibited in a myocyte or an adipocyte. In certain embodiments, the myocyte is a mature or immature myocyte. In certain embodiments, the myocyte is a skeletal myocyte or a cardiac myocyte. In a preferred embodiment, the myocyte is a skeletal myocyte or a cardiac myocyte.

In certain embodiments, an effective dose of the compound increases myofibrillar protein content and does not inhibit cellular proliferation of the myocyte. In certain embodiments, the myofibrillar protein is selected from myosin, actin, tropomyosin, myosin heavy chain, myosin light chain, troponin, titin, and nebulin. The myofibrillar protein is raised to at least 75%, at least 80%, at least 90%, at least 95%, or 100% of standard levels. In a preferred embodiment, the myofibrillar protein is a myosin heavy chain protein.

In certain embodiments, the patient has cachexia or pre-cachexia associated with a cancer. In a preferred embodiment, the patient has cachexia or pre-cachexia associated with a cancer. In one embodiment, the cancer is an epithelial-derived cancer or a mesenchemal-derived cancer. In one embodiment, the cancer is selected from one or more of a carcinoma, a sarcoma, and cancers of the skin, pancreas, stomach, colon, thorax, liver, gallbladder, musculoskeletal system, breast, lung, ovary, uterus, endometrium, prostrate, colon, skin, mouth, salivary, esophagus, head and neck, plus other tumors of the gastrointestinal tract.

Significantly, no treatment for cancer-induced cachexia was previously available.

In other embodiments, the patient has age-related weight loss or has age-related sarcopenia. In the case of age-related sarcopenia, unintended weight loss is not necessarily present. In at least a subset of this population, such conditions are characterized by an increase in one or more S100 RAGE ligands and/or a decrease in soluble RAGE relative to a reference level.

In other embodiments, the patient has disease-associated cachexia that is not associated with cancer, but that like cancer-induced cachexia fails to respond to treatment with nutritional support and anti-inflammatory therapy. Such disease-associated cachexia may, for example, be associated with AIDS, chronic obstructive lung disease, or congestive heart failure and, like cancer-induced cachexia are characterized by an increase in one or more S100 RAGE ligands and/or a decrease in soluble RAGE relative to a reference level.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", second edition (Sambrook, 1989); "Oligonucleotide Synthesis" (Gait, 1984); "Animal Cell Culture" (Freshney, 1987); "Methods in Enzymology" "Handbook of Experimental Immunology" (Weir, 1996); "Gene Transfer Vectors for Mammalian Cells" (Miller and Calos, 1987); "Current Protocols in Molecular Biology" (Ausubel, 1987); "PCR: The Polymerase Chain Reaction" (Mullis, 1994); "Current Protocols in Immunology" (Coligan, 1991). These techniques are applicable to the production of the polynucleotides and polypeptides of the invention, and, as such, may be considered in making and practicing the invention. Particularly useful techniques for particular embodiments will be discussed in the sections that follow.

The following examples are put forth so as to provide those of ordinary skill in the art with a complete disclosure and description of how to make and use the assay, screening, and therapeutic methods of the invention, and are not intended to limit the scope of what the inventors regard as their invention.

### EXAMPLES

### Example 1: Differential Cachexia induction by S100 and HMGB1 marker proteins

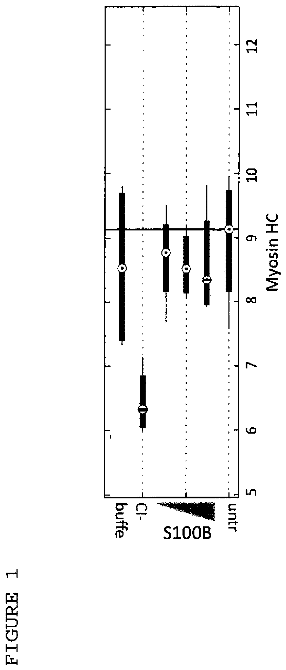

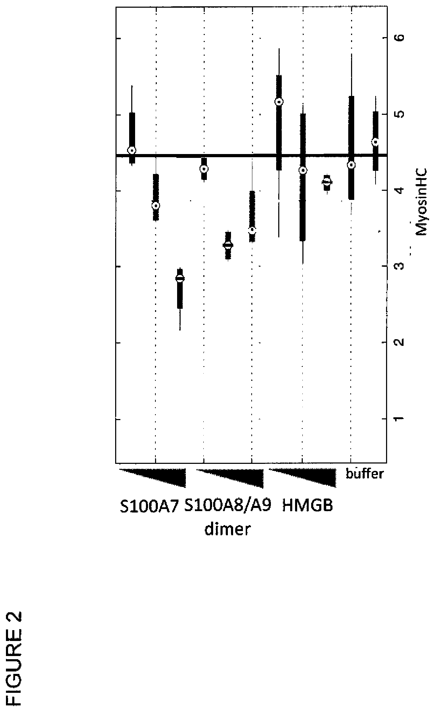

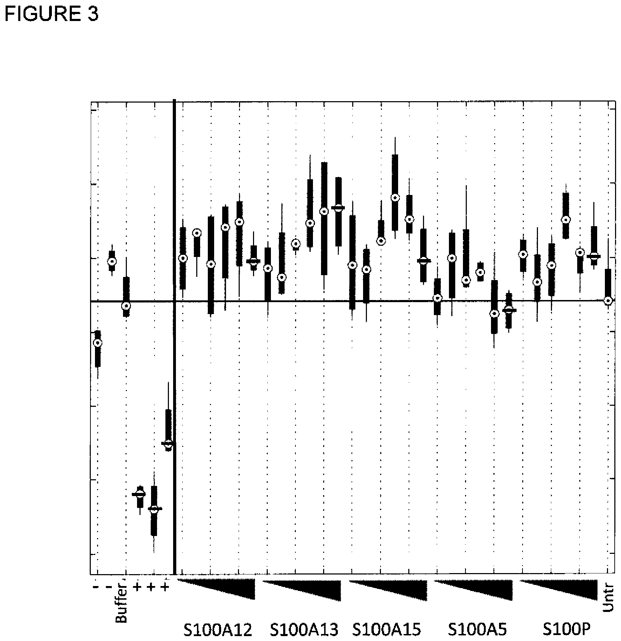

Figures 1-3 illustrate the effect of various proteins of the S100 family, as well as HMGB1, as cachexia inducers when conmpared with cachexia-inducing media. Primary human myoblast were plated and differentiated into terminal myotubes as previously described. Recombinant ligands were prepared at the same dose curve in Hanks Balance Salt Solution (buffer) and applied 1:2 v:v to fresh maintenance media each day for 3 days. Cells were assessed for myosin HC content by in-cell Western staining as previously described. S100 A7, A8 and A9 are shown to be inducers of the cachectic phenotype, whilst S100B, HMGB1 and other S100 family members are not.

### Example 2: Mapping of Helix 2A in S100

Crystallographic structures for each S100 protein involved in the induction of cachexia, as well as S100B, were obtained from public databases and the overlaid using standard software, which revealed the uniqure structural motifs shared among S100A7, A8 and A9. We hypothesize that these uniquely shared structural motifs provide unique binding sites to RAGE and are functionally important in RAGE cachexia signaling. Alignment of the protein sequences indicates that S100 A7, A8 and A9 possess residues forming helix A2, which is not present in S100B and other non-cachectic factors.

### Example 3: Inhibitory Antibodies block cachexia induction by conditioned media

7 anti-RAGE Ab targeting various domains of RAGE were analyzed by MyosinHC in-cell Western analysis in Primary human myotubes. All antibodies were dosed at the same dose curve (1, 5, 10 ug/ml) along with conditioned media from either cachexia-inducing melanoma or gastric cancer cell lines. For comparision, 2 other recently reported anti-cachexia antibodies were tested against the conditioned media as well; both were negative for anti-cachexia activity.

Antibodes tested were as follows:
1. IgG-like C2-type 1, Abbiotec 251890; raised against a KLH-conjugated synthetic peptide encompassing a sequence within the center region of human RAGE.
2. IgG-like C2-type1, Aviva OAAB04025; specific for the central regain of RAGE.
3. V Domain 1, Milipore AB9714; a portion of amino acids 1-60 of human RAGE was used as the immunogen.
4. V-Domain 2, Novus NBP2-03950; raised against a peptide representing amino acids 23-54 of RAGE of bovine origin.
5. V-Domain 4, Santa Cruz SC-3362; raised against a peptide representing amino acids 23-54 of RAGE of bovine origin.
6. V-Domain 5, Santa Cuz SC-365154; RAGE (A-9) is a mouse monoclonal antibody specific for an epitope mapping between amino acids 23-43 at the N-terminus of RAGE of human origin.
7. ECD, R&D Systems MAB-1141; raised against rh-RAGE (aa 24-344).

Antibodies 5 and 7 are effective in inhibiting cachexia.

### Example 4: Development of in vitro readout of cachexia in primary cells

To discover the secreted factors that confer cachexia-inducing ability to cell lines, a tractable *in vitro* model system was developed. In order to maximize physiological relevance, primary human skeletal muscle, adipocytes and hepatocytes were cultured *in vitro.* These cell types represent the principal end-organ tissues affected by cachexia. Interestingly, phenotypic alterations were seen in skeletal myotubes and adipocytes within 5-days of treatment with cachexiarations were seen in skeletal myotubes and adipocytes 2 of alterations in gene expression at the earliest stages of disease. By selecting various time points after contact with cachexia inducing media, markers expressed during pre-cachexia, cachexia, and in refractory cachexia can be identified. A faithful read-out of the cachectic phenotype was also developed using a novel highthroughput, low-cost gene expression profiling method (L1000). L1000 involves the profiling of 1000 human transcripts from which the remainder of the transcriptome can be computationally inferred. This method, validated in over 1 million samples, allowed for profiling of small numbers of cells grown in 384-well plates.

The L1000 platform pairs ligations grown in 384-well plates.llion samples, allbead based detection system and is an extension of a previously reported method for expression profiling based on Luminex bead technology (Peck et al., Genome Biol 7(7):R61 (2006)) to create a 1,000-plex profiling solution. Briefly, the method involves LMA, using locus-specific probes engineered to contain unique molecular barcodes, universal biotinylated primers, and 5.6-micron optically-addressed polystyrene microspheres coupled to capture probes complementary to the barcode sequences. After hybridizing the LMA products to a mixture of beads and staining with streptavidin-phycoerythrin, the hybridization events are detected using a two-laser flow cytometer, whereby one laser detects the bead color (denoting transcript identity), and the other laser detects the phycoerythrin channel (denoting transcript abundance). The plex limit for this approach (set by the number of available bead colors) is 500, whereby the 500 distinct bead colors is discerned using an instrument with the required capabilities and excellent performance characteristics. Further, a computational strategy was developed that allows for two transcripts to be detected using a single bead color (with subsequent computational deconvolution). The resulting assay is a 1,000. Theead color (with subsequent cooped that allo-well plate at very modest cost.

Using the L1000 method, cachectic patient-derived plasma samples induced a reproducible and characteristic gene expression signature in primary skeletal muscle cells in comparison to non-cachectic patient plasma. Consistent with this result and with the physiology of cachexia, skeletal myocytes and adipocytes atrophied in size, and myocytes lost expression of myosin heavy chain protein, as has been previously reported in cancer cachexia (Acharyya, S. et al., J. Clin Invest 1 14(3): 370-378 (2004). Human-cancer-cell conditioned media recapitulated the L1000 cachexia signature seen in patient plasma in *in vitro* human muscle cells. Furthermore, the L1000 cachexia signature was seen in mouse skeletal muscle tissue harvested from cachectic mice and analyzed by Affymetrix gene expression arrays. This analysis showed that the Affymetrix cachexia signature in mouse skeletal muscle tissue correlated with the L1000 cachexia signature of patient human muscles cells, demonstrating that the cachectic signatures correlated across species and across platforms.

These results showed that (1) L1000 provided a robust read-out of the cachectic state, suitable for largeic state,rdies, (2) primary skeletal muscle cells grown in vitro was a powerful model system for discovery, and (3) conditioned media from cachexia-inducing cell lines induced an L1000 signature that recapitulated that of cachectic patient samples. These studies also showed that the immune system was not required to induce cachexia: conditioned media applied directly to primary skeletal muscle in vitro was sufficient to induce the phenotype. And importantly, the conditioned media results proved that the cachexia inducing factor(s) was a secreted molecule.

### Example 5: Cachexia and Epitope identification

Applicants have assessed a preclinical model for cachexia and conducted epitope mapping of inhibitory RAGE antibodies.

Applicants determined that RAGE antibodies have different cachexia-inhibiting activities. For example, a control cachectic condtion is maintained by providing cachexia inducing conditioning media to cells (e.g. conditioning media comprising S100A7, see **Figure 37**)**.** A baseline condition is maintained by evaluating cells which are not exposed to such a conditioning media. Cachetic conditions may be reached when there is a 75% decrease / loss of the myosin heavy chain as compared to the control baseline. Once applying an antibody of the invention, the 75% decrease / loss of the myosin heavy chain as compared to the control baseline may be reversed and the myosin heavy chain levels return to levels of at least 90% of the original baseline levels. Applicants tested a set of polyclonal (anti-human RAGE Ab#1, #2, #3, #4) and monoclonal (anti-human RAGE Ab# 5, #6, #7) antibodies. Anti-human RAGE Ab#1 and #2 associated with the C-domain and anti-human RAGE Ab#3, #4, #5, #6 and #7 associated with the V-domain. Applicants determined that Ab#7 likely has a conformational epitope since it has great inhibitory activity against native RAGE protein in a functional assay, but very poor binding to denatured RAGE (i.e. does not Western blot). Applicants surmise that its epitope intersects with Ab#5, but has other surface conformational components. The RAGE antibodies were found to have different cachexia-inhibiting activities. Applicants also showed that inhibitory human RAGE antibody cross-reacted with mouse wild-type RAGE. Applicants also conducted experiments to show the effect of human RAGE antibody in mouse skeletal muscle. The data showed that human RAGE antibody has cachexia-inhibiting activity in mouse skeletal muscle. Applicants established that RAGE signaling is dependent on V-V domain dimerization. Applicants proceeded to conduct a structural epitope mapping of inhibitory huRAGE mAb and determined that when V-V dimerization occurs, the V-V domain dimers blocked amino acids#23-43 and left only amino acids #44-52 exposed to solvent. Further views of this structural analysis are illustrated herein.

Applicants also defined diagnostic and pharmacodynamic biomarkers in cachexia by conducting a deep proteomic analyses of case controlled patient plasma. Applicants found that pre-clinical cachexia or the state of pre-cachexia is characterized by an increased level of biomarkers S100A7, S100A8, S100A9, MYH1, MYH2, MYH3, MYH6, MYH7, MYH7B, BCAA and INSR in the plasma. In a particular aspect the level of cardio specific MYH, MYH6, MYH7 and/or MYH7B is increased and this is indicative of cardiac pre-cachexia.

### Analysis of a binding site on the surface of human RAGE V domain

The analysis is based on crystallographic structures of soluble human RAGE (PDB ID: 4LP4 (Yatime, L., and Andersen, G.R. (2013). Structural insights into the oligomerization mode of the human receptor for advanced glycation end-products. FEBS J. 280, 6556-6568), 4P2Y (Yatime et al, PDB structure released), S100A7 dimer 2PSR (Brodersen, D.E., Nyborg, J., and Kjeldgaard, M. (1999). Zinc-Binding Site of an S100 Protein Revealed. Two Crystal Structures of Ca2+-Bound Human Psoriasin (S100A7) in the Zn2+-Loaded and Zn2+-Free States,. Biochemistry (Mosc.) 38, 1695-1704) and results of small molecule docking to the V domain.

### Example 6: Interface between V-domains in a dimer

X-ray crystallographic structures of hRAGE and its ligands allowed building a model of a hRAGE-S100A7 oligomer.

A characteristic feature of hRAGE is its ability to form homodimers through V-V domains interactions. V-V dimerization is primarily driven by hydrophobic interactions observed in the structure available in PDB database (ID: 4LP4).

### Example 7: Antibody binding site

Applicants produced a dimer of hRAGE proteins (PDB ID: 4LP4) with surface representation of the solvent accessible area in antigens of two RAGE antibodies: Cachexia inhibiting (green + red residues) and non-inhibiting (red). The area represented in red faces a cellular membrane, and is inaccessible for antibody binding. The amino acids represented in green (within residues 44 to 54) are exposed to the solvent and fully accessible for binding.

Residues K44, P45, P46, R48, E50, and K52 form a region of approximately 31Å in length that is exposed to solvent in the hRAGE dimer.

Analysis of the antibody binding sites was based on the observations from the myosin assay (in-house developed assay to quantify effects of the cachectic phenotype) that out of two RAGE binding antibodies, only one exhibits inhibitory function with respect to Cachexia: RD9C 2 (sc-33662). The other antibody: A-9 (sc-365154) tested was shown to be ineffective in inhibiting the cachectic phenotype. Precise epitopes of the two antibodies are not know, however, datasheets provided by the manufacturer indicate that antibody RD9C 2 binds to the region 23-54 in hRAGE, whereas the ineffective antibody A-9 binds to residues 23-43. Both regions were mapped on the surface of hRAGE using the X-ray structure of a dimer (PDB ID: 4LP4) and Chimera (program for visualization (Pettersen, E.F., Goddard, T.D., Huang, C.C., Couch, G.S., Greenblatt, D.M., Meng, E.C., and Ferrin, T.E. (2004). UCSF Chimera-A visualization system for exploratory research and analysis. J. Comput. Chem. 25, 1605-1612). This analysis together with Applicants' model of hRAGE oligomer showed that ineffectiveness of the A-9 antibody could be explained by a steric hindrance arising due to the dimerization of V domains of hRAGE. As a result the entire region of A-9 binding between residues 23-43 is partially buried between the V domains and access to the solvent exposed part of the antigen is heavily limited by the proximity of a cellular membrane. On the other hand, large part of the binding site of RD9C 2 antibody between residues 44-52 is exposed to the solvent in the dimer of V domains in hRAGE.

### Example 8: Hot spot mapping

In order to detect regions on the surface of V domain of hRAGE that could be binding sites for small molecule ligands, FTMap program was applied.(Kozakov, D., Grove, L.E., Hall, D.R., Bohnuud, T., Mottarella, S.E., Luo, L., Xia, B., Beglov, D., and Vajda, S. (2015). The FTMap family of web servers for determining and characterizing ligand-binding hot spots of proteins. Nat. Protoc. 10, 733-755.)

FTMap algorithm computationally samples positions of small organic solvents as probes to detect enegetically favorable locations and clustres the conformations. A site with the largest number of probes is considered to be the primary binding hot spot. Applicants demonstrate a surface representation of hRAGE with a cluster of molecular solvents (yellow) with the highest number of members (24, with the second one consisting of 15 members) which indicates that the position of the binding site is located between residues 45-49, 66-67, and 78-82 in V domain of hRAGE.

To perform the hot spot mapping, V domain of hRAGE from the X-ray structure 4P2Y was used. Residues 21-22 in the N-terminal part of the X-ray structure constituted an expression tag. Therefore, these residues together with additional two residues 23-24 projecting from otherwise globular V domain were removed. As a result, V domain of hRAGE consisting of residues 25-117 was analyzed with the FTMap program. FTMap algorithm computationally samples positions of small organic solvents as probes to detect enegetically favorable locations and clustres the conformations. A site with the largest number of probes is considered to be the primary binding hot spot.

### Example 9: Small molecule ligand binding site

Using Chimera program for molecular visualization, Applicants inspected the results from the hot spot mapping. Potential binding site detected by the FTMap program appeared to be a highly hydrophobic pocket with four large groves projecting away from the pocket. Amino acid composition of the groves was not as hydrophobic as the main binding pocket. This should allow exploring various potential small molecule drugs and provide opportunity for fine-tuning of their SAR activity.

Docking studies of three different small molecule ligands indicated a common essential binding site, which is located at the junction of three loops: two in the region of strands: B-C and C'-C" and one loop between strands D-E.

**Table 1: Residues involved in formation of the small molecule ligand binding site on the surface of V domain in hRAGE.**

| | Residues |
|---|---|
| Main binding site | P45, P46, Q47, R48 (backbone), L49, P66, Q67, V78, L79, P80, N81, G82 |
| Grove A | E50, K52, V63, L64, S65 |
| Grove B | R48 (side chain), E50, Q100, A101, M102 |
| Grove C | K43, L44, P80, N81 |
| Grove D | G68, G69, D73, R77, F85 |

### Example 10: Docking of small molecule ligands that are known to bind to hRAGE

All-atom structure of human RAGE V domain was taken from the 42PY X-ray crystallography structure. 3D structure of the compounds was obtained using SMILES descriptors that were converted to 3D and a resulting model was subjected to geometry optimization using steepest descent algorithm with MMFF94 force field. The initial geometry optimization was followed by a Weighted Rotor conformational search with the best conformer further refined using Conjugate Gradient geometry optimization

Docking was performed using UCSF Chimera (version 1.10.1) and Autodock Vina (version 1.1.2). To allow for sampling of all possible binding sites on the surface of V domain, a grid enclosing entire V domain was selected for the Autodock Vina docking algorithm. (Trott, O., and Olson, A.J. (2010). AutoDock Vina: Improving the speed and accuracy of docking with a new scoring function, efficient optimization, and multithreading. J. Comput. Chem. 31, 455-461.)

### Results

Tables 2 through 3 show the binding of small molecule ligands to the V domain of hRAGE from the computational docking.

**Table 2: Docking results for the FPS-ZM1 compound are summarized.**

| Model | Binding affinity [kcal/mol] | RMSD (lower bound) [Å] | RMSD (upper bound) [Å] |
|---|---|---|---|
| 1 | -6.2 | 0.0 | 0.0 |
| 2 | -6.1 | 2.5 | 5.5 |
| 3 | -5.7 | 2.9 | 5.9 |
| 4 | -5.7 | 0.9 | 3.7 |
| 5 | -5.5 | 3.1 | 6.4 |
| 6 | -5.5 | 13.7 | 15.7 |
| 7 | -5.4 | 13.5 | 15.3 |
| 8 | -5.4 | 3.4 | 5.9 |
| 9 | -5.4 | 13.6 | 15.5 |

**Table 3: Docking results for the Azeliragon compound are summarized**

| Model | Binding affinity [kcal/mol] | RMSD (lower bound) [Å] | RMSD (upper bound) [Å] |
|---|---|---|---|
| 1 | -5.6 | 0.0 | 0.0 |
| 2 | -5.6 | 4.9 | 7.0 |
| 3 | -5.6 | 4.7 | 6.8 |
| 4 | -5.6 | 1.2 | 2.0 |
| 5 | -5.5 | 5.5 | 12.4 |
| 6 | -5.5 | 4.3 | 7.0 |
| 7 | -5.4 | 6.2 | 14.2 |
| 8 | -5.3 | 4.5 | 11.3 |
| 9 | -5.3 | 6.0 | 13.0 |

**Table 4: Results from docking of the FPS3 compound.**

| Model | Binding affinity [kcal/mol] | RMSD (lower bound) [Å] | RMSD (upper bound) [Å] |
|---|---|---|---|
| 1 | -6.1 | 0.0 | 0.0 |
| 2 | -5.9 | 1.4 | 2.1 |
| 3 | -5.8 | 18.8 | 22.3 |
| 4 | -5.8 | 18.8 | 22.4 |
| 5 | -5.6 | 4.5 | 10.0 |
| 6 | -5.6 | 4.5 | 10.0 |
| 7 | -5.5 | 1.3 | 3.0 |
| 8 | -5.5 | 14.0 | 16.8 |
| 9 | -5.4 | 4.4 | 9.1 |

Applicants' showed results from the docking of FPS-ZM1, Azeliragon, and FPS3, compounds that are known to bind to V domain of hRAGE. It can be noticed that all the molecules bind to the predicted binding site.

### Example 11: In vivo validation of high priority candidate cachexia-inducing proteins.

These studies demonstrate that cachexia-associated proteins are causal of the cachexia phenotype by two approaches: (1) overexpressing candidate cDNAs in non-cachexia-inducing cell lines, and determining whether xenografted mice harboring the transduced cell lines develop cachexia; and (2) ablating expression of candidate genes in cachexia-inducing cell lines, and determining whether xenografted mice now fail to develop cachexia (Boehm, J.S. and Hahn, W.C., Nat Rev Genet 12(7): 487-98 (2011)). Proteins that passed both these tests are likely to be those that explain, at least in large part, cachexia in humans, and should thus be fast-tracked for future therapeutic antibody production aimed at blocking protein activity as a treatment for cachexia.

### (1) Gain-of-function testing of candidate cachexia-inducing proteins.

In parallel experiments, sequence-validated open reading frame (ORF) constructs for each of up to 10 candidate genes are transduced into two non-cachexia-inducing cell lines (AsPC-1 and JHU012) and incorporated into the pLX_TRC206 vector with an in-frame V5 epitope tag and the blasticidin selection marker. Western blot analysis of the V5 epitope tag confirms expression in blasticidin-resistant cells. Five Balb/c nude mice are injected for each transduced cell line and its parental control. Mouse tumor volumes and measures of the cachexia phenotype are collected as described above, in order to determine which ORFs were sufficient to induce cachexia.

### (2) Loss-of-function targeting of candidate cachexia-inducing proteins.

For each of up to 10 candidates, expression is knocked down using at least two validated and shRNA constructs, each internally controlled by preserving the 7 nucleotide seed sequence of each shRNA, while mutating the remaining sequence. The vast majority of off-target effects of shRNAs are mediated by their seed sequence. Such off-target effects could be ruled out by using shRNA-specific seed controls. Stably transduced clones from cachexia-inducing cell lines (MKN1, G361 and TOV21G) are generated and assessed by qPCR, Western blots of targeted protein, and assessment of resulting conditioned media on *in vitro* myocytes. The transduced cells are then injected subcutaneously into the hind limb of 5 female Balb/c nude mice (5e⁶ cells per clone per animal). Tumor size, animal weight, and plasma, muscle, fat, and liver tissues are harvested under standard approved protocols over 8 weeks or a maximal tumor size of 1 cm³. Assessment of weight change is calculated by tumor-free carcass weight/ median weight of sham-injected negative controls. Cachexia is defined as weight loss of ≥ -5%. As an alternative strategy, CRISPR/Cas9-mediated somatic cell knock-out of candidate cachexia-inducing genes could be used rather than RNAi (Martin, L. et al., J Clin Oncol 31(12): 1539-1547 (2013)).

### Example 12: Competition, depletion and reconstitution of RAGE ligands using a recombinant soluble RAGE

Inhibition of RAGE signaling by treatment with recombinant soluble RAGE (rsRAGE) that competes with membrane-bound RAGE for binding RAGE ligands was also shown to inhibit the cachectic induced loss of MyoHC protein in human muscle cells in vitro. Muscle cells were treated with 1, 2, 10, 30 µg/ml rsRAGE in the presence of either cachexia-inducing conditioned media (CI-CM) or non-cachexia-inducing conditioned media (NCI-CM). Furthermore, depletion followed by reconstitution of RAGE ligands was shown to promote the cachectic induced loss of MyoHC protein in human muscle cells in vitro. RAGE ligands were depleted by treatment with 10 µg/ml rsRAGE and reconstituted by treatment with 1x, 0.5x, 0.25x of eluent (PBS).
Together these results further indicate that RAGE is a suitable therapeutic target for the treatment of cachexia.
From **Figure 31** gene listing
Cxa Signatures_Correlations
Left side heat map - gene_symbol

pr_gene; FDPS; TUBB2C; SC4MOL; TAGLN2; C14orf1; TUBB2C; HMGCS1; CSNK2A1; COPE; ___; SNRPB; PRPS1; ISOC2; SDHC; SFR52; SNRPA1; GLRX3; DHCR7; TUBA1B; CYC1; HDGF; XRCC6; LDLR; TUBB2C; SNRPE; HNRNPR; TNFRSF12A; GRB2; ARPC4; ___; EIF31; TCP1; SFR52; TUBB3; TUBA1B; DHCR7; TUBA1B; TUBB; HPRT1; PD1A6; TUBA1B; GPR172A; ENOl; HDGF; TUBB3; SFR52; HNRNPH1; FDFT1; EIF2S1; SNRPA1; RUVBL2; FDFT1; SEC61G; LSM4; BFAR; RPL37A; FRH; TUBA1B; PFN1; CCT5; TMX1; FKBP1A; CNBP; PP1D; PITPNB; PD1A6; GNE; **FTSJT;** EIF4G1; PTGES3; IDI1; MAP2K2; ACTN4; UBE2G2; PRMT5; PPP4C; RPN2; FKBP1A; TIMM17A; PSMD7; PRPF19; ATP1B1; PD1A6; TUBB; IARS; EBP; OGG1; PGAM1; CACYBP; CRK; HMGCS1; HN1; FXR1; ___; GLRX3; HNRNPR; TAGLN2; DST; CD1B0; UBXN6; MYST3; CAMTA2; FAM125B; ZBTB10; KIAA0467; ___; RPA4; TGM3; TPS3TG1; FGD1; CREBL2; EIF3M; RNF8; PAOX; TNFRSF12S; BIRC3; FRAT1; FAM172A; ANO3; CDK19; ___; TBPL1; SPATA2; ___; WWP1; IGKB///IGB; LTBP4; SMG6; CCDC91; CTF1; BAHD1; HTATIP2; ANKRD36B; ZFYVEZ6; MTTMR15; SG5M2; FEM1B; SEMA4C; PARP3; HDDC2; HCG26; MATN4; MPPE1; KLHL22; ACVR1; FEJ10213; PHC1; SPG11; NAALADL1; PTGER1; EML3; NCRNA0018; TBX2; TNFRSF14; CTNNBL1; TBC1D13; C6orf26///; TBX2; PLCB2; ERCC6; MAGEA11; PLAG1; VAMP2; SLC24A1; BBS9; KIAA0495; SYNJ1; FAM193A; C14orf159; LTBP1; ___; FES; ULK1; ABCC10; NYNR1N; LIST; TNXB; ZNF2H2; MPZL1; SCN1B; RAGE; FLJ38109; ZFYVE16; MED13L; CA12; TRAPPC10; LOC644172; ___; FTSJD2; IGKB///IGB; LOC10DZB7; EFS; ZMYNDB; SLC11A2; PLEKHA5; TTC39A; RALGAPA1

### Right side heat map - gene symbols

gene_symbol; Slc7a2; Cpb1; Tfdp2; ___; Nfatc4; Bcas3; 1700023H; 4921515E; Mocos; 1190002N; Mrp147; Jph4; Pank1; Dnase112; Cxcl11; Sgtb; Stc2; Mt1; Hmbox1; Fkbp5; Mtap9; ___; Tbcld1; Ddr1; Lonrf3; Tead4; Lrrn1; 4930448K; ___; Ero1l; Gabrr2; Ahr; Rev31; Scgb1a1; Gm12824; Pacrg; Ms4a10; Heatr2; 2900008C; ___; Epha1; Alcf; Fam107a; 6230414M; Uqcrq; Spats1; ___; Gja5; Klc4; lfna1; Galnt11; Unc119; Cryl1; Ttf2; Zeb2; Gfra2; Racgap1; Prkag2; Ints2; 2610002J0; Wdr76; Ero1l; Clec4a2 //; DOH4S114; Ncoa7; Cryl1; Limk1; D13Ertd60; 15000150; Aplnr; Hmgn3; Socs3; Pyroxd2; Ccnd1; Ccdc111; Cd33; Tnfaip3; Foxfla; Rad5113; Armcx2; Pvr; Lsp1; Vegfc; Zfp810; Zdhhc2; Kdelr3; D3Ertd254; Cited1; Dse; ___; Lime1; Entpd7; Kif11; Tmem8; Lpar6; Pou2f2; 3110040N; Limd2; Marcks; Col3a1

### Other Embodiments

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

## Claims

1. An antibody for use in a method selected from the group consisting of treating pre-cachexia in a subject, inhibiting the progression of pre-cachexia to cachexia in a subject, treating or inhibiting the development of cachexia or pre-cachexia in a subject, and treating or preventing undesirable muscle or fat loss in a cancer patient,
wherein the antibody is administered to the subject or the cancer patient in need thereof in an effective amount,
wherein said antibody associates with an epitope of a RAGE receptor, which prevents the epitope from interacting with a RAGE ligand selected from S100A7, S100A8, S100A9 and/or Calprotectin, and wherein the epitope comprises:
- amino acid residues 44-52 corresponding to the sequence numbering of human RAGE receptor, or
- KPPQRLEWK.

2. An antibody for use according to claim 1, wherein the method is for inhibiting the loss of myosin heavy chain in a myocyte, lipolysis in an adipocyte, or atrophy in a cell selected from an adipocyte, myocyte, or cardiomyocyte,
wherein the method comprises contacting the myocyte, the adipocyte or the cell with an effective amount of the antibody.

3. The antibody for use according to claim 2, wherein the myocyte, adipocyte, or cell is present in a subject identified as having at least one cancer.

4. The antibody for use according to any one of claims 1 or 3, wherein the cancer is selected from carcinomas and sarcomas and cancers of the skin, pancreas, stomach, colon, thorax, liver, gallbladder, musculoskeletal system, breast, lung, ovary, uterus, endometrium, prostate, colon, skin, mouth, salivary, esophagus, head and neck and/or other tumors of the gastrointestinal tract.

5. An antibody for use according to claim 1, wherein the method is for treating or inhibiting the development of cachexia or pre-cachexia, wherein cachexia or pre-cachexia is cardiac cachexia or cardiac pre-cachexia.

6. The antibody for use according to claim 1, wherein the method is for treating or inhibiting the development of cachexia or pre-cachexia;
wherein the subject in need thereof is identified by levels of at least three biomarkers being increased as compared to a control normal reference, and wherein the at least three biomarkers are selected from the group consisting of S100A7, S100A8, S100A9, MYH, INSR and BCAA;
optionally wherein said biomarker levels are analyzed from a sample obtained from the subject; or
optionally wherein the levels of biomarkers S100A7, MYH and INSR are increased; or
optionally wherein the MYH is MYH1, MYH2, MYH3, MYH6, MYH7, MYH7B; or optionally wherein the sample is blood plasma or serum.

7. The antibody for use according to claim 5, wherein the subject in need thereof is identified by levels of at least three of said biomarkers being increased as compared to a control normal reference, and wherein the at least three biomarkers are selected from the group consisting of S100A7, MYH6, MYH7, MYH7B, INSR and BCAA;
optionally wherein said biomarker levels are analyzed from a sample obtained from the subject; or
optionally wherein the levels of biomarkers S100A7, S100A8, S100A9, MYH, INSR and BCAA are increased; or
optionally wherein the sample is blood plasma or serum.

8. A method of inhibiting the loss of myosin heavy chain in a myocyte, inhibiting lipolysis in an adipocyte, or inhibiting atrophy in a cell selected from an adipocyte, myocyte, or cardiomyocyte, the method comprising contacting the myocyte, adipocyte or the cell with an effective amount an antibody,
wherein said antibody associates with an epitope of a RAGE receptor, which prevents the epitope from interacting with a RAGE ligand selected from S100A7, S100A8, S100A9 and/or Calprotectin, wherein the epitope comprises:
- amino acid residues 44-52 corresponding to the sequence numbering of human RAGE receptor, or
- KPPQRLEWK; and
wherein the method is not a method for treatment of the human or animal body by therapy.

9. The method according to claim 8, wherein the myocyte, adipocyte, or cell is *in vitro.*

10. The antibody for use according to any one of claims 1-7; or the method according to claim 8 or 9, wherein the antibody is selected from an IgG, IgA, or an antigen binding antibody fragment selected from an antibody single variable domain polypeptide, dAb, FAb, F(ab')2, an scFv, an Fv, or a disulfide-bonded Fv.

## Patentansprüche

1. Antikörper zur Verwendung in einem Verfahren, ausgewählt aus der Gruppe bestehend aus der Behandlung von Präkachexie in einem Subjekt zur Inhibierung des Fortschreitens von Präkachexie zu Kachexie in einem Subjekt, zur Behandlung oder Inhibierung der Entwicklung von Kachexie oder Präkachexie in einem Subjekt, und zur Behandlung oder Vorbeugung von unerwünschtem Muskel- oder Fettverlust bei einem Krebspatienten,
wobei der Antikörper dem Subjekt oder dem Krebspatienten mit Bedürfnis hierfür in einer wirksamen Menge verabreicht wird,
wobei sich der Antikörper mit einem Epitop eines RAGE-Rezeptors assoziiert, was das Epitop daran hindert, mit einem RAGE-Liganden, ausgewählt aus S100A7, S100A8, S100A9 und/oder Calprotectin zu interagieren, und wobei das Epitop umfasst:
- Aminosäurereste 44-52 entsprechend der Sequenznummerierung von humanem RAGE-Rezeptor, oder
- KPPQRLEWK.

2. Antikörper zur Verwendung nach Anspruch 1, wobei das Verfahren zur Inhibierung des Verlusts der schweren Kette von Myosin in einem Myozyten, Lipolyse in einem Adipozyten oder Atrophie in einer Zelle, ausgewählt aus einem Adipozyten, Myozyten oder Kardiomyozyten, dient,
wobei das Verfahren das Inkontaktbringen des Myozyten, des Adipozyten oder der Zelle mit einer wirksamen Menge des Antikörpers umfasst.

3. Antikörper zur Verwendung nach Anspruch 2, wobei der Myozyt, der Adipozyt oder die Zelle in einem Subjekt vorhanden ist, bei dem festgestellt wurde, dass es wenigstens einen Krebs hat.

4. Antikörper zur Verwendung nach einem der Ansprüche 1 oder 3, wobei der Krebs ausgewählt ist aus Karzinomen und Sarkomen und Krebsen der Haut, der Bauchspeicheldrüse, des Magens, des Dickdarms, des Thorax, der Leber, der Gallenblase, des Muskel-Skelett-Systems, der Brust, der Lunge, des Eierstocks, der Gebärmutter, des Endometriums, der Prostata, des Dickdarms, der Haut, des Mundes, des Speichels, der Speiseröhre, des Kopfes und des Halses und/oder anderen Tumoren des Gastrointestinaltraktes.

5. Antikörper zur Verwendung nach Anspruch 1, wobei das Verfahren zur Behandlung oder Inhibierung der Entwicklung von Kachexie oder Präkachexie dient, wobei Kachexie oder Präkachexie kardiale Kachexie oder kardiale Präkachexie ist.

6. Antikörper zur Verwendung nach Anspruch 1, wobei das Verfahren zur Behandlung oder Inhibierung der Entwicklung von Kachexie oder Präkachexie ist;
wobei das Subjekt mit Bedürfnis hierfür durch Werte von wenigstens drei Biomarkern identifiziert wird, die im Vergleich zu einer normalen Kontrollreferenz erhöht sind, und wobei die wenigstens drei Biomarker ausgewählt sind aus der Gruppe, bestehend aus S100A7, S100A8, S100A9, MYH, INSR und BCAA;
wobei die Biomarkerwerte gegebenenfalls aus einer von dem Subjekt erhaltenen Probe analysiert werden; oder
wobei die Werte der Biomarker S100A7, MYH und INSR gegebenenfalls erhöht sind; oder
wobei die MYH gegebenenfalls MYH1, MYH2, MYH3, MYH6, MYH7, MYH7B ist; oder
wobei die Probe gegebenenfalls Blutplasma oder Serum ist.

7. Antikörper zur Verwendung nach Anspruch 5, wobei das Subjekt mit Bedürfnis hierfür dadurch identifiziert wird, dass die Werte von wenigstens drei der Biomarker im Vergleich zu einer normalen Kontrollreferenz erhöht sind, und wobei die wenigstens drei Biomarker ausgewählt sind aus der Gruppe, bestehend aus S100A7, MYH6, MYH7, MYH7B, INSR und BCAA;
wobei die Werte der Biomarker gegebenenfalls in einer Probe analysiert werden, die von dem Subjekt erhalten wurde; oder
wobei die Werte von Biomarkern S100A7, S100A8, S100A9, MYH, INSR und BCAA gegebenenfalls erhöht sind; oder
wobei die Probe gegebenenfalls Blutplasma oder -serum ist.

8. Verfahren zur Inhibierung des Verlusts der schweren Kette des Myosins in einem Myozyten, zur Inhibierung von Lipolyse in einem Adipozyten oder zur Inhibierung von Atrophie in einer Zelle, ausgewählt aus einem Adipozyten, Myozyten oder Kardiomyozyten, wobei das Verfahren das Inkontaktbringen des Myozyten, Adipozyten oder der Zelle mit einer wirksamen Menge eines Antikörpers umfasst,
wobei sich der Antikörper mit einem Epitop eines RAGE-Rezeptors assoziiert, was das Epitop daran hindert, mit einem RAGE-Liganden, ausgewählt aus S100A7, S100A8, S100A9 und/oder Calprotectin, zu interagieren, wobei das Epitop umfasst:
- Aminosäurereste 44-52 entsprechend der Sequenznummerierung von humanem RAGE-Rezeptor, oder
- KPPQRLEWK; und
wobei das Verfahren kein Verfahren zur Behandlung des menschlichen oder tierischen Körpers durch Therapie ist.

9. Verfahren nach Anspruch 8, wobei der Myozyt, Adipozyt oder die Zelle *in vitro* ist.

10. Antikörper zur Verwendung nach einem der Ansprüche 1-7; oder Verfahren nach Anspruch 8 oder 9, wobei der Antikörper ausgewählt ist aus einem IgG, IgA oder einem Antigen-bindenden Antikörperfragment, ausgewählt aus einem Antikörper-Polypeptid mit einer einzigen variablen Domäne, dAb, FAb, F(ab')2, einem scFv, einem Fv oder einem Disulfid-gebundenen Fv.

## Revendications

1. Anticorps pour son utilisation dans un procédé sélectionné dans le groupe constitué du traitement de la pré-cachexie chez un sujet, de l'inhibition de la progression de la pré-cachexie en cachexie chez un sujet, du traitement ou de l'inhibition du développement de la cachexie ou de la pré-cachexie chez un sujet, et du traitement ou de la prévention de la perte musculaire ou graisseuse indésirable chez un patient cancéreux,
dans lequel l'anticorps est administré au sujet ou au patient cancéreux en ayant besoin en une quantité efficace,
dans lequel ledit anticorps s'associe à un épitope d'un récepteur RAGE, qui empêche l'épitope d'interagir avec un ligand de RAGE sélectionné parmi S100A7, S100A8, S100A9 et/ou la calprotectine, et dans lequel l'épitope comprend :
- les résidus d'acides aminés 44 à 52 correspondant à la numérotation de séquence du récepteur RAGE humain, ou
- KPPQRLEWK.

2. Anticorps pour son utilisation selon la revendication 1, dans laquelle le procédé est destiné à inhiber la perte de la chaîne lourde de myosine dans un myocyte, la lipolyse dans un adipocyte, ou l'atrophie dans une cellule sélectionnée parmi un adipocyte, un myocyte, ou un cardiomyocyte,
dans laquelle le procédé comprend la mise en contact du myocyte, de l'adipocyte ou de la cellule avec une quantité efficace de l'anticorps.

3. Anticorps pour son utilisation selon la revendication 2, dans laquelle le myocyte, l'adipocyte, ou la cellule est présent chez un sujet identifié comme ayant au moins un cancer.

4. Anticorps pour son utilisation selon l'une quelconque des revendications 1 ou 3, dans laquelle le cancer est sélectionné parmi des carcinomes et des sarcomes et des cancers de la peau, du pancréas, de l'estomac, du côlon, du thorax, du foie, de la vessie, du système musculosquelettique, du sein, du poumon, de l'ovaire, de l'utérus, de l'endomètre, de la prostate, du côlon, de la bouche, des glandes salivaires, de l'œsophage, de la tête et du cou et/ou d'autres tumeurs du tractus gastro-intestinal.

5. Anticorps pour son utilisation selon la revendication 1, dans laquelle le procédé est destiné au traitement ou à l'inhibition du développement de la cachexie ou de la pré-cachexie, dans lequel la cachexie ou la pré-cachexie est la cachexie cardiaque ou la pré-cachexie cardiaque.

6. Anticorps pour son utilisation selon la revendication 1, dans laquelle le procédé est destiné au traitement ou à l'inhibition du développement de la cachexie ou de la pré-cachexie ;
dans lequel le sujet en ayant besoin est identifié par des niveaux d'au moins trois biomarqueurs étant accrus par rapport à un étalon de référence normal, et dans lequel les au moins trois biomarqueurs sont sélectionnés dans le groupe constitué de S100A7, S100A8, S100A9, MYH, INSR et BCAA ;
facultativement dans lequel lesdits niveaux de biomarqueurs sont analysés à partir d'un échantillon obtenu du sujet ; ou
facultativement dans lequel les niveaux de biomarqueurs S100A7, MYH et INSR sont accrus ; ou
facultativement dans lequel le MYH est MYH1, MYH2, MYH3, MYH6, MYH7, MYH7B ; ou facultativement dans lequel l'échantillon est du plasma ou du sérum sanguin.

7. Anticorps pour son utilisation selon la revendication 5, dans laquelle le sujet en ayant besoin est identifié par des niveaux d'au moins trois desdits biomarqueurs étant accrus par rapport à un étalon de référence normal, et dans lequel les au moins trois biomarqueurs sont sélectionnés dans le groupe constitué de S100A7, MYH6, MYH7, MYH7B, INSR et BCAA ;
facultativement dans lequel lesdits niveaux de biomarqueurs sont analysés à partir d'un échantillon obtenu du sujet ; ou
facultativement dans lequel les niveaux de biomarqueurs S100A7, S100A8, S100A9, MYH, INSR et BCAA sont accrus ; ou
facultativement dans lequel l'échantillon est du plasma ou du sérum sanguin.

8. Procédé d'inhibition de la perte de la chaîne lourde de myosine dans un myocyte, d'inhibition de la lipolyse dans un adipocyte, ou d'inhibition de l'atrophie dans une cellule sélectionnée parmi un adipocyte, un myocyte, ou un cardiomyocyte, le procédé comprenant la mise en contact du myocyte, de l'adipocyte ou de la cellule avec une quantité efficace d'un anticorps,
dans lequel l'anticorps s'associe à un épitope d'un récepteur RAGE, qui empêche l'interaction de l'épitope avec un ligand de RAGE sélectionné parmi S100A7, S100A8, S100A9 et/ou la calprotectine, dans lequel l'épitope comprend :
- les résidus d'acides aminés 44 à 52 correspondant à la numérotation de séquence du récepteur RAGE humain, ou
- KPPQRLEWK ; et
dans lequel le procédé n'est pas un procédé de traitement du corps humain ou animal par thérapie.

9. Procédé selon la revendication 8, dans lequel le myocyte, l'adipocyte, ou la cellule est *in vitro.*

10. Anticorps pour son utilisation selon l'une quelconque des revendications 1 à 7 ; ou le procédé selon la revendication 8 ou 9, dans lequel l'anticorps est sélectionné parmi une IgG, une IgA, ou un fragment d'anticorps se liant à l'antigène sélectionné parmi un polypeptide d'anticorps à domaine variable unique, dAb, Fab, F(ab')2, un scFv, un Fv, ou un Fv à liaison disulfure.
